# EUROPÄISCHE PATENTSCHRIFT

(11) **EP 0 342 633 B1**
(45) Veröffentlichungstag und Bekanntmachung des Hinweises auf die Patenterteilung: **08.01.1997**
(21) Anmeldenummer: 89108848.6
(22) Anmeldetag: 17.05.1989
(51) Int. Cl.: C12N 15/00, A01N 63/00

(54) **Bacillus thuringiensis Transformation**
Transformation du Bacillus thuringiensis
Bacillus thuringiensis transformation

(30) Priorität: 20.05.1988 CH 1946/88; 02.09.1988 CH 3279/88; 20.01.1989 CH 180/89
(43) Veröffentlichungstag der Anmeldung: 23.11.1989
(73) Patentinhaber: CIBA-GEIGY AG, 4002 Basel (CH)
(72) Erfinder: Schurter, Walter, Dr., CH-4123 Allschwil (CH); Geiser, Martin, Dr., CH-4107 Ettingen (CH); Mathé, Danièle, F-68870 Bartenheim la Chaussée (FR)
(74) Vertreter: Zumstein, Fritz, Dr.

(56) Entgegenhaltungen:
- EP-A- 0 178 151
- ZEITSCHRIFT FÜR ALLGEMEINE MIKROBIOLOGIE, Band 23, Nr. 9, 1983, Seiten 595-599, Berlin, DE; N. SHIVAROVA et al
- Molecular and General Genetics, Band 191, 1983, Seiten 257-262, Berlin, DE; A. KLIER et al
- Archives of Microbiology, Band 139, 1984, Seiten 213-217, Berlin, DE; H.-M. FISCHER et al
- Annual Meeting of the American Society for Microbiology, 8-13 Mai 1988, Seite 155, Zusammenfassung H-64, Miami Beach, US; P.M. MURIANA et al
- Journal of Bacteriology, Band 157, Nr. 2, 1984, Seiten 708-711, Baltimore, US; R.E. RUHFEL et al
- Proceedings of the National Academy of Sciences, Band 79, Nr. 22, Seiten 6951-6955, Washington, US; J.M. GONZALEZ et al
- Chemical Abstracts, Band 107, Nr. 25, December 1987, Seite 226, Nr. 230533h, Columbus, Ohio, US; L.V. KOVTUNENKO et al
- Trends in Biotechnology, Band 6, Nr. 12, Dezember 1988, Seiten 303-309, Cambridge, GB; B.M. CHASSY et al
- FEMS Microbiology, Letters, Band 44, 1987, Seiten 173-177, Amsterdam, NL; B.M. CHASSY et al

## Beschreibung

Die vorliegende Erfindung beschreibt ein Verfahren, welches erstmals eine direkte und zielgerichtete genetische Manipulation von Bacillus thuringiensis sowie dem nahe verwandten B. cereus mit Hilfe der rekombinanten DNA-Technologie ermöglicht, basierend auf einem effizienten Transformationsverfahren für besagte Bacillus-Spezies.

Auch offenhart die vorliegende Erfindung die Konstruktion von Plasmiden und 'shuttle'-Vektoren sowie damit transformierte B. thuringiensis- und/oder B. cereus-Stämme selbst.

Der Gegenstand der vorliegenden Erfindung betrifft ein Verfahren zur Einschleusung und gegebenenfalls Expression von Genen oder sonstigen nützlichen DNA Sequenzen in Bacillus thuringiensis und/oder Bacillus cereus, insbesondere aber ein Verfahren zur Einschleusung und Expression von Protoxingenen.

Ebenfalls umfasst von der vorliegenden Erfindung ist ein Verfahren zur direkten Klonierung sowie gegebenfalls zur Expression und Identifizierung neuer Gene oder sonstiger nützlicher DNA Sequenzen in Bacillus thuringiensis und/oder Bacillus cereus, wodurch erstmals die Möglichkeit besteht Genbanken direkt in Bacillus thuringiensis und/oder Bacillus cereus zu etablieren und dort zu exprimieren.

Bacillus thuringiensis gehört zur grossen Gruppe der gram-positiven, aeroben, Endosporen-bildenden Bakterien. Im Unterschied zu den sehr nahe verwandten Bacillus Arten, B. cereus und B. anthracis, produziert die Mehrzahl der bisher bekannten B. thuringiensis Spezies im Verlaufe ihrer Sporulation einen parasporalen Einschlusskörper, der aufgrund seiner kristallinen Struktur allgemein auch als Kristallkörper bezeichnet wird. Dieser Kristallkörper ist aus insektizid wirksamen kristallinen Protoxin-Proteinen, dem sogenannten δ-Endotoxin zusammengesetzt.

Diese Proteinkristalle sind für die Insektentoxizität von B. thuringiensis verantwortlich. Das δ-Endotoxin entfaltet seine insektizide Aktivität jedoch erst nach der oralen Aufnahme des Kristallkörpers und dessen Auflösung im alkalischen Darmsaft der Zielinsekten sowie der Freisetzung der eigentlichen toxischen Komponente aus dem Protoxin durch limitierte Proteolyse aufgrund der Einwirkung von Proteasen aus dem Verdauungstrakt der Insekten.

Die δ-Endotoxine der verschiedenen B. thuringiensis-Stämme zeichnen sich durch ihre hohe Spezifität gegenüber bestimmten Zielinsekten, insbesondere gegenüber verschiedenen Lepidopteren-, Coleopteren- und Dipterenlarven sowie durch ihre grosse Wirksamkeit aus. Weitere Vorteile, die bei der Verwendung der δ-Endotoxine von B. thuringiensis eine Rolle spielen, liegen in der offensichtlichen Schwierigkeit für die Zielinsekten begründet, Resistenzen gegen das kristalline Protein zu entwickeln sowie in der Unschädlichkeit der Toxine gegenüber Menschen, anderen Säugetieren, Vögeln, Fischen oder Insekten, mit Ausnahme der oben genannten Zielinsekten.

Das insektizide Potential der B. thuringiensis Protoxine wurde schon sehr früh erkannt. Bereits seit Ende der 20iger Jahre werden B. thuringiensis-Präparate als Bioinsektizide zur Bekämpfung verschiedener, durch Insekten verursachter Erkrankungen von Kulturpflanzen eingesetzt. Mit der Entdeckung von B. thuringiensis var. israelensis durch ¹⁾Goldberg and Margalit (1977) sowie B. thuringiensis var. tenebrionis durch ²⁾Krieg et al (1983) konnte das Anwendungsspektrum von B. thuringiensis sogar auf Mücken- und Käferlarven ausgedehnt werden.

Mit der Einführung der Gentechnologie und den sich daraus ergebenden neuen Möglichkeiten hat das Gebiet der B. thuringiensis Toxine einen neuen Aufschwung erfahren.

So gehört die Klonierung von δ-Endotoxingenen in fremden Wirtsorganismen wie beispielsweise in E. coli bereits zur Routine. Das hat dazu geführt, dass mittlerweile die DNA-Sequenzen einer ganzen Reihe von δ-Endotoxingenen bekannt sind (z.B. ³⁾Schnepf HE and Whiteley HR, 1981; ⁴⁾Klier A et al, 1982; ⁵⁾Geiser M et al, 1986; ⁶⁾Haider MZ et al, 1987).

Die meisten B. thuringiensis Arten enthalten mehrere Gene, die ein insektizid wirksames Protein kodieren. Diese Gene, die nur während der Sporulationsphase exprimiert werden, sind in der Mehrzahl der Fälle auf grossen übertragbaren Plasmiden (30 - 150 Md) lokalisiert und können daher sehr einfach zwischen den verschiedenen B. thuringiensis Stämmen sowie zwischen B. thuringiensis und B. cereus ausgetauscht werden, sofern diese kompatibel sind (⁷⁾Gonzalez JM et al, 1982).

Die Protoxingene von B. thuringiensis var. kurstaki gehören zu einer Familie verwandter Gene, von denen verschiedene bereits kloniert und sequenziert wurden. Diese Arbeiten wurden in erster Linie in einem E. coli Klonierungssystem durchgeführt.

Die Klonierung von B. thuringiensis-Genen war somit bisher im wesentlichen auf einige wenige und ausschliesslich heterologe Wirtssysteme beschränkt, von denen das E. coli System das am besten untersuchte und verstandene ist.

Mittlerweile sind jedoch auch Berichte über eine erfolgreiche Klonierung und Expression von Protoxingenen in anderen Wirtssystemen bekannt geworden, wie z.B. in B. subtilis (⁴⁾Klier A et al, 1982), Pseudomonas fluoreszens (⁸⁾Obukowicz MG et al, 1986), sowie Saccharomyces cerevisiae (EP 0 238 441). Auch der Einbau und die Expression des δ-Endotoxingens in pflanzliche Wirtszellen ist inzwischen geglückt (EP 0 292 435).

Bei der Klonierung in E. coli wird die Tatsache ausgenützt, dass manche Protoxingene zusätzlich zu den gram-positiven Promotoren zufälligerweise auch einen E. coli-ähnlichen Promotor enthalten. Diese Promotor-ähnlichen DNA-Sequenzen machen es möglich, dass die B. thuringiensis Protoxingene auch in heterologen Wirtssystemen exprimiert werden können, sofern diese in der Lage sind die oben erwähnten Kontrollsequenzen zu erkennen.

Die exprimierten Protoxinproteine können dann, nach Aufbrechen der Wirtszellen, mit Hilfe bekannter Verfahren isoliert und identifiziert werden.

Es hat sich zwischenzeitlich aber gezeigt, dass nicht in allen Fällen E. coli-ähnliche Promotoren in Protoxingenen vorhanden sind (⁹⁾Donovan et al, 1988), sodass bisher nur ganz bestimmte Protoxingene, welche die zuvor genannten Voraussetzungen erfüllen, in heterologen Wirtssystemen exprimierbar und damit identifizierbar sind.

Die Klonierung von Genen ausserhalb des natürlichen Wirtsorganismus sowie die Verwendung dieser Stämme in der Praxis als Bioinsektizide ist somit mit einer Reihe zum Teil gravierender Nachteile verbunden:
a) Eine Expression von B. thuringiensis Protoxingenen ist nur in bestimmten Fällen möglich.
b) Es erfolgt im allgemeinen keine oder aber nur eine geringe Sekretion von exprimierten Fremdproteinen.
c) Eine korrekte Faltung der δ-Endotoxine ist im reduzierenden Milieu von heterologen Wirtszellen nicht immer gewährleistet, was eine unerwünschte Aenderung der spezifischen Aktivität oder des Wirtsbereiches der Toxine zur Folge haben kann.
d) Falls eine Expression überhaupt stattfindet, so sind die Expressionsraten der klonierten Fremdgene unter den nativen Expressionssequenzen zumeist nur gering.

^{3);10)}Schnepf und Whitley (1981; 1985) schätzen, dass das in E. coli klonierte B. thuringiensis Toxin nur 0,5 % bis 1 % des gesamten Zellproteins von E. coli ausmacht, wohingegen das Kristallprotein in B. thuringiensis zwischen 30 % und 40 % des Trockengewichts sporulierender Kulturen beträgt. Diese gravierenden Unterschiede in den Expressionsraten sind möglicherweise auf das Fehlen von Sporulations-spezifischen Kontrollsignalen in den heterologen Wirtssystemem sowie auf Schwierigkeiten bei der Erkennung der B. thuringiensis Promotoren und/oder Problemen bei der posttranslationalen Modifikation des Toxinmoleküls durch den Fremdwirt zurückzuführen.
e) Viele der allgemein zur Expression verwendeten Wirtsstämme sind toxikologisch nicht so unbedenklich wie B. thuringiensis und B. cereus.
f) B. thuringiensis und B. cereus bilden einen natürlichen Hauptbestandteil der mikrobiellen Bodenflora, was für die meisten der allgemein zur Expression verwendeten Wirtsstämme nicht zutrifft.

Diese zuvor genannten Probleme und Schwierigkeiten könnten überwunden werden, wenn es gelänge, besagte B. thuringiensis Gene direkt im homologen Wirtssystem zu klonieren, wo die natürlichen gram-positiven Promotoren der Protoxingene zur Expression benützt werden können. Es ist bisher nicht gelungen, ein effizientes Transformationssystem für B. thuringiensis dieses aus kommerzieller Sicht so wichtige Bakterium, sowie den nahe verwandten B. cereus zu entwickeln, welches hinreichend hohe Transformationsraten gewährleistet und somit die Anwendung bereits etablierter rDNA-Techniken beispielsweise eine effiziente Wiedereinschleusung eines klonierten Protoxingens in einen B. thuringiensis-Stamm.

Die bisher verwendeten Verfahren zur Herstellung neuer B. thuringiensis Stämme mit neuen insektiziden Eigenschaften basieren vornehmlich auf dem konjugalen Transfer von Plasmid kodierten Protoxingenen.

Eine erfolgreiche Wiedereinschleusung eines klonierten B. thuringiensisKristallproteingens in B. thuringiensis ist bisher erst in einem einzigen Fall beschrieben (¹¹⁾Klier A et al, 1983), wobei aber auch hier, in Ermangelung eines geeigneten Transformationssystems für B. thuringiensis, auf den konjugalen Transfer zwischen B. subtilis und B. thuringiensis zurückgegriffen werden musste. Auch bei diesem von Klier et al beschriebenen Verfahren wird E. coli als Zwischenwirt verwendet.

Die konjugalen Transfer-Verfahren weisen jedoch eine ganze Reihe von gravierenden Nachteilen auf, die sie für eine routinemässige Anwendung zur genetischen Modifikation von B. thuringiensis und/oder B. cereus ungeeignet erscheinen lassen.
a) Der konjugale Transfer von Plasmid kodierten Protoxingenen ist nur zwischen B. thuringiensis-Stämmen bzw. zwischen B. cereus und B. thuringiensis-Stämmen möglich, die kompatibel miteinander sind.
b) Bei konjugalem Plasmidtransfer zwischen weiter entfernten Stämmen wird häufig nur eine geringe Transferfrequenz erzielt.
c) Es gibt keine Möglichkeit, die Expression der Protoxingene zu regulieren oder zu modifizieren.
d) Es gibt keine Möglichkeit, das Gen selbst zu modifizieren.
e) Bei Anwesenheit mehrerer Protoxingene in einem Stamm kann die Expression einzelner Gene aufgrund des sog. Gen-Dosis-Effektes stark reduziert sein.
f) Es kann zum Auftreten von Instabilitäten kommen aufgrund einer möglichen homologen Rekombination verwandter Protoxingene.

Alternative Transformationsverfahren, die beispielsweise bei vielen grampositiven Organismen mittlerweile routinemässig Anwendung finden, erwiesen sich sowohl bei B. thuringiensis als auch bei B. cereus als ungeeignet.

Zu den vorgenannten Verfahren gehört z.B. die direkte Transformation von Bakterienprotoplasten mit Hilfe der Polyethylenglykolbehandlung, die bei vielen Streptomyces-Stämmen (¹²⁾Bibb JJ et al, 1978) sowie bei B. subtilis (¹³⁾Chang S and Cohen SN, 1979), B. megaterium (¹⁴⁾Brown BJ and Carlton BC, 1980), Streptococcus lactis (¹⁵⁾Kondo JK and McKay LL, 1984), S. faecalis (¹⁶⁾Wirth R et al), Corynebacterium glutamicum (¹⁷⁾Yoshihama M et al, 1985) sowie zahlreichen anderen grampositiven Bakterien erfolgreich angewendet wurde.

Für die Anwendung dieses Verfahrens müssen die Bakterienzellen zunächst protoplastisiert werden, d.h. die Zellwände werden mit Hilfe lytischer Enzyme verdaut.

Eine weitere Voraussetzung für die erfolgreiche Anwendung dieses direkten Transformationsverfahrens bildet die Expression der neu eingeführten genetischen Information sowie die Regeneration der transformierten Protoplasten auf komplexen Festmedien, bevor eine erfolgreiche Transformation z.B. mit Hilfe eines Selektionsmarkers nachgeweisen werden kann.

Dieses Transformationsverfahren erwies sich für B. thuringiensis und den nahe verwandten B. cereus als ungeeignet. Aufgrund der hohen Resistenz der B. thuringiensis-Zellen gegenüber Lysozym und der nur mangelhaften Regenerierbarkeit der Protoplasten zu intakten Zellwand-haltigen Zellen bleiben die erreichbaren Transformationsraten gering und schwierig zu reproduzieren (¹⁸⁾Alikhanian SJ et al, 1981; ¹⁹⁾Martin PA et al, 1981; ²⁰⁾Fischer H-M et al, 1984).

Mit diesem Verfahren lassen sich daher höchstens sehr einfache Plasmide, die für die Arbeit mit rekombinanter DNA ungeeignet sind, mit geringer Frequenz in B. thuringiensis oder B. cereus Zellen einschleusen.

Einzelne Berichte über zufriedenstellende Transformationsraten, die mit Hilfe des zuvor beschriebenen Verfahrens erreicht werden konnten, beruhen auf der Ausarbeitung sehr aufwendiger Optimierungsprogramme, die aber immer nur konkret für einen bestimmten B. thuringiensis-Stamm anwendbar sind und mit einem hohen Aufwand an Zeit und Kosten verbunden sind (²¹⁾Schall D, 1986). Diese Verfahren sind daher für eine routinemässige Anwendung in industriellem Masstab ungeeignet.

Wie die intensiven Forschungsarbeiten auf dem Gebiet der B. thuringiensis Genetik erkennen lassen, besteht ein grosses Interesse an der Entwicklung neuer Verfahren, die B. thuringiensis oder den nahe verwandten B. cereus einer direkten genetischen Modifizierbarkeit zugänglich machen, und damit beispielsweise eine Klonierung von Protoxingenen im natürlichen Wirtssystem ermöglichen. Dennoch ist es bisher nicht gelungen, die bestehenden Schwierigkeiten und Probleme zufriedenstellend zu lösen.

Zur Zeit stehen weder geeignete Transformationsverfahren zur Verfügung, die eine schnelle effiziente und reproduzierbare Transformation von B. thuringiensis und/oder B. cereus mit ausreichend hoher Transformationsfrequenz ermöglichen, noch sind geeignete Klonierungsvektoren verfügbar, die eine Anwendung der für andere bakterielle Wirstsysteme bereits etablierten rekombinanten DNA Techniken auch auf B. thuringiensis erlauben. Dies trifft in gleicher Weise auch für B. cereus zu.

Diese Aufgabe konnte jetzt überraschenderweise im Rahmen der vorliegenden Erfindung durch die Anwendung einfacher, zum Teil bekannter Verfahrensmassnahmen gelöst werden.

Die vorliegende Erfindung betrifft somit ein neuartiges Verfahren, welches basierend auf der rekombinanten DNA Technologie erstmals eine direkte, zielgerichtete und reproduzierbare genetische Manipulation von B. thuringiensis sowie von B. cereus ermöglicht, indem man Bacillus thuringiensis und/oder Bacillus cereus mit Hilfe eines einfachen Transformationsverfahrens mit hoher Effizienz transformiert, unter Verwendung einer rekombinanten DNA, die für die vorgesehene genetische Manipulation von Bacillus thuringiensis und/oder Bacillus cereus geeignet ist.

Das neuartige Verfahren umfasst auch die Einschleusung, Klonierung und Expression von Genen oder sonstigen nützlichen DNA Sequenzen, insbesondere aber von Protoxin-Genen, in B. thuringiensis und/oder B. cereus, wobei man
a) besagte Gene oder DNA isoliert;
b) die isolierten Gene oder DNA gegebenenfalls mit Expressionssequenzen, die in Bacillus thuringiensis und/oder B. cereus funktionsfähig sind, operabel verknüpft;
c) die genetischen Konstruktionen aus Abschnitt b) unter Verwendung geeigneter Vektoren in Bacillus thuringiensis und/oder B. cereus Zellen transformiert; und
d) gegebenenfalls ein entsprechendes Genprodukt exprimiert und sofern gewünscht, isoliert.

Ebenfalls umfasst von neuartigen verfahren ist eine direkte Methode zur Klonierung, Expression und Identifizierung von Genen oder sonstigen nützlichen DNA-Sequenzen, insbesondere aber von Protoxin-Genen, in B. thuringiensis und/oder B. cereus, wobei man
a) die Gesamt-DNA von Bacillus thuringiensis mit Hilfe geeigneter Restriktionsenzyme verdaut;
b) aus den resultierenden Restriktionsfragmenten solche geeigneter Grösse isoliert;
c) besagte Fragmente in einen geeigneten Vektor einspleisst;
d) Bacillus thuringiensis und/oder B. cereus Zellen mit besagtem Vektor transformiert; und
e) aus den Transformanten mit Hilfe geeigneter Screeningverfahren neue DNA Sequenzen aufspürt und gegebenenfalls isoliert.

Neben Strukturgenen können selbstverständlich auch beliebige andere nützliche DNA-Sequenzen in dem erfindungsgemässen Verfahren verwendet werden, wie z.B. nicht-kodierende DNA Sequenzen, die eine regulatorische Funktion aufweisen, wie z.B. 'anti-sense-DNA.

Das erfindungsgemässe Verfahren eröffnet damit eine Vielzahl neuer Möglichkeiten, die sowohl unter wissenschaftlichen als auch unter kommerziellen Gesichtspunkten von ausserordentlichem Interesse sind.

So ist es beispielsweise nunmehr erstmals möglich, Aufschlüsse über die Regulation der 6-Endotoxin-Synthese, insbesondere im Hinblick auf die Sporulation, auf der genetischen Ebene zu gewinnen.

Auch die Frage, an welcher Stelle des Toxinmoleküls sich der (die) für die Insektentoxizität verantwortliche(n) Abschnitt(e) befindet(n) und inwieweit diese(r) auch mit der Wirtsspezifität in Zusammenhang steht(en), sollte sich jetzt klären lassen.

Die Kenntnisse der molekularen Organisation der verschiedenen Toxinmoleküle sowie der diese Moleküle kodierenden Toxingene aus den verschiedenen B. thuringiensis Spezies ist von ausserordentlichem praktischen Interesse für eine gezielte genetische Manipulation dieser Gene, die mit Hilfe des erfindungsgemässen Verfahrens nunmehr erstmals möglich wird.

Das neue erfindungsgemässe Verfahren erlaubt neben einer gezielten Modifikation der δ-Endotoxin-Gene selbst auch die Manipulation der die Expression dieser Gene kontrollierenden regulatorischen DNA-Sequenzen, wodurch sowohl die spezifischen Eigenschaften der δ-Endotoxine wie z.B. ihre Wirtsspezifität, ihr Resorptionsverhalten u.a. gezielt verändert als auch ihre Produktionsraten gesteigert werden können, z.B. durch den Einbau von stärkeren und effizienteren Promotor-Sequenzen.

Durch gezielte Mutation ausgewählter Gene oder Subgene in vitro gelangt man somit zu neuen B. thuringiensis und/oder B. cereus Varianten.

Eine weitere Möglichkeit zur Konstruktion neuer B. thuringiensis und/oder B. cereus Varianten besteht im Zusammenspleissen von Genen oder Genabschnitten, die aus verschiedenen B. thuringiensis-Quellen stammen, wodurch B. thuringiensis und/oder B. cereus-Stämme mit einem erweiterten Anwendungsspektrum entstehen. Auch synthetisch oder halb-synthetisch hergestellte Toxin Gene können auf diese Weise für die Konstruktion neuer B. thuringiensis und/oder B. cereus Varietäten verwendet werden.

Darüberhinaus ermöglicht das erfindungsgemässe Verfahren aufgrund der starken Erhöhung der Transformationsfrequenz sowie der Vereinfachung des Verfahrens zum erstenmal die Erstellung von Genbanken sowie ein schnelles Screening von modifizierten und neuen Genen in B. thuringiensis und/oder B. cereus.

Insbesondere ermöglicht das erfindungsgemässe Verfahren jetzt erstmals eine direkte Expression von Genbanken in B. thuringiensis und/oder B. cereus sowie die Identifizierung neuer Protoxingene in B. thuringiensis mit Hilfe bekannter, vorzugsweise immunologischer oder biologischer Verfahren.

Gegenstand der vorliegenden Erfindung ist somit ein Verfahren, das basierend auf einer starken Erhöhung der Effizienz der B. thuringiensis/B**.** cereus Transformation gegenüber vorbekannten Verfahren erstmals eine direkte genetische Modifikation des B. thuringiensis und/oder B. cereus Genoms möglich macht.

Im einzelnen betrifft die vorliegende Erfindung ein Verfahren zur Transformation von B. thuringiensis und/oder B. cereus durch Einschleusung rekombinanter DNA, insbesondere von Plasmid- und/oder Vektor-DNA, in B. thuringiensis und/oder B. cereus Zellen mit Hilfe der Elektroporation.

Bevorzugt ist dabei ein Verfahren zur Transformation von B. thuringiensis und/oder B. cereus mit δ-Endotoxin kodierenden DNA-Sequenzen sowie DNA-Sequenzen, die für ein Protein kodieren, das im wesentlichen die insektentoxischen Eigenschaften besagter B. thuringiensis Toxine aufweist.

Die vorliegende Erfindung unfasst somit auch die Expression von DNA-Sequenzen, die ein δ-Endotoxin kodieren oder aber ein Protein, welches zumindest im wesentlichen die insektentoxischen Eigenschaften des B. thuringiensis Toxins aufweist, in transformierten B. thuringiensis- und/oder B. cereus-Zellen.

Im Rahnen der vorliegenden Erfindung ist ein Verfahren zur Herstellung bifunktioneller Vektoren, sogenannter 'shuttle'-Vektoren, für B. thuringiensis und/oder B. cereus sowie die Verwendung besagter 'shuttle'-Vektoren zur Transformation von B. thuringiensis und/oder B. cereus-Zellen beschrieben.

Bevorzugt ist die Konstruktion bifunktioneller Vektoren, die ausser in B. thuringiensis und/oder B. cereus in einem oder mehreren anderen, heterologen Wirtssystemen, insbesondere aber in E. coli Zellen replizieren.

Im besonderen wird ein Verfahren zur Herstellung von 'shuttle'-Vektoren für B. thuringiensis und/oder B. cereus beschreiben, die eine DNA-Sequenz enthalten, die ein δ-Endotoxin-Polypeptid kodiert, das natürlicherweise in B. thuringiensis vorkommt oder aber zumindest ein Polypeptid, das diesem im wesentlichen homolog ist, d.h. das zumindest im wesentlichen die insektentoxischen Eigenschaften des B. thuringiensis Toxins aufweist. Ebenso beschreiben ist die Verwendung dieser 'shuttle'-Vektoren zur Transformation von B. thuringiensis und/oder B. cereus-Zellen sowie die Expression der auf besagten 'shuttle'-Vektoren vorhandenen DNA-Sequenzen, insbesondere derjenigen DNA-Sequenzen, die für ein δ-Endotoxin aus B. thuringiensis kodieren oder aber zumindest für ein Protein, das im wesentlichen die insektentoxischen Eigenschaften der B. thuringiensis Toxine aufweist.

Besagte 'Shuttle'-Vektoren gestatten somit die Verwendung von B. thuringiensis und/oder B. cereus als generelle Wirtsorganismen für die Klonierung und Expression homologer sowie insbesondere auch heterologer DNA oder einer Kombination aus homologer und heterologer DNA.

Besonders hervorgehaben werden die bifunktionellen ('shuttle') Vektoren pXI61 (=pK61) und pXI93 (=pK93), die, transformiert in B. thuringiensis var. kurstaki HD1 cryB bzw. B. cereus 569 K, bei der als Internationale Hinterlegungsstelle anerkannten 'Deutschen Sammlung von Mikroorganismen' (Braunschweig, BRD) gemäss den Bestimmungen des Budapester Vertrages unter der Nummer DSM 4573 (pXI61, transformiert in B. thuringiensis var. kurstaki HD1 cryB) bzw. DSM 4571 (pXI93, transformiert in B. thuringiensis var. kurstaki HD1 cryB) und DSM 4573 (pXI93, transformiert in B. cereus 569 K) hinterlegt worden sind.

Die vorliegende Erfindung ermöglicht somit die Herstellung neuer B. thuringiensis und B. cereus-Varietäten, die mit einer DNA-Sequenz transformiert sind, welche ein δ-Endotoxin aus B. thuringiensis kodiert und exprimiert oder aber zumindest ein Protein, das im wesentlichen die toxischen Eigenschaften der B. thuringiensis Toxine aufweist.

Die transformierten B. thuringiensis und B. cereus Zellen sowie die von diesen produzierten Toxine können zur Herstellung insektizider Mittel zur Bekänpfung von Insekten verwendet werden.

Im folgenden soll eine kurze Beschreibung der Abbildungen gegeben werden.
- Abbildung 1:: Transformation von E. coli HB 101 mit pBR322 (o) und ∗B. thuringiensis HDl cryB mit pBC16(·) (△ Anzahl der überlebenden ∗HDl cryB Zellen).
- Abbildung 2:: Einfluss des Alters einer ∗B. thuringiensis HDl cryB Kultur auf die Transformationsfrequenz.
- Abbildung 3:: Einfluss des pH-Wertes der PBS-Pufferlösung auf die Transformationsfrequenz.
- Abbildung 4:: Einfluss der Saccharosekonzentration der PBS-Pufferlösung auf die Transformationsfrequenz.
- Abbildung 5:: Abhängigkeit zwischen der Anzahl der Transformanten und der Menge an pro Transformation eingesetzter DNA.
- Abbildung 6:: Vereinfachte Restriktionskarte des "shuttle"-Vektors ∗pXI61. Der schraffierte Balken kennzeichnet die vom gram-positiven pBC16 stammenden Sequenzen, der Rest stammt vom gram-negativen Plasmid pUC8.
- Abbildung 7:: Vereinfachte Restriktionskarte von ∗pXI93. Der schraffierte Balken kennzeichnet das Protoxin-Strukturgen (Pfeil, Kurhdl) und die 5' und 3' nicht-kodierenden Sequenzen. Der restliche nicht-schraffierte Teil stammt vom "shuttle"-Vektor ∗pxI61.
- Abbildung 8:: SDS (Sodiumdodecylsulfat)/Polyacrylamid-Gelelektrophorese von Extrakten sporulierender Kulturen von ∗B. thuringiensis HD1 cryB, B. cereus 569K und ihren Derivaten.
[1: ∗HD1 cryB (pXI93), 2: ∗HD1 cryB (pXI61), 3: ∗HD1 cryB, 4: HD1, LBG B-4449, 5: ∗B. cereus 569K (pXI93), 6: 569K]
a) Comassie-gefärbt, M: Molekulargewichts-Standard, MG: Molekulargewicht (Dalton), Pfeil: Position des 130 000 Dalton Protoxins.
b) Western blot des gleichen Gels, versetzt mit polyklonalen Antikörpern gegen das K-1 Kristallprotein von B. thuringiensis HD1.
   Das Auffinden positiver Banden erfolgte mit Hilfe von markierten Anti-Ziegen Antikörpern. Pfeil: Position des 130'000 Dalton Protoxins. Weitere Banden: Abbauprodukte des Protoxins.

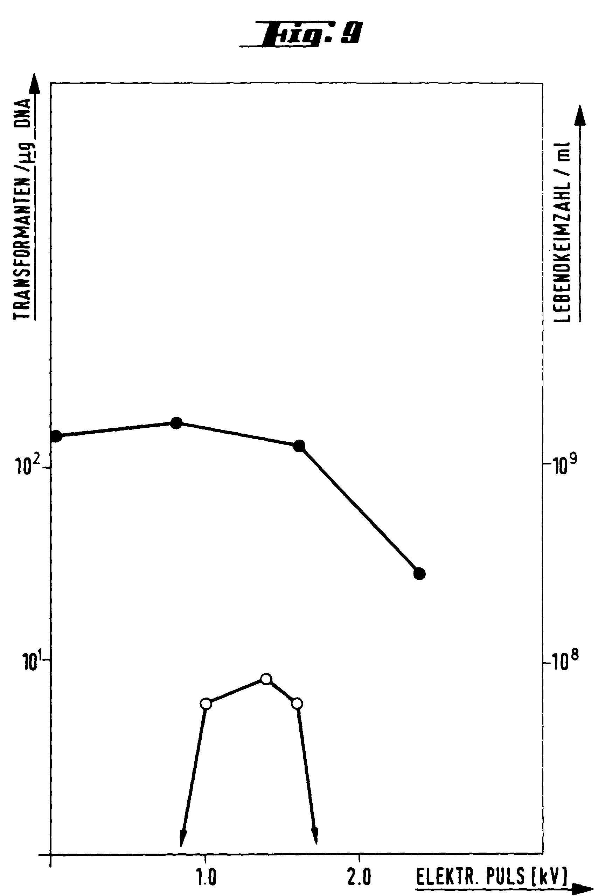

- Abbildung 9:: Transformation von B. subtilis LBG B-4468 mit pBC16 Plasmid DNA mit der für B. thuringiensis optimierten Elektroporationsmethode.
(o: Transformanten/µg Plasmid DNA; ·: Lebendkeimzahl/ml)

∗Die im Prioritätsdokument für die Benennung der Plasmide gewählte interne Bezeichnung pK wurde für die Auslandsfassung durch die offiziell anerkannte Bezeichnung pXI ersetzt.
∗Die im Prioritätsdokument für die Benennung der Plasmide gewählte interne Bezeichnung pK wurde für die Auslandsfassung durch die offiziell anerkannte Bezeichnung pXI ersetzt.

Auch die Bezeichung für die im Rahmen der Ausführungsbeispiele verwendete asporogene B. thuringiensis HD1 Mutante wurde von cryβ nach cryB geändert.

Ein wesentlicher Aspekt vorliegender Erfindung betrifft ein neuartiges Transformationsverfahren für B. thuringiensis und B. cereus, das auf der Einschleusung von Plasmid-DNA in B. thuringiensis und/oder B. cereus Zellen unter Anwendung der an sich bekannten Elektroporationstechnologie beruht.

Nachdem bis zum Zeitpunkt der vorliegenden Erfindung alle Versuche gescheitert waren, die bei anderen bakteriellen Wirtssystemen bereits etabilierten Transformationsverfahren auch auf B. thuringiensis und den nahe verwandten B. cereus zu übertragen, konnte jetzt im Rahmen dieser Erfindung durch die Anwendung der Elektroporationstechnologie sowie begleitender Massnahmen ein überraschender Erfolg erzielt werden.

Dieser Erfolg muss insbesondere auch deshalb als überraschend und unerwartet angesehen werden, als von Seiten einer sowjetischen Gruppe (²²⁾Shivarova N et al, 1983) bereits zu einem früheren Zeitpunkt Elektroporationsversuche an B. thuringiensis-Protoplasten durchgeführt wurden, die erreichten Transformationsfrequenzen aber so gering waren, dass dieses Verfahren in der Folge als unbrauchbar für eine B. thuringiensis Transformation angesehen wurde und infolgedessen keine weitere Beachtung mehr fand.

Aufbauend auf Untersuchungen der für eine Elektroporation von B. thuringiensis und/oder B. cereus-Zellen kritischen Verfahrensparameter konnte jetzt überraschenderweise ein Transformationsverfahren entwickelt werden, das in idealer Weise an die Bedürfnisse von B. thuringiensis und B. cereus angepasst ist und zu Transformationsraten führt, die in einem Bereich zwischen 10⁶ und 10⁸ Zellen/µg Plasmid DNA liegen, insbesondere aber in einem Bereich zwischen 10⁶ und 10⁷ Zellen/µg Plasmid DNA.

Annähernd gleich hohe Transformationsraten mit Werten zwischen 10² und maximal 10⁶ Transformanten/µg Plasmid DNA, konnten bisher nur mit dem bei ²¹⁾Schall (1986) beschriebenen PEG (Polyethylenglykol) Transformationsverfahren erzielt werden. Hohe Transformationsraten bleiben jedoch auf diejenigen B. thuringiensis Stämme beschränkt, für die das PEG Verfahren in sehr zeitaufwendigen Optimierungsstudien spezifisch angepasst wurde, was dieses Verfahren für eine praktische Anwendung als ungeeignet erscheinen lässt.

Darüberhinaus erweisen sich diese Verfahren in der Praxis in vielen Fällen als nicht oder aber nur schlecht reproduzierbar.

Im Gegensatz dazu handelt es sich bei dem vorliegenden erfindungsgemässen Verfahren um ein prinzipiell auf alle B. thuringiensis- sowie B. cereus-Stämme anwendbares Transformationsverfahren, das weniger zeitaufwendig, rationeller und somit effizienter ist als die traditionellen PEG Transformationsverfahren.

So können in dem erfindungsgemässen Verfahren beispielsweise ganze, intakte Zellen eingesetzt werden, wodurch die zeitaufwendige und für B. thuringiensis sowie B. cereus kritische Protoplastierung sowie die anschliessende Regeneration auf komplexen Nährmedien entfällt.

Darüberhinaus kann bei Anwendung der PEG-Verfahren die Durchführung der notwendigen Verfahrensmassnahmen bis zu einer Woche beanspruchen, während mit dem erfindungsgemässen Transformationsverfahren die transformierten Zellen innerhalb weniger Stunden (in der Regel über Nacht) erhältlich sind.

Ein weiterer Vorteil des erfindungsgemässen Verfahrens betrifft die Anzahl von B. thuringiensis und/oder B. cereus Zellen, die pro Zeiteinheit transformiert werden kann.

Während bei den traditionellen PEG-Verfahren nur kleine Aliquots gleichzeitig ausplattiert werden können, um eine Hemmung der Regeneration durch ein zu dichtes Wachstum der Zellen zu vermeiden, können bei Anwendung der Elektroporationstechnik grosse Mengen an B. thuringiensis- und/oder B. cereus Zellen gleichzeitig ausplattiert werden.

Dies ermöglicht den Nachweis von Transformanten auch bei sehr kleinen Transformationsfrequenzen, was mit den vorbeschriebenen Verfahren nicht oder aber nur mit erheblichem Aufwand möglich ist.

Darüberhinaus genügen bereits DNA-Mengen im Nanogramm-Bereich um zumindest einige Transformanten zu erhalten.

Dies ist ganz besonders dann von Bedeutung, wenn ein sehr effizientes Transformationssystem benötigt wird, wie beispielsweise bei der Verwendung von DNA-Material aus E. coli, was aufgrund eines stark ausgeprägten Restriktionssystems in B. thuringiensis-Zellen gegenüber B. thuringiensis DNA zu einer Reduktion der Transformationsfrequenzen um den Faktor 10³ führen kann.

Das erfindungsgemässe Transformationsverfahren, das im wesentlichen auf der an sich bekannten Elektroporationstechnologie basiert, ist durch die folgenden spezifischen Verfahrensmassnahmen charakterisiert:
a) Herstellen einer Zellsuspension mit geeigneter Zelldichte in einem für die Anzucht von B. thuringiensis-Zellen geeigneten Kultivierungsmedium und bei einer für das Wachstum der Zellen ausreichenden Belüftung;
b) Abtrennen der Zellen aus der Zellsuspension und resuspendieren in einem für die nachfolgende Elektroporation geeigneten Inokulationspuffer;
c) Zugabe einer DNA-Probe in einer für die Elektroporation geeigneten Konzentration;
d) Einbringen des unter Punkt b) und c) beschriebenen Ansatzes in eine Elektroporationsapparatur;
e) Eine oder mehrere kurzzeitige Entladungen eines Kondensators über die Zellsuspension zur kurzfristigen Erzeugung hoher elektrischer Feldstärken über einen Zeitraum, der für eine Transformation von B. thuringiensis und/oder B. cereus Zellen mit rekombinanter DNA ausreichend ist;
f) gegebenenfalls Nachinkubation der elektroporierten Zellen;
g) Ausplattieren der elektroporierten Zellen auf einem geeigneten Selektionsmedium; und
h) Auslesen der transformierten Zellen.

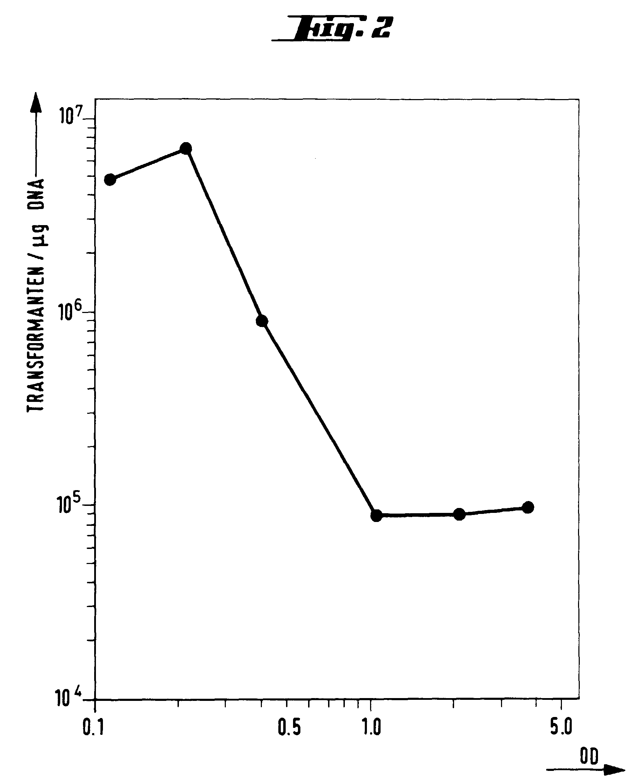

In einer spezifischen und im Rahmen dieser Erfindung bevorzugten Ausführungsform des erfindungsgemässen Verfahrens werden die B. thuringiensis-Zellen zunächst in einem geeigneten Nährmedium bei ausreichender Belüftung und bei einer geeigneten Temperatur, vorzugsweise von 20°C bis 35°C, inkubiert, bis eine optische Dichte (OD₅₅₀) von 0.1 bis 1.0 erreicht ist. Das Alter der für die Elektroporation vorgesehenen Bacillus-Kulturen hat einen deutlichen Einfluss auf die Transformationsfrequenz. Besonders bevorzugt ist daher eine optische Dichte der Bacillus-Kulturen von 0.1 bis 0.3, insbesondere aber von 0.2. Es sei jedoch darauf hingewiesen, dass sich auch mit Bacillus-Kulturen aus anderen Wachstumsphasen, insbesondere auch mit Uebernachtkulturen gute Transformationsfrequenzen erzielen lassen (siehe Abbildung 2).

Als Ausgangsmaterial verwendet man in der Regel frische Zellen oder Sporen, es kann aber ebensogut auch auf tiefgefrorenes Zellmaterial zurückgegriffen werden. Dabei handelt es sich vorzugsweise um Zellsuspensionen von B. thuringiensis und/oder B. cereus Zellen in geeigneten Flüssigmedien, welchen vorteilhafterweise ein bestimmter Anteil eines 'Frostschutzmittels' zugesetzt wird.

Geignete Frostschutzmittel sind in erster Linie Gemische von osmotisch aktiven Komponenten und DMSO in Wasser oder einer geeigneten Pufferlösung. Weitere geeignete Komponenten, die für eine Verwendung in Frostschutzmittellösungen in Frage kommen, umfassen Zucker, mehrwertige Alkohole,wie z.B. Glycerin, Zuckeralkohole, Aminosäuren und Polymere, wie z.B. Polyethylenglykol.

Geht man von B. thuringiensis-Sporen aus, so werden diese zunächst in einem geeigneten Medium inokuliert und über Nacht bei einer geeigneten Temperatur, vorzugsweise von 25°C bis 28°C und ausreichender Belüftung inkubiert. Dieser Ansatz wird anschliessend verdünnt und in der oben beschriebenen Weise weiterbehandelt.

Zur Induktion der Sporulation bei B. thuringiensis können alle Medien verwendet werden, die eine solche Sporulation hervorrufen. Bevorzugt im Rahmen dieser Erfindung ist ein GYS-Medium nach ²³⁾Yousten AA und Rogoff MH, (1969).

Der Sauerstoffeintrag in das Kultivierungsmedium erfolgt in der Regel durch Bewegen der Kulturen, z.B. auf einer Schüttelmaschine, wobei Umdrehungsgeschwindigkeiten zwischen 50 Upm und 300 Upm bevorzugt sind.

Die Kultivierung von B. thuringiensis-Sporen sowie vegetativer Mikroorganismenzellen im Rahmen der vorliegenden Erfindung erfolgt nach bekannten, allgemein gebräuchlichen Methoden, wobei aus Gründen der Praktikabilität vorzugsweise flüssige Nährmedien verwendet werden.

Die Zusammensetzung der Nährmedien kann je nach verwendetem B. thuringiensis- bzw. B. cereus Stamm leicht variieren. Allgemein werden komplexe Medien mit wenig definierten, leicht assimilierbaren Kohlenstoff-(C-) und Stickstoff-(N-) Quellen bevorzugt, wie sie üblicherweise für die Kultivierung aerober Bacillus-Arten eingesetzt werden.

Zusätzlich werden Vitamine und essentielle Metallionen benötigt, die aber in der Regel in den verwendeten komplexen Nährmedien in ausreichender Konzentration als Bestandteile bzw. Verunreinigungen enthalten sind.

Gegebenenfalls können besagte Bestandteile wie z.B. essentielle Vitamine sowie Na⁺, K⁺, Cu²⁺, Ca²⁺, Mg²⁺, Fe³⁺, NH₄ ^{⊕}, PO₄ ³⁻, SO₄ ²⁻, Cl⁻, CO₃ ²⁻ -Ionen und die Spurenelemente Cobalt und Mangan, Zink u.a. in Form ihrer Salze zugesetzt werden.

Als besonders geeignete Stickstoffquellen sind neben Hefe-Extrakten, Hefe-Hydrolysaten, Hefe-Autolysaten sowie Hefe-Zellen vor allem Sojamehl, Maismehl, Hafermehl, Edamin (enzymatisch verdautes Lactalbumin), Pepton, Caseinhydrolysat, Maisablaugen sowie Fleischextrakte zu nennen, ohne dass der Erfindungsgegenstand durch diese beispielhafte Aufzählung in irgendeiner Weise eingeschränkt werden soll.

Die bevorzugte Konzentration für die genannten N-Quellen liegt zwischen 1.0 g/l und 20 g/l.

Als C-Quelle kommen in erster Linie Glucose, Lactose, Sucrose, Dextrose, Maltose, Stärke, Cerelose, Cellulose sowie Malzextrakt in Frage. Der bevorzugte Konzentrationsbereich liegt zwischen 1.0 g/l und 20 g/l.

Neben komplexen Nährmedien können selbstverständlich auch halb- oder vollsynthetische Medien verwendet werden, die die oben näher bezeichneten Nährstoffe in geeigneter Konzentration enthalten.

Ausser dem im Rahmen der vorliegenden Erfindungen bevorzugt verwendeten LB-Medium können auch alle anderen, für die Kultivierung von B. thuringiensis und/oder B. cereus geeigneten Kulturmedien verwendet werden, wie z.B. Antibiotic Medium 3, SCGY-Medium u.a. Die Aufbewahrung sporulierter B. thuringiensis-Kulturen erfolgt bevorzugterweise auf GYS Medien (Schrägagar) bei einer Temperatur von 4°C.

Nachdem die Zellkultur die gewünschte Zelldichte erreicht hat, werden die Zellen mit Hilfe einer Zentrifugation geerntet und in einer geeigneten Pufferlösung suspendiert, die zuvor vorzugsweise mit Eis gekühlt wird.

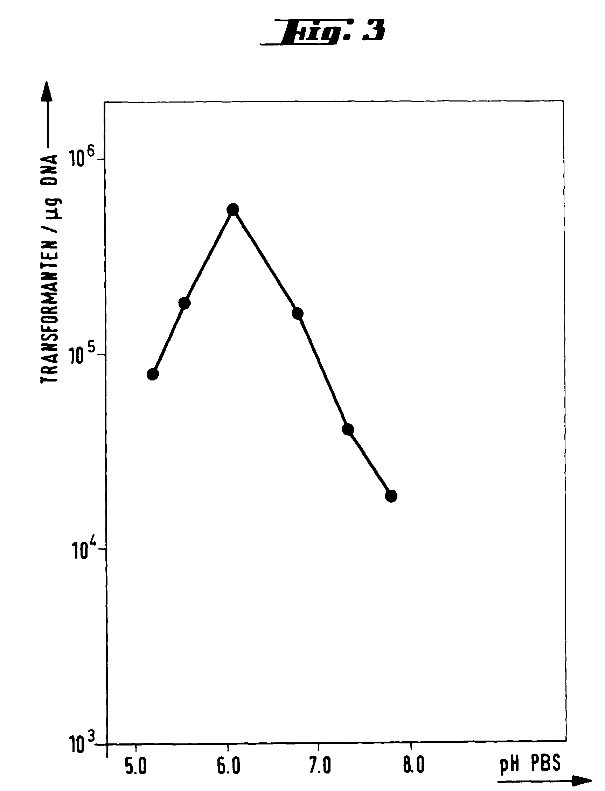

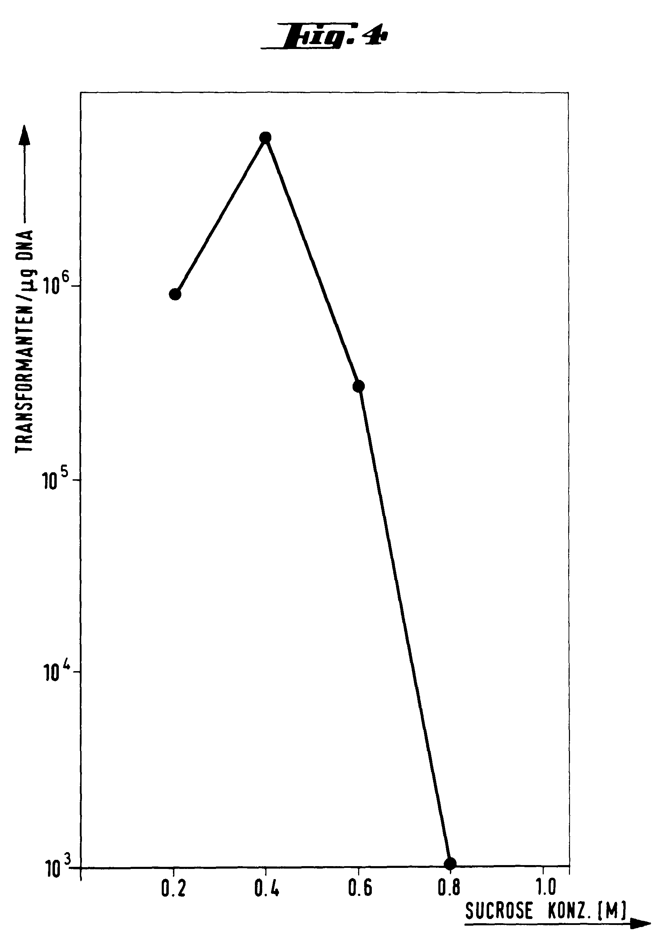

Die Temperatur erwies sich im Laufe der Untersuchungen als nicht kritisch und ist daher in einem weiten Bereich frei wählbar. Bevorzugt ist ein Temperaturbereich von 0°C bis 35°C, vorzugsweise von 2°C bis 15°C und ganz besonders bevorzugt von 4°C. Die Inkubationsdauer der Bacillus-Zellen vor und nach der Elektroporation hat nur geringen Einfluss auf die erreichbare Transformationsfrequenz (siehe Tabelle 1). Einzig eine überlange Inkubation führt zu einer Abnahme der Transformationshäufigkeit. Bevorzugt ist eine Inkubationszeit von 0.1 bis 30 Minuten, insbesondere von 10 Minuten. Die Temperatur erwies sich im Laufe der Untersuchungen als nicht kritisch und ist daher in einem weiten Bereich frei wählbar. Bevorzugt ist ein Temperaturbereich von 0°C bis 35°C, vorzugsweise von 2°C bis 15°C und ganz besonders bevorzugt von 4°C. Dieser Vorgang kann ein bis mehrmals wiederholt werden. Besonders geeignete Pufferlösungen im Rahmen dieser Erfindung sind osmotisch stabilisierte Phosphatpuffer, die als stabilisierendes Agens Zucker, wie z.B. Glucose oder Saccharose oder Zuckeralkohole, wie z.B. Mannit enthalten und auf pH-Werte von 5.0 bis 8.0 eingestellt sind. Ganz besonders bevorzugt sind Phosphatpuffer vom PBS-Typ mit einem pH-Wert von 5.0 bis 8.0, vorzugsweise von pH 5.5 bis 6.5, die Saccharose als stabilisierendes Agens in einer Konzentration von 0.1 M bis 1.0 M, vorzugsweise aber von 0.3 M bis 0.5 M enthalten (siehe Abbildungen 3 und 4).

Aliquots der suspendierten Bacillus-Zellen werden anschliessend in Küvetten oder beliebige andere, geeignete Gefässe überführt und mit einer DNA-Probe für einen geeigneten Zeitraum, vorzugsweise für einen Zeitraum von 0.1 bis 30 Minuten, insbesondere aber von 5 bis 15 Minuten, und bei einer geeigneten Temperatur, vorzugsweise bei einer Temperatur von 0°C bis 35°C, insbesondere aber bei einer Temperatur von 2°C bis 15°C und ganz besonders bevorzugt bei einer Temperatur von 4°C, gemeinsam inkubiert.

Beim Arbeiten mit tiefen Temperaturen ist es vorteilhaft bereits vorgekühlte Küvetten oder beliebige andere, geeignete und vorgekühlte Gefässe zu verwenden.

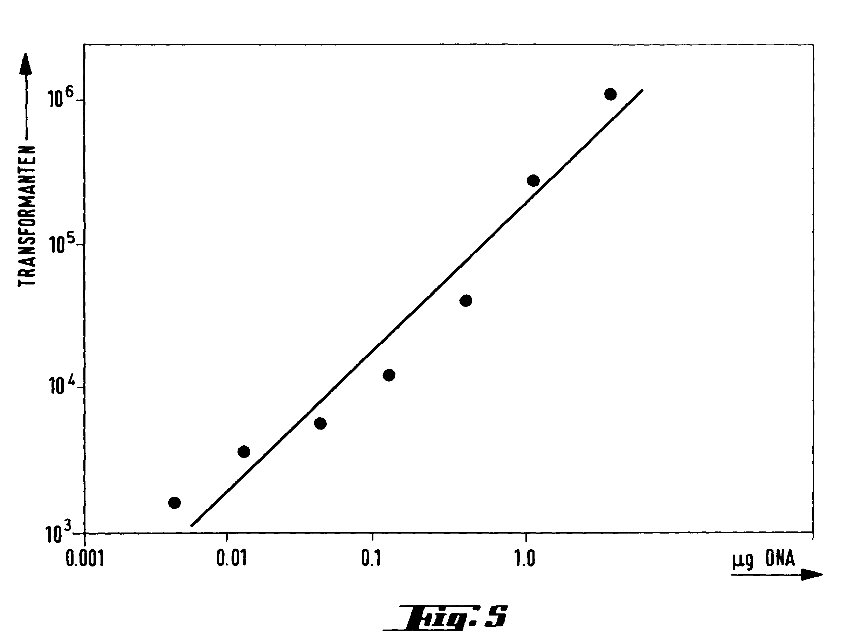

Zwischen der Anzahl transformierter Zellen und der bei der Elektroporation verwendeten DNA Konzentration besteht über einen weiten Bereich ein linearer Zusammenhang, wobei die Anzahl transformierter Zellen mit steigender DNA Konzentration zunimmt (siehe Abbildung 5). Die im Rahmen dieser Erfindung bevorzugte DNA-Konzentration liegt in einem Bereich zwischen 1 ng und 20 µg. Besonders bevorzugt ist eine DNA-Konzentration von 10 ng bis 2 µg.

Danach wird der gesamte Ansatz enthaltend B. thuringiensis- und/oder B. cereus-Zellen sowie Plasmid-DNA oder eine andere geeignete DNA-Probe in eine Elektroporationsapparatur eingebracht und einer Elektroporation unterzogen, d.h. kurzzeitig einem elektrischen Impuls ausgesetzt.

Elektroporationsapparaturen, die für eine Verwendung in dem erfindungsgemässen Verfahren geeignet sind, werden mittlerweile bereits von verschiedenen Herstellern angeboten, wie z.B. der Fa. Bio Rad (Richmond, CA, USA; 'Gene Pulser Apparatus'), Biotechnologies and Experimental Research, Inc. (San Diego, CA, USA; 'BTX Transfector 100'), Promiga (Madison, WI, USA; 'X-Cell 2000 Electroporation System'), etc..

Selbstverständlich kann in dem erfindungsgemässen Verfahren auch jedes beliebige andere geeignete Gerät verwendet werden.

Es können dabei verschiedene Impulsformen verwendet werden, so z.B. Rechteckimpulse oder aber exponentiell abklingende Impulse.

Letztere sind im Rahmen dieser Erfindung bevorzugt. Sie kommen durch die Entladung eines Kondensators zustande und sind charakterisiert durch einen zunächst sehr raschen Anstieg der Spannung sowie durch eine sich anschliessende exponentielle Abklingphase als Funktion aus Widerstand und Kapazitanz. Die Zeitkonstante RC gibt ein Mass für die Länge der exponentiellen Abklingzeit. Sie entspricht derjenigen Zeit, die benötigt wird, um die Spannung auf 37 % der Ausgangsspannung (Vₒ) abklingen zu lassen.

Einer für die Beeinflussung der Bakterien-Zelle entscheidenden Parameter betrifft die Stärke des elektrischen Feldes mit dem die Zellen beaufschlagt werden und das sich aus dem Verhältnis von angelegter Spannung und dem Abstand der Elektrodenplatten berechnet.

Ebenfalls von grosser Bedeutung ist in diesem Zusammenhang die exponentielle Abklingzeit, die sowohl von der Konfiguration der verwendeten Apparatur (z.B. der Kapazität des Kondensators) als auch von anderen Parametern abhängt, wie z.B. der Zusammensetzung der Pufferlösung oder den eingesetzten Volumina der für die Elektroporation vorgesehenen Zellsuspension.

So hat es sich beispielsweise im Verlaufe der Untersuchungen gezeigt, dass eine Reduktion des Volumens der für die Elektroporation vorgesehenen Zellsuspension um die Hälfte zu einer Erhöhung der Transformationsfrequenz um den Faktor 10 führt.

Eine Verlängerung der exponentiellen Abklingzeit über eine Optimierung der verwendeten Pufferlösung resultiert ebenfall in einer deutlichen Erhöhung der Transformationsfrequenz.

Alle Massnahmen, die zu einer Verlängerung der exponentiellen Abklingzeit und in der Folge zu einer Erhöhung der Transformationsfrequenz führen, sind daher im Rahmen dieser Erfindung bevorzugt.

Die im Rahmen des erfindugsgemässen Verfahrens bevorzugte Abklingzeit liegt zwischen ca. 2 ms und ca. 50 ms, insbesondere aber zwischen ca. 8 ms und ca. 20 ms. Ganz besonders bevorzugt ist eine exponentielle Abklingzeit von ca. 10 ms bis ca. 12 ms.

Im Rahmen der vorliegenden Erfindung werden die Bakterienzellen durch kurzzeitige Entladung(en) eines Kondensators über die DNA-haltige Zellsuspension kurzfristig mit sehr hohen elektrischen Feldstärken beaufschlagt, wodurch die Permeabilität der B. thuringiensis-Zellen kurzfristig und reversibel erhöht wird. Die Elektroporationsparameter werden im Verlaufe des erfindungsgemässen Verfahrens in einer Weise aufeinander abgestimmt, dass eine optimale Aufnahme der im Elektroporationspuffer befindlichen DNA in die Bacillus-Zellen gewährleistet ist.

Die Kapazitätseinstellung am Kondensator beträgt im Rahmen dieser Erfindung vorteilhafterweise 1 µF bis 250 µF, insbesondere aber 1 µF bis 50 µF und ganz besonders bevorzugt 25 µF. Die Wahl der Ausgangsspannung ist in weiten Bereichen unkritisch und daher frei wählbar. Bevorzugt ist eine Ausgangsspannung V₀ von 0.2 kV bis 50 kV, insbesondere aber von 0.2 kV bis 2.5 kV und ganz besonders bevorzugt von 1.2 kV bis 1.8 kV. Der Abstand der Elektrodenplatten hängt u.a. ab von der Dimensionierung der Elektroporationsapparatur. Er beträgt vorteilhafterweise zwischen 0.1 cm und 1.0 cm, vorzugsweise zwischen 0.2 cm und 1.0 cm. Besonders bevorzugt ist ein Plattenabstand von 0.4 cm. Aus dem Abstand der Elektrodenplatten und der am Kondensator eingestellten Ausgangsspannung ergeben sich die Feldstärkewerte, die auf die Zellsuspension einwirken. Diese liegen vorteilhafterweise in einem Bereich zwischen 100 V/cm und 50'000 V/cm. Besonders bevorzugt sind Feldstärken von 100 V/cm bis 10'000 V/cm, insbesondere aber von 3'000 V/cm bis 4'500 V/cm.

Die Feinabstimmung der frei wählbaren Parameter, wie z.B. Kapazität, Ausgangsspannung, Plattenabstand, etc. hängt bis zu einem gewissen Grad von der Architektur der verwendeten Geräte ab und kann daher von Fall zu Fall in bestimmten Grenzen variieren. Die angegebenen Grenzwerte können daher in gewissen Fällen auch über- oder unterschritten werden sofern dies zum Erreichen optimaler Feldstärkewerte erforderlich sein sollte.

Der eigentliche Elektroporationsvorgang kann ein- bis mehrmals wiederholt werden, bis eine für das jeweilige System optimale Transformationsfrequenz erreicht ist.

Im Anschluss an die Elektroporation kann vorteilhafterweise eine Nachinkubation der behandelten Bacillus-Zellen durchgeführt werden, vorzugsweise für einen Zeitraum von 0.1 bis 30 Minuten, bei einer Temperatur von 0°C bis 35°C, vorzugsweise von 2°C bis 15°C. Anschliessend werden die elektroporierten Zellen mit einem geeigneten Medium verdünnt und nochmals für einen geeigneten Zeitraum, vorzugsweise von 2 bis 3 Stunden unter ausreichender Belüftung und bei einer geeigneten Temperatur, vorzugsweise von 20°C bis 35°C, inkubiert.

Anschliessend werden die B. thuringiensis Zellen auf Festmedien ausplattiert, die ein für die Selektion der neu in die Bakterienzelle eingeführten DNA-Sequenzen geeignetes Agens als Zusatz enthalten. Je nach Art der verwendeten DNA kann es sich bei besagten Agenzien z.B. um antibiotisch wirksame Verbindungen, um Farbstoffe u.a. handeln. Besonders bevorzugt im Rahmen dieser Erfindung für die Selektion von Bacillus thuringiensis und/oder B. cereus Zellen sind Antibiotika, ausgewählt aus der Gruppe bestehend aus Tetracyclin, Kanamycin, Chloramphenicol und Erythromycin.

Ebenfalls bevorzugt sind chromogene Substrate, wie z.B. X-gal (5-Brom-4-chlor-3-indolyl-β-D-galaktosid), die anhand einer spezifischen Farbreaktion nachgewiesen werden können.

Andere phänotypische Marker sind dem Fachmann bekannt und können ebenfalls im Rahmen dieser Erfindung verwendet werden.

Es können dabei beliebige, für die Kultivierung von B. thuringiensis-Zellen geeignete Nährmedien verwendet werden, denen eines der üblicherweise verwendeten Verfestigungsmedien, wie z.B. Agar, Agarose, Gelatine u.a. zugesetzt wird.

Die zuvor im einzelnen für B. thuringiensis beschriebenen Verfahrensparameter sind in gleicher Weise auch auf B. cereus-Zellen anwendbar.

Das zuvor beschriebene erfindungsgemässe Verfahren zur Transformation von B. thuringiensis bzw. B. cereus bleibt nicht, wie die bisher im Stand der Technik zur Verfügung stehenden Vefahren, auf die Verwendung von bestimmten natürlicherweise in B. thuringiensis und/oder B. cereus vorkommenden Plasmiden beschränkt, sondern ist für alle Arten von DNA anwendbar. Somit ist es nunmehr erstmals möglich, B. thuringiensis und/oder B. cereus gezielt zu transformieren, wobei neben homologer, d.h. natürlicherweise in B. thuringiensis oder dem nahe verwandten B. cereus vorkommender Plasmid DNA, auch Plasmid DNA heterologen Ursprungs verwendet werden kann.

Dabei kann es sich sowohl um Plasmid DNA handeln, die natürlicherweise in einem anderen Organismus als B. thuringiensis oder dem nahe verwandten B. cereus vorkommt, wie beispielsweise die Plasmide pUB110 und pC194 aus Staphylococcus aureus (²⁴⁾Horinouchi S und Weisblum B, 1982; ²⁵⁾Polak J und Novick RP, 1982) sowie das Plasmid pIM13 aus B. subtilis (²⁶Mahler J und Halvorson HO, 1980), die in der Lage sind, in B. thuringiensis und/oder B. cereus zu replizieren, als auch um hybride Plasmid DNA, die mit Hilfe der rekombinanten DNA Technologie aus homologer Plasmid DNA oder aus heterologer Plasmid DNA oder aber aus einer Kombination von homologer und heterologer Plasmid DNA konstruiert wird. Letztgenannte hybride Plasmid-DNA ist besser geeignet für die Arbeiten mit rekombinanter DNA als die natürlichen Isolate.

Beispielshaft seien hier die Plasmide pBD64 (²⁷⁾Gryczan T et al, 1980), pBD347, pBD348 sowie pUB1664 genannt, ohne dadurch jedoch den Gegenstand vorliegender Anmeldung in irgendeiner Weise zu beschränken.

Von besonderer Bedeutung für die Durchführung von Klonierungsexperimenten bei verschiedenen B. thuringiensis- bzw. B. cereus-Stämmen dürften die für B. subtilis bereits etablierten Klonierungsvektoren, wie beispielsweise pBD64 sein.

Neben Plasmid-DNA kann im Rahmen der vorliegenden Erfindung auch jede beliebige andere DNA in B. thuringiensis bzw. B. cereus transformiert werden. Die transformierte DNA kann dabei entweder autonom oder integriert im Chromosom replizieren. Dabei kann es sich beispielsweise um eine Vektor-DNA handeln, die sich nicht von einem Plasmid, sondern von einem Phagen ableitet.

Besonders bevorzugt im Rahmen dieser Erfindung ist die Konstruktion und Verwendung von bifunktionellen (hybriden) Plasmid-Vektoren, sogenannten 'shuttle'-Vektoren, die in der Lage sind, ausser in B. thuringiensis oder dem nahe verwandten B. cereus in einem oder aber in mehreren heterologen Wirtsorganismen zu replizieren und die sowohl in homologen als auch in heterologen Wirtssystemen identifizierbar sind.

Als heterologe Wirtsorganismen sind im Rahmen dieser Erfindung alle diejenigen Organismen zu verstehen, die nicht in die Gruppe B. thuringiensis/B. cereus gehören und die in der Lage sind, eine sich selbst replizierende DNA stabil zu erhalten.

Als heterologe Wirtsorganismen können gemäss obiger Definition somit sowohl prokaryontische als auch eukaryontische Organismen fungieren. Beispielhaft seien an dieser Stelle als Vertreter aus der Gruppe der prokaryontischen Wirtsorganismen einzelne Vetreter aus den Gattungen Bacillus, wie z.B. B. subtilis oder B. megaterium, Staphylococcus, wie z.B. S. aureus, Streptococcus, wie z.B. Streptococcus faecalis, Streptomyces, wie z.B. Streptomyces spp., Pseudomonas, wie z.B. Pseudomonas spp., Escherichia, wie z.B. E. coli, Agrobacterium, wie z.B. A. tumefaciens oder A. rhizogenes, Salomonella, Erwinia, etc. genannt. Aus der Gruppe der eukaryontischen Wirte sind in erster Linie Hefen sowie tierische und pflanzliche Zellen zu nennen. Diese beispielhafte Aufzählung ist nicht abschliessend und soll den Gegenstand der vorliegenden Erfindung in keiner Weise limitieren. Dem Fachmann sind weitere geeignete Vertreter aus der Gruppe der prokaryontischen und eukaryontischen Wirtsorganismen bekannt.

Besonders bevorzugt im Rahmen dieser Erfindung sind B. subtilis oder B. megaterium, Pseudomonas spp., und insbesondere E. coli aus der Gruppe der prokaryontischen Wirte sowie Hefen und tierische oder pflanzliche Zellen aus der Gruppe der eukaryontischen Wirte.

Ganz besonders bevorzugt sind bifunktionelle Vektoren, die in der Lage sind, sowohl in B. thuringiensis- und/oder B. cereus-Zellen als auch in E. coli zu replizieren.

Umfasst von der vorliegenden Erfindung ist somit die Verwendung besagter bifunktioneller Vektoren zur Transformation von B. thuringiensis und B. cereus.

Die Konstruktion von 'shuttle'-Vektoren erfolgt mit Hilfe der rekombinanten DNA Technologie, wobei Plasmid und/oder Vektor-DNA homologen (B. thuringiensis, B. cereus) sowie heterologen Ursprungs zunächst mit Hilfe von geeigneten Restriktionsenzymen geschnitten wird und anschliessend diejenigen DNA-Fragmente, welche die für die Replikation in dem jeweiligen gewünschten Wirtssystem essentiellen Funktionen enthalten, in Gegenwart geeigneter Enzyme wieder miteinander verknüpft werden.

Als Quelle für Plasmid- und/oder Vektor DNA-heterologen Ursprungs können die zuvor genannten heterologen Wirtsorganismen dienen.

Die Verknüpfung der verschiedenen DNA-Fragmente muss in einer Weise erfolgen, dass die für die Replikation in den verschiedenen Wirtssystemen essentiellen Funktionen erhalten bleiben.

Daneben kann selbstverständlich auch Plasmid- und/oder Vektor-DNA rein heterologen Ursprungs für die Konstruktion von 'shuttle'-Vektoren verwendet werden, wobei aber zumindest einer der heterologen Fusionspartner DNA-Abschnitte enthalten muss, die eine Replikation im homologen B.thuringiensis/B.cereus Wirtssystem ermöglichen.

Als Quelle von Plasmid- und/oder Vektor-DNA heterolgen Ursprungs, die dennoch in der Lage ist im B.thuringiensis/B.cereus Wirtssystem zu replizieren, seien an dieser Stelle beispielhaft einige Vertreter aus der Gruppe der gram positiven Bakterien genannt, ausgewählt aus der Gruppe bestehend aus den Gattungen Staphylococcus, wie z.B. Staphylococcus aureus, Streptococcus wie z.B. Streptococcus faecalis, Bacillus, wie z.B. Bacillus megaterium oder B. subtilis, Streptomyces, wie z.B. Streptomyces spp. etc. Neben den hier beispielhaft aufgezählten Vertretern aus der Gruppe der gram positven Bakterien, gibt es eine ganze Reihe weiterer Organismen, die in dem erfindungsgemässen Verfahren verwendet werden können und die dem Fachmann bekannt sind.

Verfahren zur Herstellung bifunktioneller Vektoren die für die Transformation von B. thuringiensis und/oder B. cereus geeignet sind, sind dadurch kennzeichnet, dass man Plasmid DNA homologen sowie heterologen Ursprungs zunächst
a) mit Hilfe geeigneter Restriktionsenzyme in Fragmente zerlegt und
b) anschliessend diejenigen Fragmente, welche die für die Replikation sowie die Selektionierung in dem jeweiligen gewünschten Wirtssystem essentiellen Funktionen enthalten, in Gegenwart geeigneter Enzyme wieder miteinander verknüpft und zwar in einer Weise, dass die für die Replikation und Selektion in den verschiedenen Wirtssystemen essentiellen Funktionen erhalten bleiben.

Auf diese Weise entstehen bifunktionelle Plasmide, die neben den für eine Replikation in B. thuringiensis oder B. cereus notwendigen Funktionen weitere DNA-Sequenzen enthalten, die die Replikation in zumindest einem weiteren heterologen Wirtssystem sicherstellen.

Um eine schnelle und effiziente Selektionierung der bifunktionellen Vektoren sowohl in homologen als auch in heterologen Wirtssystem(en) zu gewährleisten, ist es vorteilhaft, besagte Vektoren mit spezifischen Selektionsmarkern zu versehen, die sowohl in B. thuringiensis und/oder B. cereus als auch in den heterologen Wirtssystem(en) brauchbar sind, d.h. eine schnelle und unkomplizierte Selektionierung ermöglichen. Besonders bevorzugt im Rahmen dieser Erfindung ist die Verwendung Antibiotikaresistenzen kodierender DNA-Sequenzen, insbesondere von DNA-Sequenzen, die für Resistenzen gegenüber Antibiotika ausgewählt aus der Gruppe bestehend aus Kanamycin, Tetracyclin, Chloramphenicol, Erythromycin u.a. kodieren.

Ebenfalls bevorzugt sind Gene, die Enzyme mit einem chromogenen Substrat, wie z.B. X-gal (5-Brom-4-chlor-3-indolyl-β-D-galaktosid), kodieren. Die transformierten Kolonien können dann sehr einfach anhand einer spezifischen Farbreaktion nachgewiesen werden.

Andere phänotypische Markergene sind dem Fachmann bekannt und können ebenfalls im Rahmen dieser Erfindung verwendet werden.

Besonders bevorzugt ist die Konstruktion von 'shuttle'-Vektoren, die neben DNA Sequenzen, die eine Replikation in B. thuringiensis oder B. cereus oder in beiden Wirtssystemen erlauben, auch solche DNA-Abschnitte enthalten, die für eine Replikation in weiteren bakteriellen Wirtssystemen, wie beispielsweise in B. subtilis, B. megaterium, Pseudomonas spp., E. coli etc. notwendig sind.

Ebenfalls bevorzugt sind 'shuttle' Vektoren, die einerseits sowohl in B. thuringiensis oder B. cereus oder in beiden replizieren, als auch andererseits in eukaryontischen Wirtsystemen ausgewählt aus der Gruppe bestehend aus Hefe, tierischen und pflanzlichen Zellen u.a.

Ganz besonders bevorzugt ist die Konstruktion von 'shuttle'-Vektoren, die neben DNA-Sequenzen, die für die Replikation besagter Vektoren in B. thuringiensis oder B. cereus oder in beiden Systemen notwendig sind, auch solche DNA-Sequenzen enthalten, die eine Replikation besagter 'shuttle'-Vektoren in E. coli ermöglichen.

Beispiele solcher Ausgangsplasmide für die Konstruktion von 'shuttle'-Vektoren für das B. thuringiensis-B. cereus/E. coli System, die aber in keiner Weise als limitierend angesehen werden dürfen, sind das B. cereus Plasmid pBC16 sowie das vom E. coli Plasmid pBR322 abgeleitete Plasmid pUC8 (²⁸⁾Vieira J und Messing J, 1982).

Zu erwähnen sind auch bifunktionelle ('shuttle') Vektoren, die neben den für die Replikation und Selektion in homologen und heterologen Wirtssystemen essentliellen Funktionen zusätzlich noch ein oder mehrer Gene in exprimierbarer Form oder sonstige nützliche DNA Sequenzen enthalten. Die Herstellung dieser Vektoren, ist dadurch gekennzeichnet sind, dass man besagte Gene oder sonstigen nützlichen DNA-Sequenzen mit Hilfe geeigneter Enzyme in diese bifunktionellen Vektoren einspleisst.

Mit Hilfe besagten 'shuttle'-Vektoren sowie des zuvor beschriebenen Transformationsverfahrens ist es somit jetzt erstmals möglich, ausserhalb von B. thuringiensis-Zellen, in einem fremden Wirts-system klonierte DNA-Sequenzen mit hoher Effizienz in B. thuringiensis und/oder B. cereus Zellen zu transformieren.

Damit können jetzt erstmals Gene oder sonstige nützliche DNA Sequenzen, insbesondere auch solche mit regulatorischer Funktion, stabil in B. thuringiensis- bzw. B. cereus-Zellen eingebracht und dort gegebenenfalls exprimiert werden, wodurch B. thuringiensis- bzw. B. cereus-Zellen mit neuen und wünschenswerten Eigenschaften entstehen.

Als Gene, die im Rahmen der vorliegenden Erfindung Verwendung finden können, kommen sowohl homologe als auch heterologe Gen(e) oder DNA sowie synthetische Gen(e) oder DNA gemäss der im Rahmen der vorliegenden Erfindung gemachten Definition in Betracht, sowie Kombinationen besagter DNA's.

Die kodierende DNA-Sequenz kann dabei ausschliesslich aus genomischer, aus cDNA bzw. synthetischer DNA konstruiert sein. Eine andere Möglichkeit besteht in der Konstruktion einer hybriden DNA-Sequenz bestehend sowohl aus cDNA als auch aus genomischer DNA und synthetischer DNA oder aber aus einer Kombination dieser DNA's.

In diesem Fall kann die cDNA aus demselben Gen stammen wie die genomische DNA oder aber, sowohl die cDNA, wie auch die genomische DNA können aus verschiedenen Genen stammen. In jedem Fall können aber, sowohl die genomischen DNA und/oder die cDNA, jede für sich, aus dem gleichen oder aus verschiedenen Genen hergestellt sein.

Wenn die DNA-Sequenz Anteile von mehr als einem Gen beinhaltet, können diese Gene entweder von ein und demselben Organismus, von mehreren Organismen, die verschiedenen Stämmen, oder Varietäten derselben Art oder verschiedenen Spezies derselben Gattung angehören, oder aber von Organismen, die mehr als einer Gattung derselben oder einer anderen taxonomischen Einheit angehören, abstammen.

Um die Expression besagter Strukturgene in der bakteriellen Zelle zu gewährleisten, müssen die kodierenden Gensequenzen zunächst in operabler Weise mit in B. thuringiensis und/oder B. cereus-Zellen funktionsfähigen Expressions-Sequenzen verknüpft werden.

Die hybriden Genkonstruktionen im Rahmen der vorliegenden Erfindung enthalten somit neben dem (den) Strukturgen(en) Expressions-Signale, die sowohl Promotor- und Terminator-Sequenzen als auch weitere regulatorische Sequenzen der 3' und 5' nicht translatierten Regionen miteinschliessen.

Besonders bevorzugt im Rahmen dieser Erfindung sind die natürlichen Expressionssignale aus B. thuringiensis und/oder B. cereus selbst sowie Mutanten und Varianten davon, die im wesentlichen der natürlichen Sequenz homolog sind. Im Rahmen dieser Erfindung ist eine DNA-Sequenz einer zweiten DNA-Sequenz dann im wesentlichen homolog, wenn wenigstens 70 %, vorzugsweise wenigstens 80 %, insbesondere aber wenigstens 90 % der aktiven Abschnitte der DNA-Sequenz homolog sind. Gemäss vorliegender Definition des Begriffs "im wesentlichen homolog" werden zwei unterschiedliche Nukleotide in einer DNA-Sequenz einer kodierenden Region dann als homolog angesehen, wenn der Austausch des einen Nukleotids gegen das andere eine stille Mutation darstellt.

Ganz besonders bevorzugt ist die Verwendung von Sporulations-abhängigen Promotoren aus B. thuringiensis, die eine Expression in Abhängigkeit der Sporulation gewährleisten.

Besonders bevorzugt für die Transformation von B. thuringiensis bzw. B. cereus im Rahmen dieser Erfindung ist die Verwendung von DNA-Sequenzen, die für ein δ-Endotoxin kodieren.

Die kodierende Region des chimären Gens enthält vorzugsweise eine Nukleotidsequenz, die ein Polypeptid kodiert, welches natürlicherweise in B. thuringiensis vorkommt oder aber ein Polypeptid, das diesem im wesentlichen homolog ist, d.h. das zumindest im wesentlichen die Toxizitätseigenschaften eines kristallinen δ-Endotoxin Proteins von B. thuringiensis aufweist. Im Rahmen der vorliegenden Erfindung hat ein Polypeptid definitionsgemäss dann im wesentlichen die Toxizitätseigenschaften des kristallinen δ-Endotoxin Proteins von B. thuringiensis, wenn es gegen ein ähnliches Spektrum von Insekten-Larven insektizid wirkt wie das kristalline Protein einer Unterart von B. thuringiensis. Einige geeignete Unterarten sind beispielsweise solche, ausgewählt aus der Gruppe bestehend aus kurstaki, berliner, alesti, tolworthi, sotto, dendrolimus, tenebrionis und israelensis. Die für Lepidoptera-Larven bevorzugte Unterart ist kurstaki und insbesondere kurstaki HD1.

Bei der kodierenden Region kann es sich um eine Region handeln, die natürlicherweise in B. thuringiensis vorkommt. Alternativ dazu kann die kodierende Region gegebenenfalls aber auch eine Sequenz enthalten, die sich von der Sequenz in B. thuringiensis unterscheidet, die ihr aber aufgrund der Degenerierung des genetischen Kodes äquivalent ist.

Die kodierende Region des chimären Gens kann auch ein Polypeptid kodieren, das sich von einem natürlich vorkommenden kristallinen δ-Endotoxin Protein unterscheidet, das aber immer noch im wesentlichen die Insektentoxizitätseigenschaften des kristallinen Proteins hat. Solch eine kodierende Sequenz wird gewöhnlicherweise eine Variante einer natürlichen kodierenden Region sein. Unter einer "Variante" einer natürlichen DNA-Sequenz ist im Rahmen dieser Erfindung definitionsgemäss eine modifizierte Form einer natürlichen Sequenz zu verstehen, die aber noch dieselbe Funktion erfüllt. Die Variante kann eine Mutante oder eine synthetische DNA-Sequenz sein und ist im wesentlichen der entsprechenden natürlichen Sequenz homolog. Im Rahmen dieser Erfindung ist eine DNA-Sequenz einer zweiten DNA-Sequenz dann im wesentlichen homolog, wenn wenigstens 70 %, vorzugsweise wenigstens 80 %, insbesondere aber wenigstens 90 % der aktiven Abschnitte der DNA-Sequenz homolog sind. Gemäss vorliegender Definition des Begriffs "im wesentlichen homolog" werden zwei unterschiedliche Nukleotide in einer DNA-Sequenz einer kodierenden Region dann als homolog angesehen, wenn der Austausch des einen Nukleotids gegen das andere eine stille Mutation darstellt.

Im Rahmen der vorliegenden Erfindung kann somit jedes, eine Aminosäuresequenz-kodierende, chimäre Gen verwendet werden, das die insektiziden Eigenschaften eines B. thuringiensis δ-Endotoxins aufweist und welches die offenbarten und beanspruchten Erfordernisse erfüllt. Besonders bevorzugt ist die Verwendung einer Nukleotidsequenz, die im wesentlichen wenigstens dem Teil oder den Teilen der natürlichen Sequenz homolog ist, die für die insektizide Aktivität und/oder die Wirtsspezifität des B. thuringiensis Toxins verantwortlich ist (sind).

Das durch das chimäre Gen exprimierte Polypeptid hat in der Regel auch wenigstens einige immunologische Eigenschaften mit einem natürlichen kristallinen Protein gemeinsam, weil es wenigstens zum Teil die gleichen antigenen Determinanten besitzt.

Dementsprechend ist das Polypeptid, das von besagtem chimären Gen kodiert wird, vorzugsweise strukturell mit dem δ-Endotoxin des von B. thuringiensis produzierten kristallinen Proteins verwandt. B. thuringiensis produziert ein kristallines Protein mit einer Untereinheit, die einem Protoxin mit einem Molekulargewicht (MG) von etwa 130 000 bis 140 000 entspricht. Diese Untereinheit kann durch Proteasen oder durch Alkali in insektizide Fragmente mit einem MG von 70.000 und möglicherweise sogar weniger gespalten werden.

Für die Konstruktion chimärer Gene, deren kodierender Abschnitt solche Fragmente des Protoxins oder noch kleinere Teile davon umfasst, kann die Fragmentierung der kodierenden Region solange fortschreiten, wie die Fragmente oder Teile dieser Fragmente noch die erforderliche insektizide Aktivität aufweisen. Das Protoxin, insektizide Fragmente des Protoxins und insektizide Teile dieser Fragmente können mit anderen Molekülen, wie Polypeptiden und Proteinen, verbunden werden.

Kodierende Regionen, die für eine Verwendung im Rahmen des erfindungsgemässen Verfahrens geeignet sind, können von Genen aus B. thuringiensis, die das kristalline Toxingen kodieren, erhalten werden (Whiteley et al, PCT Anmeldung WO86/01536 und US Patente 4 448 885 und 4 467 036). Eine bevorzugte Nukleotidsequenz, die ein kristallines Protein kodiert, befindet sich zwischen den Nukleotiden 156 und 3623 in Formel I oder ist eine kürzere Sequenz, die ein insektizides Fragment solch eines kristallinen Proteins kodiert (⁵⁾Geiser et al., 1986 und EP 238,441).

Formel I

10 20 30 40 50 60
GTTAACACCC TGGGTCAAAA ATTGATATTT AGTAAAATTA GTTGCACTTT GTGCATTTTT

70 80 90 100 110 120
TCATAAGATG AGTCATATGT TTTAAATTGT AGTAATGAAA AACAGTATTA TATCATAATG

130 140 150 160 170 180
AATTGGTATC TTAATAAAAG AGATGGAGGT AACTTATGGA TAACAATCCG AACATCAATG

190 200 210 220 230 240
AATGCATTCC TTATAATTGT TTAAGTAACC CTGAAGTAGA AGTATTAGGT GGAGAAAGAA

250 260 270 280 290 300
TAGAAACTGG TTACACCCCA ATCGATATTT CCTTGTCGCT AACGCAATTT CTTTTGAGTG

310 320 330 340 350 360
AATTTGTTCC CGGTGCTGGA TTTGTGTTAG GACTAGTTGA TATAATATGG GGAATTTTTG

370 380 390 400 410 420
GTCCCTCTCA ATGGGACGCA TTTCTTGTAC AAATTGAACA GTTAATTAAC CAAAGAATAG

430 440 450 460 470 480
AAGAATTCGC TAGGAACCAA GCCATTTCTA GATTAGAAGG ACTAAGCAAT CTTTATCAAA

490 500 510 520 530 540
TTTACGCAGA ATCTTTTAGA GAGTGGGAAG CAGATCCTAC TAATCCAGCA TTAAGAGAAG

550 560 570 580 590 600
AGATGCGTAT TCAATTCAAT GACATGAACA GTGCCCTTAC AACCGCTATT CCTCTTTTTG

610 620 630 640 650 660
CAGTTCAAAA TTATCAAGTT CCTCTTTTAT CAGTATATGT TCAAGCTGCA AATTTACATT

670 680 690 700 710 720
TATCAGTTTT GAGAGATGTT TCAGTGTTTG GACAAAGGTG GGGATTTGAT GCCGCGACTA

730 740 750 760 770 780
TCAATAGTCG TTATAATGAT TTAACTAGGC TTATTGGCAA CTATACAGAT CATGCTGTAC

790 800 810 820 830 840
GCTGGTACAA TACGGGATTA GAGCGTGTAT GGGGACCGGA TTCTAGAGAT TGGATAAGAT

850 860 870 880 890 900
ATAATCAATT TAGAAGAGAA TTAACACTAA CTGTATTAGA TATCGTTTCT CTATTTCCGA

910 920 930 940 950 960
ACTATGATAG TAGAACGTAT CCAATTCGAA CAGTTTCCCA ATTAACAAGA GAAATTTATA

970 980 990 1000 1010 1020
CAAACCCAGT ATTAGAAAAT TTTGATGGTA GTTTTCGAGG CTCGGCTCAG GGCATAGAAG

1030 1040 1050 1060 1070 1080
GAAGTATTAG GAGTCCACAT TTGATGGATA TACTTAACAG TATAACCATC TATACGGATG

1090 1100 1110 1120 1130 1140
CTCATAGAGG AGAATATTAT TGGTCAGGGC ATCAAATAAT GGCTTCTCCT GTAGGGTTTT

1150 1160 1170 1180 1190 1200
CGGGGCCAGA ATTCACTTTT CCGCTATATG GAACTATGGG AAATGCAGCT CCACAACAAC

1210 1220 1230 1240 1250 1260
GTATTGTTGC TCAACTAGGT CAGGGCGTGT ATAGAACATT ATCGTCCACT TTATATAGAA

1270 1280 1290 1300 1310 1320
GACCTTTTAA TATAGGGATA AATAATCAAC AACTATCTGT TCTTGACGGG ACAGAATTTG

1330 1340 1350 1360 1370 1380
CTTATGGAAC CTCCTCAAAT TTGCCATCCG CTGTATACAG AAAAAGCGGA ACGGTAGATT

1390 1400 1410 1420 1430 1440
CGCTGGATGA AATACCGCCA CAGAATAACA ACGTGCCACC TAGGCAAGGA TTTAGTCATC

1450 1460 1470 1480 1490 1500
GATTAAGCCA TGTTTCAATG TTTCGTTCAG GCTTTAGTAA TAGTAGTGTA AGTATAATAA

1510 1520 1530 1540 1550 1560
GAGCTCCTAT GTTCTCTTGG ATACATCGTA GTGCTGAATT TAATAATATA ATTCCTTCAT

1570 1580 1590 1600 1610 1620
CACAAATTAC ACAAATACCT TTAACAAAAT CTACTAATCT TGGCTCTGGA ACTTCTGTCG

1630 1640 1650 1660 1670 1680
TTAAAGGACC AGGATTTACA GGAGGAGATA TTCTTCGAAG AACTTCACCT GGCCAGATTT

1690 1700 1710 1720 1730 1740
CAACCTTAAG AGTAAATATT ACTGCACCAT TATCACAAAG ATATCGGGTA AGAATTCGCT

1750 1760 1770 1780 1790 1800
ACGCTTCTAC CACAAATTTA CAATTCCATA CATCAATTGA CGGAAGACCT ATTAATCAGG

1810 1820 1830 1840 1850 1860
GGAATTTTTC AGCAACTATG AGTAGTGGGA GTAATTTACA GTCCGGAAGC TTTAGGACTG

1870 1880 1890 1900 1910 1920
TAGGTTTTAC TACTCCGTTT AACTTTTCAA ATGGATCAAG TGTATTTACG TTAAGTGCTC

1930 1940 1950 1960 1970 1980
ATGTCTTCAA TTCAGGCAAT GAAGTTTATA TAGATCGAAT TGAATTTGTT CCGGCAGAAG

1990 2000 2010 2020 2030 2040
TAACCTTTGA GGCAGAATAT GATTTAGAAA GAGCACAAAA GGCGGTGAAT GAGCTGTTTA

2050 2060 2070 2080 2090 2100
CTTCTTCCAA TCAAATCGGG TTAAAAACAG ATGTGACGGA TTATCATATT GATCAAGTAT

2110 2120 2130 2140 2150 2160
CCAATTTAGT TGAGTGTTTA TCTGATGAAT TTTGTCTGGA TGAAAAAAAA GAATTGTCCG

2170 2180 2190 2200 2210 2220
AGAAAGTCAA ACATGCGAAG CGACTTAGTG ATGAGCGGAA TTTACTTCAA GATCCAAACT

2230 2240 2250 2260 2270 2280
TTAGAGGGAT CAATAGACAA CTAGACCGTG GCTGGAGAGG AAGTACGGAT ATTACCATCC

2290 2300 2310 2320 2330 2340
AAGGAGGCGA TGACGTATTC AAAGAGAATT ACGTTACGCT ATTGGGTACC TTTGATGAGT

2350 2360 2370 2380 2390 2400
GCTATCCAAC GTATTTATAT CAAAAAATAG ATGAGTCGAA ATTAAAAGCC TATACCCGTT

2410 2420 2430 2440 2450 2460
ACCAATTAAG AGGGTATATC GAAGATAGTC AAGACTTAGA AATCTATTTA ATTCGCTACA

2470 2480 2490 2500 2510 2520
ATGCCAAACA CGAAACAGTA AATGTGCCAG GTACGGGTTC CTTATGGCCG CTTTCAGCCC

2530 2540 2550 2560 2570 2580
CAAGTCCAAT CGGAAAATGT GCCCATCATT CCCATCATTT CTCCTTGGAC ATTGATGTTG

2590 2600 2610 2620 2630 2640
GATGTACAGA CTTAAATGAG GACTTAGGTG TATGGGTGAT ATTCAAGATT AAGACGCAAG

2650 2660 2670 2680 2690 2700
ATGGCCATGC AAGACTAGGA AATCTAGAAT TTCTCGAAGA GAAACCATTA GTAGGAGAAG

2710 2720 2730 2740 2750 2760
CACTAGCTCG TGTGAAAAGA GCGGAGAAAA AATGGAGAGA CAAACGTGAA AAATTGGAAT

2770 2780 2790 2800 2810 2820
GGGAAACAAA TATTGTTTAT AAAGAGGCAA AAGAATCTGT AGATGCTTTA TTTGTAAACT

2830 2840 2850 2860 2870 2880
CTCAATATGA TAGATTACAA GCGGATACCA ACATCGCGAT GATTCATGCG GCAGATAAAC

2890 2900 2910 2920 2930 2940
GCGTTCATAG CATTCGAGAA GCTTATCTGC CTGAGCTGTC TGTGATTCCG GGTGTCAATG

2950 2960 2970 2980 2990 3000
CGGCTATTTT TGAAGAATTA GAAGGGCGTA TTTTCACTGC ATTCTCCCTA TATGATGCGA

3010 3020 3030 3040 3050 3060
GAAATGTCAT TAAAAATGGT GATTTTAATA ATGGCTTATC CTGCTGGAAC GTGAAAGGGC

3070 3080 3090 3100 3110 3120
ATGTAGATGT AGAAGAACAA AACAACCACC GTTCGGTCCT TGTTGTTCCG GAATGGGAAG

3130 3140 3150 3160 3170 3180
CAGAAGTGTC ACAAGAAGTT CGTGTCTGTC CGGGTCGTGG CTATATCCTT CGTGTCACAG

3190 3200 3210 3220 3230 3240
CGTACAAGGA GGGATATGGA GAAGGTTGCG TAACCATTCA TGAGATCGAG AACAATACAG

3250 3260 3270 3280 3290 3300
ACGAACTGAA GTTTAGCAAC TGTGTAGAAG AGGAAGTATA TCCAAACAAC ACGGTAACGT

3310 3320 3330 3340 3350 3360
GTAATGATTA TACTGCGACT CAAGAAGAAT ATGAGGGTAC GTACACTTCT CGTAATCGAG

3370 3380 3390 3400 3410 3420
GATATGACGG AGCCTATGAA AGCAATTCTT CTGTACCAGC TGATTATGCA TCAGCCTATG

3430 3440 3450 3460 3470 3480
AAGAAAAAGC ATATACAGAT GGACGAAGAG ACAATCCTTG TGAATCTAAC AGAGGATATG

3490 3500 3510 3520 3530 3540
GGGATTACAC ACCACTACCA GCTGGCTATG TGACAAAAGA ATTAGAGTAC TTCCCAGAAA

3550 3560 3570 3580 3590 3600
CCGATAAGGT ATGGATTGAG ATCGGAGAAA CGGAAGGAAC ATTCATCGTG GACAGCGTGG

3610 3620 3630 3640 3650 3660
AATTACTTCT TATGGAGGAA TAATATATGC TTTATAATGT AAGGTGTGCA AATAAAGAAT

3670 3680 3690 3700 3710 3720
GATTACTGAC TTGTATTGAC AGATAAATAA GGAAATTTTT ATATGAATAA AAAACGGGCA

3730 3740 3750 3760 3770 3780
TCACTCTTAA AAGAATGATG TCCGTTTTTT GTATGATTTA ACGAGTGATA TTTAAATGTT

3790 3800 3810 3820 3830 3840
TTTTTTGCGA AGGCTTTACT TAACGGGGTA CCGCCACATG CCCATCAACT TAAGAATTTG

3850 3860 3870 3880 3890 3900
CACTACCCCC AAGTGTCAAA AAACGTTATT CTTTCTAAAA AGCTAGCTAG AAAGGATGAC

3910 3920 3930 3940 3950 3960
ATTTTTTATG AATCTTTCAA TTCAAGATGA ATTACAACTA TTTTCTGAAG AGCTGTATCG

3970 3980 3990 4000 4010 4020
TCATTTAACC CCTTCTCTTT TGGAAGAACT CGCTAAAGAA TTAGGTTTTG TAAAAAGAAA

4030 4040 4050 4060 4070 4080
ACGAAAGTTT TCAGGAAATG AATTAGCTAC CATATGTATC TGGGGCAGTC AACGTACAGC

4090 4100 4110 4120 4130 4140
GAGTGATTCT CTCGTTCGAC TATGCAGTCA ATTACACGCC GCCACAGCAC TCTTATGAGT

4150 4160 4170 4180 4190 4200
CCAGAAGGAC TCAATAAACG CTTTGATAAA AAAGCGGTTG AATTTTTGAA ATATATTTTT

4210 4220 4230 4240 4250 4260
TCTGCATTAT GGAAAAGTAA ACTTTGTAAA ACATCAGCCA TTTCAAGTGC AGCACTCACG

4270 4280 4290 4300 4310 4320
TATTTTCAAC GAATCCGTAT TTTAGATGCG ACGATTTTCC AAGTACCGAA ACATTTAGCA

4330 4340 4350 4360
CATGTATATC CTGGGTCAGG TGGTTGTGCA CAAACTGCAG

Die durch die Nukleotide 156 bis 3623 von Formel I definierte kodierende Region kodiert ein Polypeptid der Formel II.

Formel II

Met Asp Asn Asn Pro Asn Ile Asn Glu Cys 10
Ile Pro Tyr Asn Cys Leu Ser Asn Pro Glu 20
Val Glu Val Leu Gly Gly Glu Arg Ile Glu 30
Thr Gly Tyr Thr Pro Ile Asp Ile Ser Leu 40
Ser Leu Thr Gln Phe Leu Leu Ser Glu Phe 50
Val Pro Gly Ala Gly Phe Val Leu Gly Leu 60
Val Asp Ile Ile Trp Gly Ile Phe Gly Pro 70
Ser Gln Trp Asp Ala Phe Leu Val Gln Ile 80
Glu Gln Leu Ile Asn Gln Arg Ile Glu Glu 90
Phe Ala Arg Asn Gln Ala Ile Ser Arg Leu 100
Glu Gly Leu Ser Asn Leu Tyr Gln Ile Tyr 110
Ala Glu Ser Phe Arg Glu Trp Glu Ala Asp 120
Pro Thr Asn Pro Ala Leu Arg Glu Glu Met 130
Arg Ile Gln Phe Asn Asp Met Asn Ser Ala 140
Leu Thr Thr Ala Ile Pro Leu Phe Ala Val 150
Gln Asn Tyr Gln Val Pro Leu Leu Ser Val 160
Tyr Val Gln Ala Ala Asn Leu His Leu Ser 170
Val Leu Arg Asp Val Ser Val Phe Gly Gln 180
Arg Trp Gly Phe Asp Ala Ala Thr Ile Asn 190
Ser Arg Tyr Asn Asp Leu Thr Arg Leu Ile 200
Gly Asn Tyr Thr Asp His Ala Val Arg Trp 210
Tyr Asn Thr Gly Leu Glu Arg Val Trp Gly 220
Pro Asp Ser Arg Asp Trp Ile Arg Tyr Asn 230
Gln Phe Arg Arg Glu Leu Thr Leu Thr Val 240
Leu Asp Ile Val Ser Leu Phe Pro Asn Tyr 250
Asp Ser Arg Thr Tyr Pro Ile Arg Thr Val 260
Ser Gln Leu Thr Arg Glu Ile Tyr Thr Asn 270
Pro Val Leu Glu Asn Phe Asp Gly Ser Phe 280
Arg Gly Ser Ala Gln Gly Ile Glu Gly Ser 290
Ile Arg Ser Pro His Leu Met Asp Ile Leu 300

Asn Ser Ile Thr Ile Tyr Thr Asp Ala His 310
Arg Gly Glu Tyr Tyr Trp Ser Gly His Gln 320
Ile Met Ala Ser Pro Val Gly Phe Ser Gly 330
Pro Glu Phe Thr Phe Pro Leu Tyr Gly Thr 340
Met Gly Asn Ala Ala Pro Gln Gln Arg Ile 350
Val Ala Gln Leu Gly Gln Gly Val Tyr Arg 360
Thr Leu Ser Ser Thr Leu Tyr Arg Arg Pro 370
Phe Asn Ile Gly Ile Asn Asn Gln Gln Leu 380
Ser Val Leu Asp Gly Thr Glu Phe Ala Tyr 390
Gly Thr Ser Ser Asn Leu Pro Ser Ala Val 400
Tyr Arg Lys Ser Gly Thr Val Asp Ser Leu 410
Asp Glu Ile Pro Pro Gln Asn Asn Asn Val 420
Pro Pro Arg Gln Gly Phe Ser His Arg Leu 430
Ser His Val Ser Met Phe Arg Ser Gly Phe 440
Ser Asn Ser Ser Val Ser Ile Ile Arg Ala 450
Pro Met Phe Ser Trp Ile His Arg Ser Ala 460
Glu Phe Asn Asn Ile Ile Pro Ser Ser Gln 470
Ile Thr Gln Ile Pro Leu Thr Lys Ser Thr 480
Asn Leu Gly Ser Gly Thr Ser Val Val Lys 490
Gly Pro Gly Phe Thr Gly Gly Asp Ile Leu 500
Arg Arg Thr Ser Pro Gly Gln Ile Ser Thr 510
Leu Arg Val Asn Ile Thr Ala Pro Leu Ser 520
Gln Arg Tyr Arg Val Arg Ile Arg Tyr Ala 530
Ser Thr Thr Asn Leu Gln Phe His Thr Ser 540
Ile Asp Gly Arg Pro Ile Asn Gln Gly Asn 550
Phe Ser Ala Thr Met Ser Ser Gly Ser Asn 560
Leu Gln Ser Gly Ser Phe Arg Thr Val Gly 570
Phe Thr Thr Pro Phe Asn Phe Ser Asn Gly 580
Ser Ser Val Phe Thr Leu Ser Ala His Val 590
Phe Asn Ser Gly Asn Glu Val Tyr Ile Asp 600
Arg Ile Glu Phe Val Pro Ala Glu Val Thr 610
Phe Glu Ala Glu Tyr Asp Leu Glu Arg Ala 620
Gln Lys Ala Val Asn Glu Leu Phe Thr Ser 630
Ser Asn Gln Ile Gly Leu Lys Thr Asp Val 640
Thr Asp Tyr His Ile Asp Gln Val Ser Asn 650
Leu Val Glu Cys Leu Ser Asp Glu Phe Cys 660
Leu Asp Glu Lys Lys Glu Leu Ser Glu Lys 670

Val Lys His Ala Lys Arg Leu Ser Asp Glu 680
Arg Asn Leu Leu Gln Asp Pro Asn Phe Arg 690
Gly Ile Asn Arg Gln Leu Asp Arg Gly Trp 700
Arg Gly Ser Thr Asp Ile Thr Ile Gln Gly 710
Gly Asp Asp Val Phe Lys Glu Asn Tyr Val 720
Thr Leu Leu Gly Thr Phe Asp Glu Cys Tyr 730
Pro Thr Tyr Leu Tyr Gln Lys Ile Asp Glu 740
Ser Lys Leu Lys Ala Tyr Thr Arg Tyr Gln 750
Leu Arg Gly Tyr Ile Glu Asp Ser Gln Asp 760
Leu Glu Ile Tyr Leu Ile Arg Tyr Asn Ala 770
Lys His Glu Thr Val Asn Val Pro Gly Thr 780
Gly Ser Leu Trp Pro Leu Ser Ala Pro Ser 790
Pro Ile Gly Lys Cys Ala His His Ser His 800
His Phe Ser Leu Asp Ile Asp Val Gly Cys 810
Thr Asp Leu Asn Glu Asp Leu Gly Val Trp 820
Val Ile Phe Lys Ile Lys Thr Gln Asp Gly 830
His Ala Arg Leu Gly Asn Leu Glu Phe Leu 840
Glu Glu Lys Pro Leu Val Gly Glu Ala Leu 850
Ala Arg Val Lys Arg Ala Glu Lys Lys Trp 860
Arg Asp Lys Arg Glu Lys Leu Glu Trp Glu 870
Thr Asn Ile Val Tyr Lys Glu Ala Lys Glu 880
Ser Val Asp Ala Leu Phe Val Asn Ser Gln 890
Tyr Asp Arg Leu Gln Ala Asp Thr Asn Ile 900
Ala Met Ile His Ala Ala Asp Lys Arg Val 910
His Ser Ile Arg Glu Ala Tyr Leu Pro Glu 920
Leu Ser Val Ile Pro Gly Val Asn Ala Ala 930
Ile Phe Glu Glu Leu Glu Gly Arg Ile Phe 940
Thr Ala Phe Ser Leu Tyr Asp Ala Arg Asn 950
Val Ile Lys Asn Gly Asp Phe Asn Asn Gly 960
Leu Ser Cys Trp Asn Val Lys Gly His Val 970
Asp Val Glu Glu Gln Asn Asn His Arg Ser 980
Val Leu Val Val Pro Glu Trp Glu Ala Glu 990
Val Ser Gln Glu Val Arg Val Cys Pro Gly 1000
Arg Gly Tyr Ile Leu Arg Val Thr Ala Tyr 1010
Lys Glu Gly Tyr Gly Glu Gly Cys Val Thr 1020
Ile His Glu Ile Glu Asn Asn Thr Asp Glu 1030
Leu Lys Phe Ser Asn Cys Val Glu Glu Glu 1040

Val Tyr Pro Asn Asn Thr Val Thr Cys Asn 1050
Asp Tyr Thr Ala Thr Gln Glu Glu Tyr Glu 1060
Gly Thr Tyr Thr Ser Arg Asn Arg Gly Tyr 1070
Asp Gly Ala Tyr Glu Ser Asn Ser Ser Val 1080
Pro Ala Asp Tyr Ala Ser Ala Tyr Glu Glu 1090
Lys Ala Tyr Thr Asp Gly Arg Arg Asp Asn 1100
Pro Cys Glu Ser Asn Arg Gly Tyr Gly Asp 1110
Tyr Thr Pro Leu Pro Ala Gly Tyr Val Thr 1120
Lys Glu Leu Glu Tyr Phe Pro Glu Thr Asp 1130
Lys Val Trp Ile Glu Ile Gly Glu Thr Glu 1140
Gly Thr Phe Ile Val Asp Ser Val Glu Leu 1150
Leu Leu Met Glu Glu End 1156

Um ein chimäres Gen mit Hilfe des erfindungsgemässen Verfahrens in B. thuringiensis bzw. B. cereus Zellen zu transformieren, wird das Gen vorzugsweise zuerst in einen Vektor eingebaut. Besonders bevorzugt ist der Einbau in die zuvor erwähnten bifunktionellen Vektoren.

Wenn das entsprechende Gen nicht in einer Menge zur Verfügung steht, die für die Einschleusung in die Bacillus-Zellen ausreicht, kann der Vektor zunächst durch Replikation in einer heterologen Wirtszelle amplifiziert werden. Am besten geeignet für die Amplifikation von Genen sind Bakterien- oder Hefezellen. Wenn eine ausreichende Menge des Gens zur Verfügung steht, wird es in die Bacillus-Zellen eingeschleust. Die Einführung des Gens in B. thuringiensis oder B. cereus-Zellen kann mit dem gleichen Vektor, der zur Replikation genutzt wurde, oder mit einem anderen Vektor erfolgen. Besonders geeignet sind die erfindungsgemässen bifunktionellen Vektoren.

Einige Beispiele für bakterielle Wirtszellen, die für eine Replikation des chimären Gens geeignet sind, umfassen Bakterien, ausgewählt aus den Gattungen Escherichia, wie E. coli, Agrobacterium, wie A. tumefaciens oder A. rhizogenes, Pseudomonas, wie Pseudomonas spp., Bacillus, wie B. megaterium oder B. subtilis, etc. Durch das erfindungsgemässe Transformationsverfahren wird es jetzt im Rahmen dieser Erfindung erstmals möglich, auch B. thuringiensis bzw. B. cereus selbst als Wirtszellen einzusetzen. Verfahren der Klonierung heterologer Gene in Bakterien sind in den US Patenten 4,237,224 und 4,468,464 beschrieben.

Die Replikation von Genen in E. coli, die das kristalline Protein von B. thuringiensis kodieren, wird von ²⁹⁾Wong et al. (1983) beschrieben.

Einige Beispiele für Hefewirtszellen, die sich zur Replikation der erfindungsgemässen Gene eignen, schliessen solche, ausgewählt aus der Gattung Saccharomyces, ein (Europäische Patentanmeldung EP 0 238 441).

Für die Amplifikation der erfindungsgemässen Gene kann jeder beliebige Vektor verwendet werden, in den das chimäre Gen eingebaut werden kann und der sich in einer geeigneten Wirtszelle, wie in Bakterien oder Hefe, repliziert. Der Vektor kann beispielsweise von einem Phagen oder einem Plasmid abgeleitet sein. Beispiele für Vektoren, die von Phagen abgeleitet und im Rahmen dieser Erfindung verwendet werden können, sind Vektoren, die von M13- und von λ-Phagen abgeleitet sind. Einige geeignete Vektoren, die von M13-Phagen abgeleitet sind, schliessen M13mp18 und M13mp19 ein. Einige von λ-Phagen abgeleitete geeignete Vektoren schliessen λgt11, λgt7 und λCharon4 ein.

Von den Vektoren, die von Plasmiden abgeleitet und ganz besonders für die Replikation in Bakterien geeignet sind, seien hier beispielhaft pBR322 (³⁰⁾Bolivar et al., 1977), pUC18 und pUC19 (³¹⁾Norrander et al., 1983) sowie Ti-Plasmide (³²⁾Bevan et al., 1983) genannt, ohne dadurch jedoch den Erfindungsgegenstand in irgendeiner Weise zu limitieren. Die bevorzugten Vektoren zur Amplifikation von Genen in Bakterien sind pBR322, pUC18 und pUC19.

Für eine Klonierung direkt in B. thuringiensis und/oder B. cereus sind in erster Linie Direktklonierungsvektoren zu nennen, wie z.B. pBD347, pBD348, pBD64 sowie pUB1664, sowie insbesondere 'shuttle'-Vektoren, die zuvor bereits im Detail beschrieben wurden, ohne jedoch darauf beschränkt zu sein.

Besonders bevorzugt im Rahmen dieser Erfindung sind die bifunktionellen ('shuttle') Vektoren pXI61 (=pK61) und pXI93 (=pK93), die, transformiert in B. thuringiensis var. kurstaki HD1 cryB bzw. B. cereus 569 K, bei der als Internationale Hinterlegungsstelle anerkannten 'Deutschen Sammlung von Mikroorganismen' (Braunschweig, BRD) gemäss den Bestimmungen des Budapester Vertrages unter der Nummer DSM 4573 (pXI61, transformiert in B. thuringiensis var. kurstaki HD1 cryB) bzw. DSM 4571 (pXI93, transformiert in B. thuringiensis var. kurstaki HD1 cryB) und DSM 4573 (pXI93, transformiert in B. cereus 569 K) hinterlegt worden sind.

Um ein für die Replikation in Bakterien geeignetes chimäres Gen zu konstruieren, werden eine Promotorsequenz, eine 5′-nichttranslatierte Sequenz, eine kodierende Sequenz und eine 3′-nichttranslatierte Sequenz in einen Vektor eingefügt oder in der richtigen Reihenfolge in einem der zuvor beschriebenen Vektoren zusammengebaut. Geeignete Vektoren sind erfindungsgemäss solche, die in der Lage sind, sich in der Wirtszelle zu replizieren.

Der Promotor, die 5′-nichttranslatierte Region, die kodierende Region und die 3′-nichttranslatierte Region, können gegebenenfalls zuerst ausserhalb des Vektors in einer Einheit zusammengefasst und dann in den Vektor eingefügt werden. Alternativ dazu können aber auch Teile des chimären Gens einzeln in den Vektor eingefügt werden.

Im Falle der B. thuringiensis bzw. B. cereus Klonierungsvektoren kann dieser Verfahrensschritt entfallen, da die gesamte aus B. thuringiensis isolierte Einheit, bestehend aus einer 5′-nicht-translatierten Region, der kodierenden Region und einer 3′-nicht-translatierten Region, in den Vektor eingespleisst werden kann.

Der Vektor enthält darüberhinaus vorzugsweise auch ein Markergen, welches der Wirtszelle eine Eigenschaft verleiht, durch die man die mit dem Vektor transformierten Zellen erkennen kann. Bevorzugt sind Markergene, die eine Antibiotikaresistenz kodieren. Einige Beispiele für geeignete Antibiotika sind Ampicillin, Chloramphenicol, Erythromycin, Tetrazyklin, Hygromycin, G418 und Kanamycin.

Ebenfalls bevorzugt sind Markergene, die Enzyme mit einem chromogenen Substrat, wie z.B. X-gal (5-Brom-4-chlor-3-indolyl-β-D-galaktosid), kodieren. Die transformierten Kolonien können dann sehr einfach anhand einer spezifischen Farbreaktion nachgewiesen werden.

Die Einfügung oder der Zusammenbau des Gens im Vektor wird mit Hilfe von Standardverfahren durchgeführt, beispielsweise die Verwendung von rekombinanter DNA (³³⁾Maniatis et al., 1982) und die Anwendung der homologen Rekombination (³⁴⁾Hinnen et al., 1978).

Die Verfahren der rekombinanten DNA-Technologie beruhen darauf, dass der Vektor zunächst geschnitten und die gewünschte DNA-Sequenz zwischen die geschnittenen Stücke des Vektors eingefügt wird; die Enden der gewünschten DNA-Sequenz werden anschliessend mit den entsprechenden Enden des Vektors verknüpft.

Der Vektor wird vorzugsweise mit geeigneten Restriktionsendonukleasen geschnitten. Geeignete Restriktionsendonukleasen sind beispielsweise solche, die glatte Enden bilden, wie SmaI, HpaI und EcoRV, sowie solche, die kohäsive Enden bilden, wie EcoRI, SacI und BamHI.

Die gewünschte DNA-Sequenz existiert normalerweise als Teil eines grösseren DNA-Moleküls, wie eines Chromosoms, eines Plasmids, eines Transposons oder eines Phagen. Die gewünschte DNA-Sequenz wird in diesen Fällen aus ihrer ursprünglichen Quelle herausgeschnitten und gegebenenfalls so modifiziert, dass ihre Enden mit denen des geschnittenen Vektors verbunden werden können. Wenn die Enden der gewünschten DNA-Sequenz und des geschnittenen Vektors glatte Enden sind, können sie beispielsweise mit für glatte Enden spezifischen Ligasen, wie der T4 DNA-Ligase, miteinander verbunden werden.

Die Enden der gewünschten DNA-Sequenz können auch in der Form von kohäsiven Enden mit den Enden des geschnittenen Vektors verbunden werden, in welchem Fall eine für kohäsive Enden spezifische Ligase, die auch T4 DNA-Ligase sein kann, benutzt wird. Eine andere geeignete, für kohäsive Enden spezifische Ligase ist beispielsweise die E. coli DNA-Ligase.

Kohäsive Enden werden zweckmässigerweise gebildet, indem die gewünschte DNA-Sequenz und der Vektor mit der gleichen Restriktionsendonuklease geschnitten werden. In diesem Fall haben die gewünschte DNA-Sequenz und der geschnittene Vektor kohäsive Enden, die einander komplementär sind.

Die kohäsiven Enden können auch konstruiert werden, indem mit Hilfe der terminalen Desoxynukleotidyl-Transferase komplementäre, homopolymere Schwänze an die Enden der gewünschten DNA-Sequenz und des geschnittenen Vektors angehängt werden. Alternativ können auch kohäsive Enden hergestellt werden, indem eine synthetische Oligonukleotid-Sequenz, die von einer bestimmten Restriktionsendonuklease erkannt wird und die unter der Bezeichnung Linker bekannt ist, angehängt und die Sequenz mit der Endonuklease gespalten wird (siehe beispielsweise ³³⁾Maniatis et al., 1982).

Damit ist es nunmehr im Rahmen dieser Erfindung erstmals möglich B. thuringiensis-Gene, insbesondere aber δ-Endotoxin-kodierende DNA-Sequenzen, ausserhalb von B. thuringiensis genetisch zu modifizieren, zu klonieren und anschliessend in B. thuringiensis- und/oder B. cereus-Zellen zurückzuführen, wo besagte δ-Endtoxingene (in einem homologen bakteriellen Wirtssystem) exprimiert werden können.

Dies bedeutet, dass nunmehr auch das Genom von B. thuringiensis gezielt genetisch manipuliert werden kann, indem zunächst grosse Mengen an Plasmid-Material in einem fremden Klonierungssystem gewonnen werden und dieses anschliessend in B. thuringiensis transformiert wird.

Von besonderem Interesse ist dabei die Möglichkeit zur Modifikation der δ-Endotoxingene, sowie der die Expression dieser Gene regulierenden Kontrollsequenzen.

Neben chimären Genen kann selbstverständlich auch jede beliebige andere chimäre genetische Konstruktion mit Hilfe des erfindungsgemässen Verfahrens in Bacillus thuringiensis- und/oder Bacillus cereus-Zellen eingeschleust werden.

So ist es besipielsweise denkbar unter Anwendung des erfindungsgemässen Verfahrens nicht-kodierende, 'antisense'-DNA in das Genom einer Bacillus thuringiensis und/oder Bacillus cereus Zelle einzuschleusen, sodass im Zuge der Expression besagter 'antisense' DNA eine mRNA transkribiert wird, welche die Expression der korrespondierenden 'sense' DNA hemmt. Auf diese Weise ist es möglich, die Expression bestimmter, unerwünschter Gene in Bacillus thuringiensis und/oder Bacillus cereus gezielt zu unterdrücken.

Darüberhinaus ist nunmehr auch die Möglichkeit gegeben, neben der Bereitstellung verbesserter, gut definierter B. thuringiensis-Stämme zur Herstellung von verbesserten Bioinsektiziden, B. thuringiensis als generellen Wirt für die Klonierung und gegebenenfalls Expression heterologer und/oder homologer Gene zu verwenden.

In einer spezifischen und bevorzugten Ausführungsform des erfindungsgemässen Verfahrens ist es darüberhinaus nunmehr erstmals möglich neue Gene, insbesondere aber neue Protoxingene direkt im natürlichen Wirt, d.h. in B. thuringiensis oder B. cereus zu klonieren.

Bei der Suche nach neuen Protoxingenen wird zunächst eine Genbibliothek von B. thuringiensis erstellt.

In einem ersten Verfahrensschritt wird dabei die Gesamt-DNA eines Protoxin-produzierenden B. thuringiensis Stammes mit Hilfe an sich bekannter Verfahrensmassnahmen isoliert und anschliessend in einzelne Fragmente zerlegt. Die B. thuringiensis DNA kann dabei entweder mechanisch, beispielsweise unter Einwirkung von Scherkräften, oder aber vorzugsweise durch Verdauung mit geeigneten Restriktionsenzymen fragmentiert werden. Je nach Wahl der Enzyme erhält man eine partielle oder eine vollständige Verdauung der DNA Probe. Besonders bevorzugt im Rahmen dieser Erfindung ist die Verwendung von Restriktionsenzymen, die quartäre Erkennungsstellen enthalten und/oder zu einer partiellen Verdauung der B. thuringiensis DNA führen, wie z.B. das Restriktionsenzym SauIIIA, ohne jedoch darauf beschränkt zu sein. Selbstverständlich können auch alle anderen geeigneten Restriktionsenzyme in dem erfindungsgemässen Verfahren eingesetzt werden.

Die auf die zuvor beschriebene Weise gewonnenen Restriktionsfragmente werden dann mit Hilfe an sich bekannter Verfahren entsprechend ihrer Grösse aufgetrennt. Für die Grössen-abhängige Auftrennung von DNA Fragmenten verwendet man in der Regel Zentrifugationsverfahren wie z.B. die Saccharosegradientenzentrifugation oder elektrophoretische Verfahren, wie die Agarose Gelelektrophorese oder aber eine Kombination dieser Verfahren.

Diejenigen Fraktionen, welche Fragmente der richtigen Grösse enthalten, d.h. Fragmente, die aufgrund ihrer Grösse in der Lage sind ein Protoxin zu kodieren, werden vereinigt (gepoolt) und für die weiteren Verfahrensschritte verwendet.

Zunächst werden die zuvor isolierten Fragmente unter Verwendung von Standardverfahren in geeignete Klonierungsvektoren eingespleisst und anschliessend mit Hilfe des erfindungsgemässen Transformationsverfahrens direkt in Bacillus thuringiensis oder B. cereus, vorzugsweise aber in Protoxin-freie Stämme von Bacillus thuringiensis eingeschleust.

Als Vektoren können sowohl gram-positive Plasmide, wie z.B. pBC16, pUB110, pC194, als auch die zuvor im einzelnen beschriebenen 'Shuttle' Vektoren verwendet werden. Besonders bevorzugt im Rahmen dieser Erfindung ist der Shuttle Vektor pXI200, der im folgenden im Detail beschrieben ist (siehe Beispiel 9.1.). Geeignete Vektoren enthalten vorzugsweise DNA-Sequenzen, die eine leichte Identifizierbarkeit der transformierten, Vektor-haltigen Klone aus der Unzahl nicht transformierter Klone gewährleistet. Besonders bevorzugt sind DNA Sequenzen, die einen spezifischen Marker kodieren, der bei Expression zu einem leicht selektionierbaren Merkmal führt wie z.B. einer Antibiotika Resistenz. Beispielhaft sei hier eine Resistenz gegenüber Ampicillin, Chloramphenicol, Erythromycin, Tetracyclin, Hygromycin, G418 oder Kanamycin genannt.

Ebenfalls bevorzugt sind Markergene, die Enzyme mit einem chromogenen Substrat, wie z.B. X-gal (5-Brom-4-chlor-3-indolyl-β-D-galaktosid), kodieren. Die transformierten Kolonien können dann sehr einfach anhand einer spezifischen Farbreaktion nachgewiesen werden.

Nach der Elektroporation werden die behandelten Bacillus thuringiensis oder B. cereus Zellen auf ein selektives Sporulationsmedium übertragen und dort bis zur vollständigen Sporulation bei einer Temperatur von 10°C bis 40°C, vorzugsweise aber bei einer Temperatur von 20°C bis 35°C und ganz besonders bevorzugt bei einer Temperatur von 29°C bis 31°C inkubiert. Das Sporulationsmedium enthält als Selektivsubstanz vorzugsweise eine der oben genannten Antibiotika, abhängig vom verwendeten Vektor, sowie ein geeignetes Verfestigungsmittel, wie z.B. Agar, Agarose, Gelatine, etc.

Im Zuge der Sporulation kommt es zur Autolyse der sporulierenden Zellen, was für das nachfolgende Screening von grossem verfahrenstechnischen Vorteil ist, da ein künstliches Aufbrechen der Zellen entfällt. Bei Klonen, die das gesuchte Protoxingen enthalten und unter der Kontrolle ihres natürlichen Promotors exprimieren, liegen die gebildeten Kristallproteine frei zugänglich im Medium vor. Diese frei im Medium vorliegenden Kirstallporteine können dann z.B. mit Hilfe von Membranfiltern oder durch andere geeigntete Massnahmen fixiert werden. Geeignete Membranfilter sind beispielsweise Nylon- oder Nitrozellulose-Membranen. Membranen dieser Art sind frei käuflich.

Die so fixierten Kristallproteine können dann sehr einfach im Rahmen eines geeigneten Screenings aufgespürt und identifiziert werden.

Bevorzugt im Rahmen dieser Erfindung ist ein immunologisches Screening unter Verwendung Protoxin-spezifischer Antikörper. Die Verfahren des Immunscreenings sind bekannt und beispielsweise bei ³⁵⁾Young et al, 1983 im einzelnen beschrieben. Besonders bevorzugt ist die Verwendung monoklonaler Antikörper, die ganz spezifisch einen bestimmten Teil des Proteinmoleküls erkennen. Diese Antikörper können entweder einzeln oder aber in Form eines Gesmisches verwendet werden. Darüberhinaus können aber selbstverständlich auch polyklonale Antiseren für das Immunscreening verwendet werden. Auch Gemische basierend auf monoklonalen und polyklonalen Antikörpern sind denkbar.

Verfahren zur Herstellung monoklonaler Antikörper gegen Bacillus thuringiensis Protoxin Proteine sind bekannt und z.B. bei ³⁶⁾ Huber-Luka 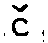 ,(1984) bzw. bei ³⁷⁾Huber-Luka 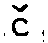 et al, (1986) im Detail beschrieben. Diese Verfahren lassen sich auch im vorliegenden Fall anwenden.

Darüberhinaus können im Rahmen dieser Erfindung selbstverständlich auch andere geeignete Screeningverfahren zum Aufspüren neuer DNA-Sequenzen in B. thuringiensis und/oder B. cereus verwendet werden.

Bacillus thuringiensis und B. cereus-Zellen, die mit Hilfe des zuvor beschriebenen Verfahrens transformiert worden sind, sowie die von diesen transformierten Bacillus-Zellen produzierten Toxine eignen sich in hervorragender Weise für die Bekämpfung von Insekten, insbesondere aber zur Bekämpfung von Insekten aus den Ordnungen Lepidoptera, Diptera und Coleoptera.

Besagte transformierte Zellen konnen somit in einen Verfahren zur Bekämpfung von Insekten verwendet werden, das sich dadurch kennzeichnet, dass man Insekten oder deren Lebensraum mit
a) B. thuringiensis oder B. cereus-Zellen oder mit einem Gemisch von beiden behandelt, die mit einem rekombinanten DNA-Molekül transformiert sind, das ein Strukturgen enthält, welches für ein 6-Endotoxin-Polypeptid kodiert, das natürlicherweise in B. thuringiensis vorkommt oder aber ein Polypeptid, das diesem im wesentlichen homolog ist; oder aber
b) mit zellfreien Kristallkörperpräparaten, die ein Protoxin enthalten, das von besagten transformierten Bacillus-Zellen produziert wird.

Entsprechende insektizide Mittel, enthalten neben den üblicherweise verwendeten Trägermitteln, Verteilungsmitteln oder Träger- und Verteilungsmitteln
a) B. thuringiensis oder B. cereus Zellen oder ein Gemisch besagter Bacillus-Zellen enthalten, die mit einem rekombinanten DNA-Molekül transformiert sind, das ein Strukturgen enthält, welches für ein δ-Endotoxin-Polypeptid kodiert, das natürlicherweise in B. thuringiensis vorkommt oder aber ein Polypeptid, das diesen im wesentlichen homolog ist; oder aber
b) zellfreie Kristallkörperpräparate, die ein Protoxin enthalten, das von besagten transformierten Bacillus-Zellen produziert wird.

Für die Anwendung als Insektizide werden die transformierten Mikroorganismen, die das rekombinante B. thuringiensis Toxin-Gen enthalten, vorzugsweise transformierte lebende oder tote B. thuringiensis oder B. cereus-Zellen, einschliesslich Gemischen aus lebenden und toten B. thuringiensis und B. cereus-Zellen, sowie die von besagten transformierten Zellen produzierten Toxin-Proteine in unveränderter Form oder vorzugsweise zusammen mit den in der Formulierungstechnik üblichen Hilfsstoffen, eingesetzt und in an sich bekannter Weise formuliert, z.B. zu Suspensionskonzentraten, streichbaren Pasten, direkt versprühbaren oder verdünnbaren Lösungen, benetzbaren Pulvern, löslichen Pulvern, Stäubemitteln, Granulaten, und auch Verkapselungen in z.B. polymeren Stoffen.
Die Anwendungsverfahren wie Versprühen, Vernebeln, Verstäuben, Verstreuen, Bestreichen oder Giessen werden gleich wie die Art der Mittel den angestrebten Zielen und den gegebenen Verhältnissen entsprechend gewählt.

Es können darüberhinaus selbstverständlich auch insektizide Gemische verwendet werden, bestehend aus transformierten, lebenden oder toten B. thuringiensis und/oder B. cereus Zellen sowie aus zellfreien Kristallkörperpräparaten, die ein Protoxin enthalten, das von besagten transformierten Bacillus Zellen produziert wird.

Die Formulierungen, d.h. die die transformierten lebenden oder toten Bacillus-Zellen oder Mischungen davon sowie die von besagten transformierten Bacillus-Zellen produzierten Toxin-Proteine und gegebenenfalls feste oder flüssige Hilfsmittel enthaltenden Mittel oder Zubereitungen werden in bekannter Weise hergestellt, z.B. durch inniges Vermischen der transformierten Zellen und/oder Toxin-Proteine mit festen Trägerstoffen und gegebenenfalls oberflächenaktiven Verbindungen (Tensiden).

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen nichtionogene, kation- und/oder anionaktive Tenside mit guten Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen seien die Alkali-, Erdalkali- oder unsubstituierte oder substituierte Ammoniumsalze von höheren Fettsäuren (C₁₀-C₂₂), wie z.B. die Na- oder K-Salze der Oel- oder Stearinsäure, oder von natürlichen Fettsäuregemischen, die z.B. aus Kokosnuss- oder Talgöl gewonnen werden können, genannt. Ferner sind auch die Fettsäure-methyl-taurinsalze zu erwähnen, wie z.B. das Natriumsalz der cis-2-(methyl-9-octadecenylamino)-ethansulfonsäure (Gehalt in Formulierungen vorzugsweise etwa 3 %).

Häufiger werden jedoch sogenannte synthetische Tenside verwendet, insbesondere Fettsulfonate, Fettsulfate, sulfonierte Benzimidazolderivate oder Alkylarylsulfonate oder Fett-Alkohole, wie z.B. 2,4,7,9-tetramethyl-5-decin-4,7-diol (Gehalt in Formulierungen vorzugsweise bei etwa 2 %).

Die Fettsulfonate oder -sulfate liegen in der Regel als Alkali-, Erdalkali- oder gegebenenfalls unsubstituierte oder substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylsulfats oder eines aus natürlichen Fettsäuren hergestellten Fettalkoholsulfatgemisches. Hierher gehören auch die Salze der Schwefelsäureester und Sulfonsäuren von Fettalkohol-Ethylenoxid-Addukten. Die sulfonierten Benzimidazolderivate enthalten vorzugsweise 2 Sulfonsäuregruppen und einen Fettsäurerest mit 8-22 C-Atomen. Alkylarylsulfonate sind z.B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes.

Ferner kommen auch entsprechende Phosphate wie z.B. Salze des Phosphorsäureesters eines p-Nonylphenol-(4 bis 14)-ethylenoxid-Adduktes in Frage.

Als nicht-ionische Tenside kommen in erster Linie Polyglykoletherderivate von aliphatischen oder cycloaliphatischen Alkoholen, gesättigten oder ungesättigten Fettsäuren und Alkylphenolen in Frage, die 3 bis 30 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nicht-ionische Tenside sind die wasserlöslichen, 20 bis 250 Ethylenglykolethergruppen und 10 bis 100 Propylenglykolethergruppen enthaltenden Polyethylenoxid-Addukte an Polypropylenglykol, Ethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Ethylenglykoleinheiten.

Als Beispiele nicht-ionischer Tenside seien Nonylphenolpolyethoxyethanole, Ricinusölpolyglykolether, Polypropylen/Polyethylenoxid-Addukte, Tributylphenoxypolyethoxyethanol, Polyethylenglykol und Octylphenoxypolyethoxyethanol erwähnt. Ferner kommen auch Fettsäureester von Polyoxyethylensorbitan wie das Polyoxyethylensorbitan-trioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten unsubstituierte oder halogenierte Nieder-Alkyl-, -Benzyl- oder niedrige Hydroxyalkylreste aufweisen. Die Salze liegen vorzugsweise als Halogenide, Methylsulfate oder Ethylsulfate vor, z.B. das Stearyltrimethylammoniumchlorid oder das Benzyldi(2-chlorethyl)ethylammoniumbromid.

Die in der Formulierungstechnik gebräuchlichen Tenside sind u.a. in folgenden Publikationen beschrieben:
³⁸⁾1986 International McCutcheon's Emulsifiers & Detergents, The Manufacturing Confectioner Publishing Co., Glen Rock, NJ, USA; Helmut Stache "Tensid-Taschenbuch" Carl Hanser-Verlag München/Wien 1981.

Die agrochemischen Mittel enthalten in der Regel 0,1 bis 99 %, insbesondere 0,1 bis 95 %, der transformierten, lebenden oder toten Bacilluszellen oder Mischungen davon bzw. der von besagten transformierten Bacillus-Zellen produzierten Toxin-Proteinen, 99,9 bis 1 %, insbesondere 99,8 bis 5 %, eines festen oder flüssigen Zusatzstoffes und 0 bis 25 %, insbesondere 0,1 bis 25 %, eines Tensides.

Während als Handelsware eher konzentrierte Mittel bevorzugt werden, verwendet der Endverbraucher in der Regel verdünnte Formulierungen.

Solche Mittel können noch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Die transformierten lebenden oder toten Bacillus-Zellen oder Mischungen davon, die die rekombinanten B. thuringiensis Toxin-Gene enthalten, sowie die von besagten transformierten Bacillus-Zellen produzierten Toxin-Proteine selbst sind hervorragend für die Bekämpfung von Schadinsekten geeignet. Vorzugsweise sind dabei pflanzenzerstörende Insekten der Ordnung Lepidoptera zu nennen, insbesondere solche der Gattungen Pieris, Heliothis, Spodoptera und Plutella, wie beispielsweise Pieris brassicae, Heliothis virescens, Heliothis zea, Spodoptera littoralis und Plutella xylostella.

Weitere Schadinsekten, die mit Hilfe der zuvor beschriebenen insektiziden Zubereitungen bekämpft werden können, sind beispielsweise Käfer der Ordnung Coleoptera, insbesondere solche der Familie Chrysomelidae, wie z.B. Diabrotica undecimpunctata, D. longicornis, D. virgitera, D. undecimpunctata howardi, Agelastica alni, Leptinotarsa decemlineata etc. sowie Insekten der Ordnung Diptera, wie beispielsweise Anopheles sergentii, Uranatenia unguiculata, Culex univittatus, Aedes aegypti, Culex pipiens, etc.

Die Aufwandmengen, in denen die Bacilluszellen bzw. die von diesen produzierten Toxin-Proteine eingesetzt werden, hängen von den jeweiligen Bedingungen ab, wie beispielsweise den Witterungsverhältnissen, der Bodenbeschaffenheit, dem Pflanzenwachstum und dem Applikationszeitpunkt.

### Formulierungsbeispiele für B. thuringiensis-Toxin enthaltendes Material

Bei den folgenden Formulierungsbeispielen sind unter dem Begriff "Bacillus-Zellen" solche B. thuringiensis und/oder B. cereus-Zellen zu verstehen, die ein erfindungsgemässes rekombinantes B. thuringiensis-Gen enthalten. (Bei den Angaben handelt es sich durchgehend um Gewichtsprozente.)

| F1. Granulate | a) | b) |
|---|---|---|
| Bacillus-Zellen und/oder von diesen produziertes Toxin-Protein | 5 % | 10 % |
| Kaolin | 94 % | - |
| Hochdisperse Kieselsäure | 1 % | - |
| Attapulgit | - | 90 % |

Die Bacillus-Zellen und/oder von diesen produziertes Toxin-Protein werden zunächst in Methylenchlorid suspendiert, anschliessend wird die Suspension auf das Trägermaterial aufgesprüht und danach das Suspendierungsagens im Vakuum verdampft.

| F2. Stäubemittel | a) | b) |
|---|---|---|
| Bacillus-Zellen und/oder von diesen produziertes Toxin-Protein | 2 % | 5 % |
| Hochdisperse Kieselsäure | 1 % | 5 % |
| Talkum | 97 % | - |
| Kaolin | - | 90 % |

Gebrauchsfertige Stäubemittel erhält man durch inniges Vermischen der Trägerstoffe mit den Bacillus-Zellen und/oder dem von diesen produzierten Toxin-Protein.

| F3. Spritzpulver | a) | b) | c) |
|---|---|---|---|
| Bacillus-Zellen und/oder von diesen produziertes Toxin-Protein | 25 % | 50 % | 75 % |
| Natrium-Ligninsulfonat | 5 % | 5 % | - |
| Natrium-Laurylsulfat | 3 % | - | 5 % |
| Natrium-Diisopropylnaphthalinsulfonat | - | 6 % | 10 % |
| Octylphenolpolyethylenglykolether (7-8 Mole Ethylenoxid | - | 2 % | - |
| Hochdisperse Kieselsäure | 5 % | 10 % | 10 % |
| Kaolin | 62 % | 27 % | - |

Die Bacilluszellen und/oder von diesen produziertes Toxin-Protein werden sorgfältig mit den Zusatzstoffen vermischt und das erhaltene Gemisch anschliessend in einer geeigneten Mühle gut vermahlen.
Man erhält Spritzpulver, die sich mit Wasser zu Suspensionen jeder gewünschten Konzentration verdünnen lassen.

| F4. Extruder Granulate | |
|---|---|
| Bacillus-Zellen und/oder von diesen produziertes Toxin-Protein | 10 % |
| Natrium-Ligninsulfonat | 2 % |
| Carboxymethylcellulose | 1 % |
| Kaolin | 87 % |

Die Bacillus-Zellen und/oder von diesen produziertes Toxin-Protein werden mit den Hilfsstoffen gemischt, sorgfältig vermahlen und mit Wasser angefeuchtet. Dieses Gemisch wird extrudiert und anschliessend in Luftstrom getrocknet.

| F5. Umhüllungs-Granulat | |
|---|---|
| Bacillus-Zellen und/oder von diesen produziertes Toxin-Protein | 3 % |
| Polyethylenglykol 200 | 3 % |
| Kaolin | 94 % |

Die homogen vermischten Bacillus-Zellen und/oder von diesen produziertes Toxin-Protein werden in einem Mischer auf das mit Polyethylenglykol angefeuchtete Kaolin gleichmässig aufgetragen. Auf diese Weise erhält man staubfreie Umhüllungs-Granulate.

| F6. Suspensions-Konzentrat | |
|---|---|
| Bacillus-Zellen und/oder von diesen produziertes Toxin-Protein | 40 % |
| Ethylenglykol | 10 % |
| Nonylphenolpolyethylenglykol (15 Mole Ethylenoxid) | 6 % |
| Alkylbenzolsulfonsäuretriethanolaminsalz∗ | 3 % |
| Carboxymethylcellulose | 1 % |
| Silikonöl in Form einer 75 %igen wässrigen Emulsion | 0,1 % |
| Wasser | 39 % |

| | |
|---|---|
| ∗ Alkyl ist vorzugsweise linear mit 10 bis 14, insbesondere 12-14 Kohlenstoffatomen wie beispielsweise n-Dodecylbenzolsulfonsäuretriethanolaminsalz. | |

Die homogen vermischten Bacillus-Zellen und/oder von diesen produziertes Toxin-Protein werden mit den Zusatzstoffen innig vermischt. Man erhält so ein Suspensionkonzentrat, aus welchem durch Verdünnen mit Wasser Suspensionen jeder gewünschten Konzentration hergestellt werden können.

### Beispiele

### Allgemeine rekombinante DNA-Techniken

Da viele der in dieser Erfindung genutzten rekombinanten DNA-Techniken Routine für den Fachmann sind, wird im folgenden eine kurze Beschreibung der allgemein gebräuchlichen Techniken gegeben, sodass auf diese allgemeinen Angaben im konkreten Ausführungsbeispiel verzichtet werden kann. Sofern nicht ausdrücklich darauf hingewiesen wird, sind alle diese Verfahren in der Referenz von ³³⁾Maniatis et al, 1982 beschrieben.

### A. Schneiden mit Restriktionsendonukleasen

Typischerweise sind etwa 50 µg/ml bis 500 µg/ml DNA in dem Reaktionsansatz enthalten, in der vom Hersteller, New England Biolabs, Beverly, MA., empfohlenen Pufferlösung. Für jedes µg DNA werden 2 bis 5 Einheiten von Restriktionsendonukleasen hinzugefügt und der Reaktionsansatz bei der vom Hersteller empfohlenen Temperatur für eine bis drei Stunden inkubiert. Die Reaktion wird durch 10 minütiges Erhitzen auf 65°C oder durch Extraktion mit Phenol, gefolgt durch Präzipitation der DNA mit Ethanol, beendet. Diese Technik wird auch auf den Seiten 104 bis 106 der ³³⁾Maniatis et al-Referenz beschrieben.

### B. Behandlung der DNA mit Polymerase, um glatte Enden zu erzeugen

50 µg/ml bis 500 µg/ml DNA-Fragmente werden zu einem Reaktionsansatz hinzugefügt, in dem vom Hersteller, New England Biolabs, empfohlenen Puffer. Der Reaktionsansatz enthält alle vier Desoxynukleotidtriphosphate in Konzentrationen von 0.2 mM. Nach Zusatz einer geeigneten DNA-Polymerase erfolgt die Reaktion während 30 Minuten bei 15°C und wird dann durch 10 minütiges Erhitzen auf 65°C beendet. Für Fragmente, die durch Schneiden mit Restriktionsendonukleasen erhalten werden, welche 5′-überstehende Enden erzeugen, wie EcoRI und BamHI, wird das grosse Fragment, oder Klenow-Fragment, der DNA-Polymerase verwendet. Für Fragmente, die durch Endonukleasen erhalten werden, welche 3′-überstehende Enden erzeugen, wie PstI und SacI, wird die T4-DNA-Polymerase verwendet. Die Verwendung dieser beiden Enzyme wird auf den Seiten 113 bis 121 der ³³⁾Maniatis et al-Referenz beschrieben.

### C. Agarose-Gelelektrophorese und Reinigung der DNA-Fragmenten von Gelverunreinigungen.

Die Agarose-Gelelektrophorese wird in einer horizontalen Apparatur durchgeführt, wie dies auf den Seiten 150 bis 163 der ³³⁾Maniatis et al-Referenz beschrieben ist. Der verwendete Puffer entspricht dem dort beschriebenen Tris-Borat- oder Tris-Acetatpuffer. Die DNA-Fragmente werden durch 0.5 µg/ml Ethidiumbromid gefärbt, das entweder im Gel- oder im Tankpuffer während der Elektrophorese vorhanden ist oder aber wahlweise erst nach der Elektrophorese hinzugefügt wird. Die DNA wird durch Beleuchtung mit langwelligem Ultraviolettlicht sichtbar gemacht. Wenn die Fragmente vom Gel abgetrennt werden sollen, wird eine Agarose verwendet, die bei niedriger Temperatur geliert und von Sigma Chemical, St. Louis, Missouri, bezogen werden kann. Nach der Elektrophorese wird das gewünschte Fragment ausgeschnitten, in ein Plastikröhrchen gegeben, etwa 15 Minuten auf 65°C erhitzt, dreimal mit Phenol extrahiert und zweimal mit Ethanol gefällt. Dieses Verfahren ist gegenüber dem von ³³⁾Maniatis et al auf Seite 170 beschriebenen leicht verändert.

Als Alternative kann die DNA auch aus dem Agarosegel mit Hilfe des 'Geneclean Kits' (Bio 101 Inc., La Jolla, CA, US) isoliert werden.

### D. Entfernen von 5′-terminalen Phosphaten von DNA-Fragmenten

Während der Plasmidklonierungsschritte vermindert eine Behandlung des Vektorplasmids mit Phosphatase die Rezirkularisation des Vektors (diskutiert auf Seite 13 der ³³⁾Maniatis et al-Referenz). Nach dem Schneiden der DNA mit der richtigen Restriktionsendonuklease wird eine Einheit alkalische Phosphatase aus dem Darm von Kälbern hinzugefügt, die von Boehringer-Mannheim, Mannheim, erworben werden kann. Die DNA wird eine Stunde bei 37°C inkubiert und anschliessend zweimal mit Phenol extrahiert und mit Ethanol gefällt.

### E. Verknüpfen der DNA-Fragmente

Wenn Fragmente mit komplementären kohäsiven Enden miteinander verknüpft werden sollen, werden etwa 100 ng von jedem Fragment in einem Reaktionsgemisch von 20 µl bis 40 µl mit etwa 0.2 Einheiten T4 DNA-Ligase von New England Biolabs im vom Hersteller empfohlenen Puffer inkubiert. Die Inkubation wird 1 bis 20 Stunden lang bei 15°C durchgeführt. Wenn DNA-Fragmente mit glatten Enden verknüpft werden sollen, werden sie wie oben beschrieben inkubiert, wobei aber die Menge der T4 DNA-Ligase in diesem Fall auf 2 bis 4 Einheiten erhöht wird.

### F. Die Transformation von DNA in E. coli

E. coli Stamm HB101 wird für die meisten Experimente verwendet. DNA wird mit dem Kalziumchloridverfahren, wie es von ³³⁾Maniatis et al., Seiten 250 bis 251, beschrieben wurde, in E. coli eingeführt.

### G. Screening von E. coli auf Plasmide

Nach der Transformation werden die resultierenden Kolonien von E. coli auf das Vorhandensein des gewünschten Plasmids durch ein schnelles Plasmidisolationsverfahren geprüft. Zwei gebräuchliche Verfahren werden auf den Seiten 366 bis 369 der ³³⁾Maniatis et al-Referenz beschrieben.

### H. Isolierung von Plasmid-DNA in grossem Massstab

Verfahren zur Isolierung von Plasmiden aus E. coli in grossem Massstab werden auf den Seiten 88 bis 94 der ³³⁾Maniatis et al-Referenz beschrieben.

### Medien und Pufferlösungen

| LB-Medium | [g/l] |
|---|---|
| Trypton | 10 |
| Hefeextrakt | 5 |
| NaCl | 5 |

### Antibiotic Medium Nr. 3 (Difco Laboratories)

| | [g/l] |
|---|---|
| Rinder Fleischextrakt | 1.5 |
| Hefeextrakt | 1.5 |
| Peptone | 5 |
| Glucose | 1 |
| NaCl | 3.5 |
| K₂HPO₄ | 3.68 |
| KH₂PO₄ | 1.32 |

| SCGY-Medium | [g/l] |
|---|---|
| Casaminoacids | 1 |
| Hefeextrakt | 0.1 |
| Glucose | 5 |
| K₂HPO₄ | 14 |
| KH₂PO₄ | 6 |
| Na₃-Citrat | 1 |
| (NH₄)₂SO₄ | 2 |
| MgSO₄ · 7 H₂O | 0.2 |

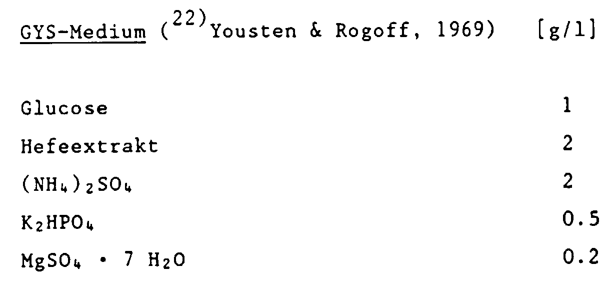

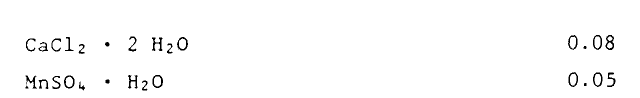

### pH vor Autoklavieren auf 7.3 einstellen.

| PBS-Puffer | [mM] |
|---|---|
| Saccharose | 400 |
| MgCl₂ | 1 |
| Phosphat-Puffer, pH 6.0 | 7 |

| TBST-Puffer | [mM] |
|---|---|
| Tween 20∗ | 0.05% (w/v) |
| Tris/HCl∗ (pH 8.0) | 10 |
| NaCl | 150 |

| | |
|---|---|
| ∗Tween 20 Polyethoxysorbitanlaurat ∗Tris/HCl α,α,α-Tris(hydroxymethyl)methylaminohydrochlorid | |

Die im Prioritätsdokument für die Benennung der Plasmide gewählte interne Bezeichnung pK wurde für die Auslandsfassung durch die offiziell anerkannte Bezeichnung pXI ersetzt.

Auch die Bezeichung für die im Rahmen der Ausführungsbeispiele verwendete asporogene B. thuringiensis HD1 Mutante wurde von cryβ nach cryB geändert.

### Beispiel 1: Transformation von B. thuringiensis mit Hilfe der Elektroporation

Beispiel 1.1: 10 ml eines LB Mediums (Trypton 10 g/l, Hefeextrakt 5 g/l, NaC1 5 g/l) werden mit Sporen von B. thuringiensis var. kurstaki HD1cryB (³⁹⁾Stahly DP et al, 1978) einer plasmidfreien Variante von B. thuringiensis var. kurstaki HD1, inokuliert.

Dieser Ansatz wird über Nacht bei einer Temperatur von 27°C auf einer Rundschüttelmaschine bei 50 Upm inkubiert. Danach wird die B. thuringiensis Kultur in 100 ml bis 400 ml LB-Medium 100fach verdünnt und bei einer Temperatur von 30°C auf einer Rundschüttelmaschine bei 250 Upm weiter kultiviert, bis eine optische Dichte (OD₅₅₀) von 0.2 erreicht ist.

Die Zellen werden mit Hilfe einer Zentrifugation geerntet und in 1/40 Volumen eines eisgekühlten PBS-Puffers (400 mM Saccharose, 1 mM MgCl₂, 7 mM Phosphat-Puffer pH 6.0) suspendiert. Die Zentrifugation und anschliessende Suspension der geernteten B. thuringiensis-Zellen in PBS-Puffer wird einmal wiederholt.

Die so vorbehandelten Zellen können dann entweder direkt elektroporiert oder aber nach Zugabe von Glycerin in die Pufferlösung [20% (w/v)], bei -20°C bis -70°C aufbewahrt und zu einem späteren Zeitpunkt verwendet werden.

800 µl Aliquots der eisgekühlten Zellen werden dann in vorgekühlte Küvetten überführt, anschliessend wird 0.2 µg pBC16 Plasmid DNA (⁴⁰⁾Bernhard K. et al, 1978) (20 µg/ml) zugegeben und der gesamte Ansatz 10 Minuten bei 4°C inkubiert.

Bei Verwendung von tiefgekühltem Zellmaterial wird zunächst ein geeignetes Aliquot gefrorener Zellen in Eis oder bei Raumtemperatur aufgetaut. Die weitere Behandlung erfolgt analog dem Vorgehen bei Verwendung frischen Zellmaterials.

Danach wird die Küvette in eine Elektroporationsapparatur eingebracht und die in der Suspension vorliegenden B. thuringiensis-Zellen werden einer Elektroporation unterzogen, indem sie durch einmaliges Entladen eines Kondensators mit Spannungen zwischen 0.1 kV und 2.5 kV beaufschlagt werden.

Der hier verwendete Kondensator weist eine Kapazität von 25 µF auf, die Küvetten haben einen Elektrodenabstand von 0.4 cm, was bei einer Entladung je nach Einstellung zu einer exponentiell abnehmenden Feldstärke mit anfänglichen Spitzenwerten von 0.25 kV/cm bis 6.25 kV/cm führt. Die exponentielle Abklingzeit liegt in einem Bereich zwischen 10 ms und 12 ms.

Für die beschriebenen Elektroporationsversuche kann beispielsweise ein Elektroporationsgerät der Firma Bio Rad verwendet werden ('Gene Pulser Apparatus', # 165-2075, Bio Rad, 1414 Harbour Way South, Richmond, CA 94804, USA).

Selbstverständlich ist auch jedes andere geeignete Gerät in dem erfindungsgemässen Verfahren einsetzbar.

Nach einer weiteren 10 minütigen Inkubation bei 4°C wird die Zellsuspension mit 1.2 ml LB Medium verdünnt und 2 Stunden bei einer Temperatur von 30°C auf einer Rundschüttelmaschine bei 250 Upm inkubiert.

Anschliessend werden geeignete Verdünnungen auf LB Agar (LB Medium verfestigt mit Agar, 15 g/l) ausplattiert, der ein für die Selektion des neu erhaltenen Plasmids geeignetes Antibiotikum als Zusatz enthält. Im Falle von pBC16 handelt es sich dabei um Tetracyclin, das dem Medium in einer Konzentration von 20 mg/l zugegeben wird.

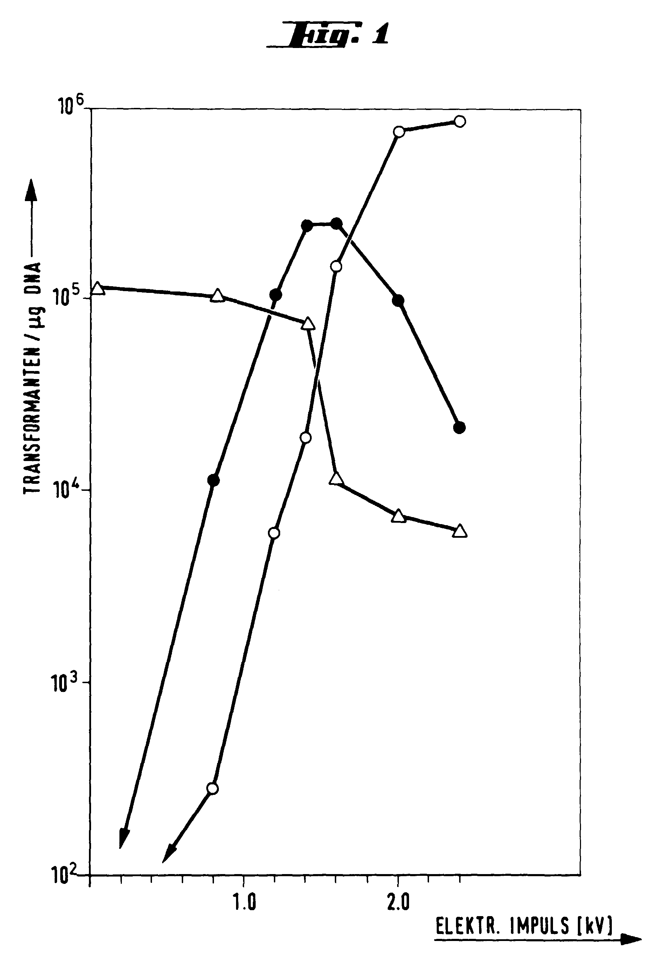

Die für B. thuringiensis HD1cryB und pBC16 in Abhängigkeit der angelegten Ausgangsspannung bei gegebenem Plattenabstand erzielten Transformationsfrequenzen sind in Abbildung 1 wiedergegeben.

Die Expression der eingeschleusten DNA kann anhand der auftretenden Tetracyclinresistenz nachgewiesen werden. Bereits 2 Stunden nach der Transformation von pBC16 in B. thuringiensis erfolgt eine vollständige phänotypische Expression der neu eingeführten Tetracyclin-Resistenz (siehe Tabelle 2).

Beispiel 1.2: Die Transformation von B. thuringiensis Zellen wird in genau analoger Weise zu Beispiel 1.1. durchgeführt, mit der Ausnahme, dass das Volumen der für die Elektroporation vorgesehenen Zellsuspension in diesem Fall 400 µl beträgt.

Durch diese Verfahrensmassnahme kann die Transformationsfrequenz um den Faktor 10 erhöht werden.

### Beispiel 2: Transformation von B. thuringiensis HD1cryB mit einer Reihe verschiedener Plasmide

Die meisten Versuche werden mit dem Plasmid pBC16 durchgeführt, einem natürlich vorkommenden Plasmid aus B. cereus. Daneben können aber auch andere natürlich vorkommende Plasmide erfolgreich in B. thuringiensis-Zellen eingeschleust werden, wie z.B. pUB110 (²⁵⁾Polack J. und Novik RP, 1982), pC194 (²⁴⁾Horinouchi S und Weisblum B, 1982) und pIM13 (²⁶⁾Mahler I und Halvorson HO, 1980) (siehe Tabelle 3).

Auch Varianten dieser Plasmide, die besser geeignet sind für die Arbeiten mit rekombinanter DNA als die natürlichen Isolate lassen sich mit Hilfe des erfindungsgemässen Verfahrens in den B. thuringiensis-Stamm HD1cryβ transformieren, wie z.B. der B. subtilis Klonierungsvektor pBD64 (²⁷⁾Gryczan T et al, 1980) sowie die Plasmide pBD347, pBD348 und pUB1664 (siehe Tabelle 3; die Plasmide pBD347, pBD348 und pUB1164 können von Dr. W. Schurter, CIBA-GEIGY AG, Basel bezogen werden).

Die Transformationsergebnisse der Tabelle 3 machen deutlich, dass unabhängig von der verwendeten Plasmid-DNA bei Anwendung des erfindungsgemässen Transformationsverfahrens Transformationsfrequenzen erreicht werden, die - mit einer Ausnahme - alle im Bereich zwischen 10⁵ bis 10⁷ liegen.

### Beispiel 3: Konstruktion eines 'Shuttle'-vektors für Bacillus thuringiensis

Bestehende bifunktionelle Vektoren für E. coli und B. subtilis wie z.B. pHV 33 (⁴¹⁾Primrose SB und Ehrlich SD, Plasmid, 6: 193-201, 1981) sind für B. thuringiensis HD1cryB nicht geeignet (siehe Tabelle 3).

Für die Konstruktion eines potenten bifunktionellen Vektors wird zunächst das grosse EcoRI-Fragment von pBC16 mit Hilfe von T4DNA-Ligase in die EcoRI-Stelle des Plasmids pUC8 (²⁸⁾Vieira J und Messing J, 1982) eingespleisst. Anschliessend werden E. coli-Zellen mit diesem Konstrukt transformiert. Eine mit Hilfe einer Restriktionsanalyse als korrekt erkannte Konstruktion wird pXI 62 genannt.

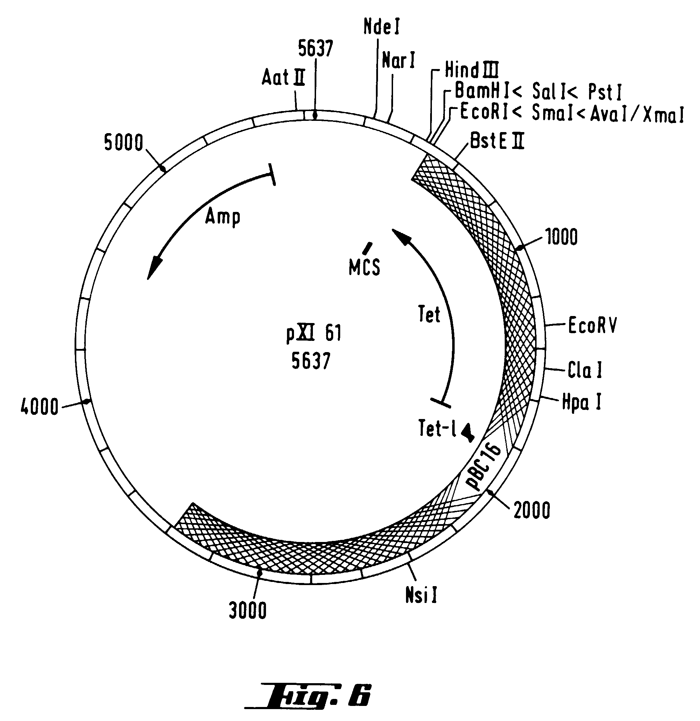

Es folgt die Entfernung der distal der pUC8 Polylinkerregion gelegenen EcoRI Schnittstelle. Durch eine partielle EcoRI-Verdauung wird pXI 62 linearisiert. Die kohäsiven EcoRI-Enden werden mit Klenow-Polymerase aufgefüllt und mit T4 DNA-Ligase wieder zusammengefügt. Nach Transformation in E. coli wird eine mit Hilfe einer Restriktionsanalyse als richtig erkannte Konstruktion ausgewählt und pXI 61 genannt. Eine Karte von pXI 61 mit den Schnittstellen von Restriktionsenzymen, die pXI 61 nur einmal schneiden, ist in Abbildung 6 gezeigt.

Diese Konstruktion lässt sich mit Hilfe der in Beispiel 1 beschriebenen Transformationsmethode direkt in B. thuringiensis HD1cryB transformieren.

Aufgrund starker Restriktionsbarrieren in B. thuringiensis Stämmen liegen die Transformationsraten in diesem Fall bei Verwendung der aus E. coli stammenden pXI61 DNA niedriger als bei Verwendung der aus B. thuringiensis HD1cryB stammenden Plasmid-DNA (siehe Tabelle 3). Dennoch erweist sich pXI61 als sehr geeignet für die Durchführung von Klonierungsexperimenten in B. thuringiensis.

### Beispiel 4: Einschleusung des Kurhd1 delta-Endotoxingens in Stämme von B. thuringiensis und B. cereus

Die im Rahmen dieser Erfindung für die Einschleusung und Expression in B. thuringiensis bzw. B. cereus verwendete DNA-Sequenz, die für ein kurhd1 delta-Endotoxin-Protein kodiert, stammt aus dem Plasmid pK36, das mit Datum vom 4. März 1986 bei der als Internationale Hinterlegungsstelle anerkannten Deutschen Sammlung von Mikroorganismen, BRD, entsprechend den Anforderungen des Budapester Vertrages für die Internationale Anerkennung der Hinterlegung von Mikroorganismen zum Zwecke der Patentierung unter der Hinterlegungsnummer DSM 3668 hinterlegt wurde.

Eine detallierte Beschreibung der Verfahren zur Identifizierung und Isolierung der δ-Endotoxin-Gene sowie der Konstruktion des Plasmids pK36 ist in der Europäischen Patentanmeldung EP 0238 441 enthalten und in Form einer Referenz ein Bestandteil der vorliegenden Erfindung.

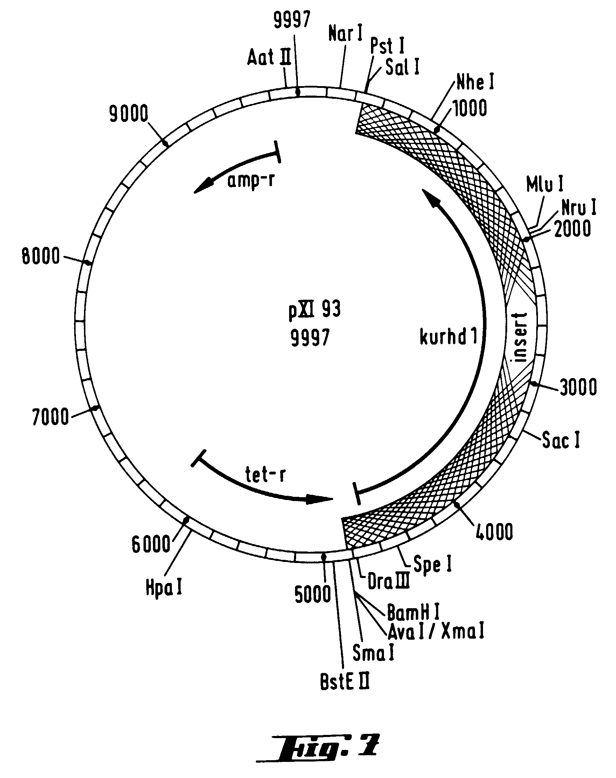

pK36 Plasmid DNA wird mit den Restriktionsenzymen Pst1 und BamH1 komplett verdaut und das 4.3 Kb umfassende Fragment, das das Kurhd1 delta-Endotoxingen (vgl. Formel I) enthält, aus einem Agarosegel isoliert. Dieses Fragment wird dann in pXI61 eingespleisst, welches zuvor mit Pst1 und BamH1 verdaut und mit alkalischer Phosphatase aus dem Kälberdarm behandelt wurde. Nach der Transformation von E. coli HB 101 wird ein mit Hilfe einer Restriktionsanalyse als korrekt erkannte Konstruktion isoliert, die die Bezeichnung pXI93 erhält. Eine Restriktionskarte von pXI93 ist in Abbildung 7 wiedergegeben.

pXI93 kann auf 2 verschiedenen Wegen in B. thuringiensis HD1cryB eingebracht werden.
a) B. thuringiensis Zellen werden direkt mit einem pXI93 Isolat aus E. coli mit Hilfe des in Beispiel 1 beschriebenen erfindungsgemässen Transformationsverfahrens transformiert.
b) pXI93 wird zunächst in B. subtilis Zellen transformiert, wie bei Chang und Cohen, 1979 beschrieben. Aus einem Transformanten, der die komplette und intakte pXI93 Plasmid DNA enthält, wird diese isoliert und anschliessend mit Hilfe der in Beispiel 1 beschriebenen Elektroporationsmethode in B. thuringiensis HD1cryB transformiert.

Die Anwendung beider Verfahren führt zu Transformanten, die das intakte pXI93 Plasmid enthalten, was anhand einer Restriktionsanalyse gezeigt werden kann.

### Beispiel 5: Nachweis der Expression des delta-Endotoxingens in B. thuringiensis

Sporulierende Kulturen von B. thuringiensis HD1 cryB, HD1cryB (pXI61) und HD1cryB (pXI93) und HD1 werden im Phasenkontrastmikroskop bei 400-facher Vergrösserung miteinander verglichen. Nur im pXI93 und in HD1 enthaltenden Stamm können die typischen bipyramidalen Proteinkristalle nachgewiesen werden. Extrakte aus den selben Kulturen werden auf einem SDS-Polyacrylamidgel elektrophoretisch aufgetrennt. Nur für den das Plasmid pXI93 und bei HD1 enthaltenden Stamm konnte auf dem Gel eine Proteinbande von 130'000 Dalton nachgewiesen werden, die dem Kurhd1 Genprodukt entspricht (Abbildung 8a).

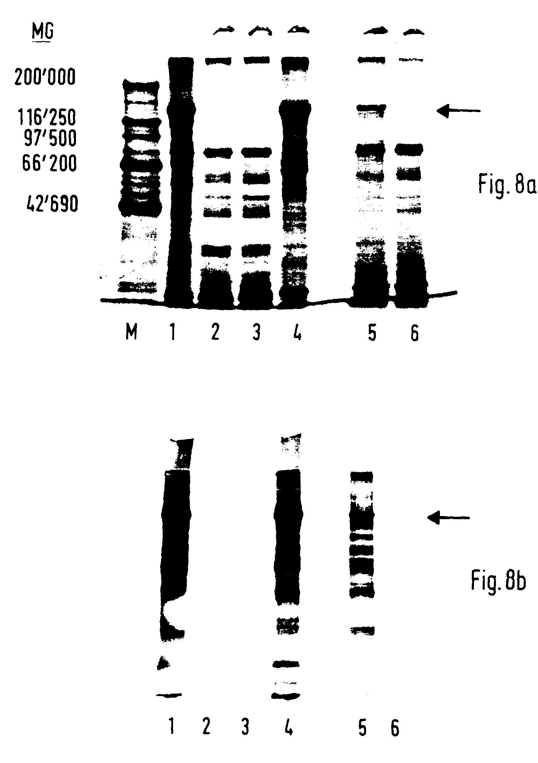

In der 'Western blot' Analyse (Abbildung 8b) reagiert dieses 130'000 Dalton Protein und seine Abbauprodukte spezifisch mit polyklonalen Antikörpern, die zuvor gegen Kristallprotein aus B. thuringiensis var. Kurstaki HD1 gemäss dem bei ⁴²⁾Huber-Lukac H, 1982 beschriebenen Verfahren hergestellt werden. Eine detallierte Beschreibung dieses Verfahrens findet man in der Europäischen Patentanmeldung EP 238,441, die in Form einer Referenz ein Bestandteil dieser Erfindung ist. Auf dem Plasmid pXI93 befindet sich stromaufwärts der Toxin-kodierenden Region ein 156 Bp umfassender DNA Abschnitt, der den vorbeschriebenen Sporulation-abhängigen Tandem-Promotor (²⁹⁾Wong HC et al, 1983), enthält. Diese Sequenz ist ausreichend für eine hohe Expression des delta-Endotoxingens in B. thuringiensis HD1 cryB und B. cereus 569K.

### Beispiel 6: Nachweis der Toxizität des rekombinanten B. thuringiensis HD1 cryB (pXI93)

B. thuringiensis HD1 cryB und HD1 cryB (pXI93) werden bei 25°C in Sporulationsmedium (GYS-Medium) gezüchtet. Nach vollständiger Sporulation, die im Phasenkontrastmikroskop kontrolliert wird, werden Sporen und (soweit vorhanden) Protoxinkristalle durch Zentrifuation geerntet und Sprüh-getrocknet. Das daraus resultierende Pulver wird in verschiedenen Konzentrationen der Diät von L-1 Larven von Heliothis virescens (tobacco budworm) beigemischt. Die Mortalität der Larven wird nach sechs Tagen bestimmt.

Wie erwartet ist der Protoxingen-freie Stamm HD1 cryB nicht toxisch für Heliothis virescens, während der mit Plasmid pXI 93 transformierte Stamm eine Dosis-abhängige Mortalität von H. virescens verursacht (Tabelle 4). Dies zeigt, dass durch das Elektroporationsverfahren hergestellte rekombinante Stämme tatsächlich als Bioinsektizid verwendet werden können.

### Beispiel 7: Elektroporation verschiedener B. thuringiensis und B. spec. Stämme

Das unter Beispiel 1 beschriebene Transformationsprotokoll für B. thuringiensis HD1 cryB lässt sich auch auf andere Stämme anwenden.

Alle getesteten Stämme von B. thuringiensis var. kurstaki lassen sich mit dieser Methode sehr einfach und effizient transformieren (Tabelle 5).

Mit einem Laborstamm von B. cereus können ebenfalls ausgezeichnete Transformationsfrequenzen erreicht werden. Das gleiche gilt auch für weitere der getesteten B. thuringiensis Varietäten (var. israelensis, var. kurstaki). B. subtilis dagegen lässt sich durch das Elektroporationsverfahren nur sehr mangelhaft transformieren.

Bei Anwendung der Protoplasten-abhängigen PEG-Methode konnten dagegen mit B. subtilis Transformationsraten von 4 x 10⁶/µg Plasmid DNA erreicht werden.

Die niedrigen Transformationsraten von B. subtilis bei Anwendung der Elektroporationstechnik stehen nicht in Zusammenhang mit falsch gewählten Parametern, wie z.B. einer ungeeigneten Spannung oder mit einer hohen Mortalitätsrate, verursacht durch elektrische Impulse, wie anhand der Abb. 9 ersichtlich ist.

### Beispiel 8: Transformation von B. thuringiensis HD1 cryβ mit dem β-Galaktosidasegen

### 8.1. Einbau einer BamHI-Restriktionsschnittstelle unmittelbar vor das erste AUG - Kodon des B. thuringiensis Protoxingens

Bevor das β-Galaktosidase Gen aus dem Plasmid piWiTh5 (erhältlich von Dr. M. Geiser, CIBA-GEIGY AG, Basel, Schweiz) mit dem Promotor des Kurhd1 δ-Endotoxingens aus B. thuringiensis verknüpft werden kann, musss zunächst die in der Umgebung des AUG Startkodons befindliche DNA Sequenz des Protoxingens modifiziert werden.

Diese Modifikation wird mit Hilfe einer Oligonukleotid-vermittelten Mutagenese durchführt, unter Verwendung des einzelsträngingen Phagen Ml3mp8, der das 1.8 kB umfassende HincII-HindIII Fragment aus dem 6-Endotoxingen, das die 5'-Region des Toxingens umfasst, enthält.

Zunächst werden 3 µg des Plasmids pK36 (vgl. Beispiel 4) mit den Restriktionsenzymen HindIII und HincII verdaut. Das resultierende 1.8 kb-Fragment wird über eine Agarosegelelektrophorese gereinigt und anschliessend aus dem Gel isoliert.

Parallel dazu werden 100 ng M13mp8 RF Phagen-DNA (Biolab, Tozer Road, Beverly MA, 01915, USA oder jeder beliebige andere Hersteller) mit den Restriktionsenzymen SmaI und HindIII verdaut, phenolisiert und durch Zugabe von Ethanol präzipitiert. Die so behandelte Phagen-DNA wird dann mit 200 ng des zuvor isolierten Protoxin-Fragmentes vermischt und durch Zugabe von T4 DNA Ligase mit diesem verknüpft.

Nach der Transfektion von E. coli JM103 werden 6 weisse Plaques herausgelesen und mit Hilfe einer Restriktionskartierung analysiert.

Ein Isolat, bei dem die Verknüpfung zwischen dem β-Galaktosidase Gen und dem Promotor des Kurhdl 6-Endotoxingens aus B. thuringiensis korrekt erfolgt ist, wird ausgelesen und erhält die Bezeichnung M13mp8/Hinc-Hind.

Mit Hilfe eines DNA-Syntheseapparates ('APPLIED BIOSYSTEM DNA SYNTHESIZER') wird ein Oligonukleotid synthetisiert, das die folgende Sequenz aufweist:

(5') GTTCGGATTGGGATCCATAAG (3')

Dieses synthtische Oligonukleotid ist einer Region auf der M13mp8/Hinc-Hind DNA komplementär, die von Position 153 bis Position 173 des Kurdhl 6-Endotoxingens reicht (vgl. Formel I). Die oben wiedergegebene Oligonukleotidsequenz weist jedoch in Position 162 und 163 ein 'Mismatch' gegenüber der in Formel I wiedergegebenen Sequenz auf, wodurch es zur Ausbildung einer BamHI Restriktionsschnittstelle kommt. Die allgemeine Vorgehensweise bei der Mutagenese ist bei JM Zoller und M Smith (⁴³⁾ JM Zoller and M Smith; 19) beschrieben. Etwa 5 µg einzelsträngiger M13mp18/Hinc-Hind Phagen-DNA wird mit 0,3 µg phosphorylierten Oligonukleotiden in einem Gesamt-Volumen von 40 µl gemischt. Diese Mischung wird für 5 Minuten auf 65°C erhitzt, dann zunächst auf 50°C abgekühlt und anschliessend allmählich auf 4°C heruntergekühlt. Danach werden Puffer, Nucleotidtriphosphate, ATP, T4-DNA-Ligase und das grosse Fragment der DNA-Polymerase hinzugefügt und über Nacht bei 15°C, wie beschrieben (⁴³⁾ JM Zoller and M Smith) inkubiert. Nach einer Agarosegelelektrophorese wird zirkuläre doppelsträngige DNA gereinigt und mittels Transfektion in den E. coli Stamm JM103 eingeschleust. Alternativ dazu kann auch der E. coli Stamm JM 107 verwendet werden.

Die resultierenden Plaques werden auf Sequenzen hin untersucht, die mit ³²P-markiertem Oligonukleotid hybridisieren; die Phagen werden mit Hilfe der DNA Restriktionsendonukleasen-Analyse untersucht.

Ein Phage, der eine korrekte Konstruktion enthält, bei der sich eine BamHI Schnittstelle unmittelbar vor dem ersten AUG Kodon des Protoxingens befindet, wird mit M13mp8/Hinc-Hind/Bam bezeichnet.

### 8.2. Verknüpfung des β-Galktosidasegens mit dem δ-Endotoxin-Promotor

8.2.1: Der δ-Endotoxin Promotor befindet sich auf einem 162 Bp umfassenden EcoRI/BamHI Fragment der M13mp8/Hinc-Hind/Bam-Phagen DNA. RF-Phagen DNA wird mit dem Restriktionsenzym BamHI verdaut. Die dadurch an den 5′-Enden entstehenden Ueberhänge werden durch Behandlung mit 'Mung Bean'-Nuklease (Biolabs) gemäss den Angaben des Herstellers entfernt. Danach wird die DNA mit der Restriktionsendonuklease EcoRI verdaut und das 162 Bp umfassende Frgment nach Durchführung einer Agarosegelelektrophorese aus dem Agarosegel isoliert.

Das β-Galaktosidasegen wird aus dem Plasmid piWiTh5 isoliert. piwiTh5 DNA wird zunächst an der einzigen HindIII Schnittstelle geschnitten. Die 3′ ausgesparten Enden werden mit Hilfe des Klenow-Fragments der DNA Polymerase aufgefüllt (vgl. ³³⁾ Maniatis et al., 1983, Seite 113-114) und die modifizierte DNA wird anschliessend mit dem Restriktionsenzym SalI verdaut. Das DNA-Fragment, welches das β-Galaktosidasegen enthält wird mit Hilfe einer Agarosegelelektrophorese isoliert.

Der Vektor pXI61 (vgl. Beispiel 3) wird mit den Restriktionsenzymen EcoRI und SalI verdaut und die beiden zuvor isolierten Fragmente werden in den Vektor pXI61 eingespleisst.

Nach der Transformation dieser Ligationsmischung in den E. coli Stamm HB101 bzw. JM 107 werden die korrekt verknüpften Zellklone mit Hilfe der Restriktionsanalyse und anhand ihrer β-Galktosidaseaktivität gegenüber dem chromogenen Substrat X-gal (5-Brom-4-chlor-3-indolyl-β-D-galaktosid) selektioniert. Ein Klon, der eine korrekte genetische Konstruktion enthält wird mit pXI80 bezeichnet.

8.2.2.: In einer alternativen Ausführungsform wird das 162 Bp umfassende EcoRI/BamHI Fragment, welches den δ-Endotoxin Promotor enthält, durch Schneiden von M13mp8/Hinc-Hind/Bam mit EcoRI und BamHI und anschliessende gelelektrophoretische Auftrennung isoliert.

Das β-Galaktosidasegen wird auch in diesem Fall aus dem Plasmid piWiTh5 isoliert (vgl. Beispiel 8.1.). Die Plasmid DNA wird dabei mit den Restriktionsenzymen BamHI und BglII verdaut und das grossen Fragment nach Gelelektrophorese aus dem Agarosegel eluiert.

Der Vektor pHY300 PLK (#PHY-001; Toyobo Co., Ltd., 2-8 Dojima Hama 2-Chome, Kita-ku, Osaka, 530 Japan), der kommerziell bezogen werden kann (vlg. Beispiel 9.1), wird mit den Restriktionsenzymen EcoRI und BglII verdaut. Die beiden zuvor isolierten Fragmente werden dann in den Vektor pHY300 PLK eingespleisst.

Die gesamte Ligationsmischung wird anschliessend in den E. coli Stamm JM107 [Bethesda Research Laboratories (BRL), 411 N, Stonestreet Avenue, Rockville, MD 20850, USA] transformiert. Ein Klon, der eine β-Galaktosidasaktivität aufweist wird weiter mit Hilfe von Restriktionsverdauungen analysiert. Ein Klon, der eine korrekte genetische Konstrukiton enthält, wird mit pXI101 bezeichnet.

### 8.3. Transformation des Plasmids pXI80 bzw. pXI101 in B. subtilis und B. thuringiensis

pXI80 bzw. PXI101 Plasmid DNA wird zunächst gemäss einem bekannten, bei Chang und Cohen beschriebenen Versuchsprotokoll (¹³⁾ Chang und Cohen, 1979), in B. subtilis Protoplasten transformiert.

Ein korrekter Klon wird ausgelesen, die zu transformierende DNA mit Hilfe von Standardverfahren isoliert und via Elektroporation (vgl. Beispiel 1) in B. thuringiensis HD1 cryB Zellen transformiert.

Die transformierten B. thuringiensis Zellen werden auf GYS-Agar (Sporulationsmedium), der X-gal als Zusatz enthält, ausplattiert.

Korrekt transformierte Klonen färben sich beim Einsetzen der Sporulation blau.

Ein B. thuringiensis HD1 cryB Stamm, der mit dem pXI61 Vektor transformiert wird, bleibt dagegen unter den gleichen Bedingungen weiss.

Die Restriktionsanalyse zeigt, dass bei korrekt transformierten Klonen ein intaktes pXI80 bzw. pXI101 Plasmid in den B. thuringiensis Zellen vorliegt.

### 8.4. β-Glaktosidasegen unter der Kontrolle eines Sporulationsabhängigen Promotors

B. thuringiensis HD1 cryB Zellen, die das pXI80 bzw. pXI101 Plasmid enthalten, werden wie zuvor beschrieben auf GYS-Medium kultiviert. Zu verschiedenen Zeitpunkten während der Wachstumsphase (sowohl während der vegetativen Wachstumsphase als auch während der Sporulationsphase) wird ein β-Galaktosidase-Assay gemäss dem bei ⁴⁴⁾ JH Miller beschriebenen Versuchsprotokoll ("Experiments in Molecular Genetics", Gold Spring Harbor Laboratory, 1972, Experiment 48 und 49) durchgeführt.

Die einzigen Unterschiede zu dem oben erwähnten Versuchsprotokoll beziehen sich auf die Verwendung von X-gal als chromogenes Substrat und auf die Messung des farbigen Hydrolyseproduktes, das von den Zellen nach ca. 1 Stunde gebildet wird.

Die Zellen werden anschliessend mit Hilfe einer Zentrifugation entfernt und die optische Dichte des Ueberstandes wird bei einer Wellenlänge von 650 nm bestimmt (OD₆₅₀).

Es zeigt sich, dass die Zunahme der optischen Dichte in Abhängigkeit von der Sporulation erfolgt. Die nicht-transformierten B. thuringiensis Zellen können dagegen das chromogene Substrat X-gal nicht hydrolysieren.

### Beispiel 9: Erstellen von Genbanken in Bacillus thuringiensis

### 9.1. Konstruktion von pXI200

Das Plasmid pXI200 ist ein Derivat des Plasmides pHY300 PLK, das von Toyobo Co., Ltd. (#PHY-001; Toyobo Co., Ltd., 2-8 Dojima Hama 2-Chome, Kita-ku, Osaka, 530 Japan) kommerziell bezogen werden kann. Plasmid pHY300 PLK, dessen Konstruktion in der Europäischen Patentanmeldung EP 162 725 beschrieben ist, enthält sowohl ein Ampicillin (amp^{R})- als auch ein Tetrazyclin (tetr^{R})-Resistenzgen.

Das Plasmid pHY300 PLK wird mit BglI und PvuI vollständig verdaut. Die resultierenden Restriktionsfragmente werden anschliessend mit Hilfe einer Agarosgelelektrophorese aufgetrennt. Das 4.4 Kb Fragment wird aus dem Agarosegel isoliert, gereinigt und anschliessend mit T4 DNA-Ligase religiert.

Der gesamte Ligationsansatz wird in E.coli HB101 transformiert. Nach Inkubation der transformierten E.coli HB101 Zellen bei 37°C auf einem selektiven L-Agar, der 20 µg/ml Tertrazyklin enthält, werden die Tetrazyklin-resistenten (Tc^{r}) Transformanten selektioniert. Aus einem Ampicillin sensitiven (Ap^{s}) Klon (100 µg/ml Ampicillin) kann dann ein Plasmid isoliert werden, das die PstI Schnittstelle im Ap^{r}-Gen zusammen mit dem 0.3 Kb umfassenden PvuI/BglI Fragment verloren hat. Dieses Plasmid erhält die Bezeichnung pXI200.

### 9.2. Klonierung von Protoxingenen aus Bacillus thuringiensis var. kurstaki HD1 in Bacillus thuringiensis HD1 cryβ.

Die Gesamt-DNA (50 µg) von Bacillus thuringiensis var. kurstaki HD1 wird durch Inkubation mit den Restriktionsenzymen Pst1 und Hpa1 vollständig verdaut. Die so gewonnenen Restriktionsfragmente werden auf einen kontinuierlichen Saccharosegradienten [5 % (w/v) - 23 % (w/v)] übertragen und dort mit Hilfe einer Dichtegradientenzentrifugation entsprechend ihrer Grösse aufgetrennt und in Fraktionen von je 500 µl gesammelt. Die Zentrifugation wird in einem TST 41-Rotor (Kontron Ausschwingrotor) bei max. 2.4 x 10⁵ g über einen Zeitraum von 16 Stunden und bei einer Temperatur von 15°C durchgeführt. Anschliessend werden je 50 µl Aliquots zur Bestimmung der Fragmentgrösse auf ein Agarosegel [0.8 % (w/v) Agarose in Tris Acetat EDTA oder Tris Borat EDTA; siehe ³³⁾Maniatis et al, 1982] übertragen. Diejenigen Fraktionen, welche Fragmente zwischen 3 Kb und 6 Kb enthalten werden gepoolt und durch Ethanolfällung auf ein Volumen von 10 µl konzentriert.

5 µg des in Beispiel 9.1 beschriebenen 'Shuttle' Vektors pXI200 werden mit den Restriktionsenzymen Pst1 und Sma1 vollständig verdaut. Die 5′-Phosphatgruppen der resultierenden Restriktionsfragmente werden anschliessend durch Behandlung mit alkalischer Phosphatase aus Kälberdarm entfernt.
0.2 µg bis 0.3 µg der zuvor isolierten HD1 DNA werden anschliessend mit 0.5 µg pXI200 Vektor DNA vermischt und unter Zugabe von 0.1 U (sog. 'Weiss Units'; eine Einheit T4 DNA-Ligase entspricht einer Enzymaktivität, die ausreicht 1 nM [³²P] aus Pyrophosphat bei einer Temperatur von 37°C und innerhalb eines Zeitraumes von 20 Minuten in ein Norit-absorbierbares Material umzuwandeln] T4 DNA-Ligase über Nacht bei 14°C inkubiert. Der gesamte Ligationsansatz wird anschliessend durch Elektroporation (vergl. Beispiel 1) direkt in Bacillus thuringiensis HD1 cryB-Zellen transformiert. Die elektroporierten B. thuringiensis Zellen werden dann auf einen selektiven Sporulationsagar, der 20 µg/ml Tetrazyklin als Selektionsmittel enthält, ausplattiert und bei einer Temperatur von 25°C bis zur vollständigen Sporulation inkubiert.

### 9.3. Herstellung monoklonaler Antikörper gegen B. thuringiensis Protoxinprotein

Die Herstellung monoklonaler Antikörper gegen δ-Endotoxin aus Bacillus thuringiensis var. kurstaki HD1 wird analog der Beschreibung bei ³⁶⁾Huber-Luka 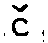 ,(1984) bzw. bei ³⁷⁾Huber-Luka 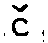 et al, (1986) durchgeführt.

Bei den für die Antikörperherstellung verwendeten Hybridoma-Zellen handelt es sich um Fusionsprodukte von Sp2/0-Ag Myelomzellen (beschrieben bei ⁴⁵⁾Shulman et al, 1978; kann bei der 'American Type Culture Collection' in Rockville, Maryland, USA bezogen werden) und Milzlymphozyten von BALB/c Mäusen, die zuvor mit δ-Endotoxin und B. thuringiensis kurstaki HD1 immunisiert wurden.

Auf diese Weise können monoklonale Antikörper erhalten werden, die spezifisch gegen das δ-Endotoxin von B. thruingiensis gerichtet sind. Besonders bevorzugt sind monoklonale Antikörper, die entweder spezifisch an ein Epitop in der N-terminalen Hälfte des Protoxin-Proteins binden (z.B. Antikörper 54.1 der Huber-Luka 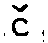 et al, 1986 Referenz) oder aber ein Epitop in dem bei Lepidopteren-aktiven Protoxinen konstanten Teil des Proteins, der C-terminalen Hälfte (z.B. Antikörper 83.16 der Huber-Luka 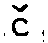 et al, 1986 Referenz), erkennen.

Es können aber durchaus auch andere monoklonale oder auch polyklonale Antikörper für das sich anschliessende Immunscreening (vgl. Beispiel 9.4) verwendet werden.

### 9.4. Immunologisches Screening

Für das immunologische Screening werden die gemäss Beispiel 9.3 hergestellten oder andere geeignete monoklonalen Antikörper verwendet.

Zunächst werden die nach der Sporulation der B.thuringiensis Zellen frei vorliegenden Kristallproteine mit Hilfe von Transfer Membranen (z.B. Pall Biodyne Transfer-Membran; Pall Ultrafine Filtration Corporation, Glen Cove, N.Y.) gebunden, indem diese für einen Zeitraum von ca. 5 Minuten auf die Platten aufgelegt werden. Die Filter werden danach 5 Minuten mit TBST Puffer [0.05 % (w/v) Tween 20, 10 mM Tris/HCl (pH 8.0), 150 mM NaCl in H₂O bidest.] gewaschen und anschliessend für 15 bis 30 Minuten zur Blockierung unspezifischer Bindungen in einem Gemisch aus TBST Puffer und 1 % (w/v) Magermilch inkubiert.

Die so vorbereiteten Filter werden dann über Nacht mit den Protoxinspezifischen Antikörpern [Antikörpergemisch aus 54.1 und 83.16, (³⁷⁾Huber-Luka 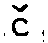 et al, (1986)] inkubiert. Die nicht gebundenen Antikörper werden durch dreimaliges Waschen des Filters mit TBST Puffer während jeweils 5 bis 10 Minuten entfernt. Zum Nachweis des Antikörpergebundenen Protoxins werden die Filter mit einem weiteren Antikörper inkubiert. Als sekundärer Antikörper fungiert dabei ein mit alkalischer Phosphatase markierter anti-Maus Antikörper, der beispielsweise bei Bio-Rad [Katalog # 170-6520, Ziegen anti-Maus IgG(H+L)-alkalische Phosphatase Konjugat] kommerziell erworben werden kann. Nach einer Inkubationszeit von 30 Minuten werden die nicht gebundenen sekundären Antikörper, wie zuvor beschrieben, durch dreimaliges Waschen (jeweils 5 bis 10 Minuten) der Filter mit TBST Puffer entfernt. Anschliessend werden die Filter mit einem Substratgemisch bestehend aus NBT ['p-nitro blue tetrazolium chloride; Nitro-Blaues Tetrazoliumchlorid] und BCIP [5-Brom-4-chlor-3-indolylphosphat-p-toluidin Salz] inkubiert. Die enzymatische Reaktion wird gemäss den Angaben des Hersteller [Bio-Rad; 1414 Harbour Way South, Richmond CA, 94804, USA] durchgeführt.

Positive, d.h. Protoxin-haltige Klone, können sehr einfach anhand ihrer violetten Färbung erkannt werden. Diese kommt zustande durch die enzymatische Reaktion der alkalischen Phosphatase mit dem zuvor erwähnten Substratgemisch. Aus der in Beispiel 9.2. beschriebenen Transformation mit dem dort angegebenen Ligationsansatz resultierten zwischen 800 und 1000 Transformanten. Davon zeigen 2 Kolonien eindeutig positive Signale in der zuvor beschriebenen Enzymreaktion.

Aus positiven Klonen, bei denen anhand der beschriebenen Enzymreaktion eine Expression des Protoxingens nachgewiesen werden konnte, wird Plasmid DNA isoliert. Mit Hilfe der Restriktionsanalyse und durch Vergleich mit bekannten Restriktionskarten können die klonierten Protoxingene weiter charakterisiert und schliesslich identifiziert werden.

Beide Klone enthalten ein rekombinantes Plasmid mit einem Insert von 4.3 Kb. Die folgende Restriktionsverdauungen mit HindIII, PvuII, EcoRI und XbaI erlauben eine Identifizierung des Gens auf dem Insert durch Vergleich mit den bekannten Restriktionskarten der Endotoxingene aus B. thuringiensis var kurstaki HD1. Es handelt sich in beiden Fällen um das kurhdl Gen, das auch als 5.3 Kb Protoxingen bekannt und bei ⁵⁾Geiser et al, 1986 beschrieben ist.

Dieses direkt in B. thuringiensis klonierte und mit Hilfe eines immunologischen Screenings identifizierte Gen hybridisiert darüberhinaus mit einem 1847 Bp umfassenden BamHI/HindIII Fragment des 5.3 Kb Gens im Plasmid pK36 (⁵⁾Geiser et al, 1986). Im SDS/PAGE zeigen beide Klone eine für das Protoxin typische Bande von 130'000 Dalton, die im Western Blot (⁴⁶⁾Towbin et al, 1979) spezifisch mit den zuvor beschriebenen (siehe Beispiel 9.4) monoklonalen Antikörpern reagieren.

### Tabellen

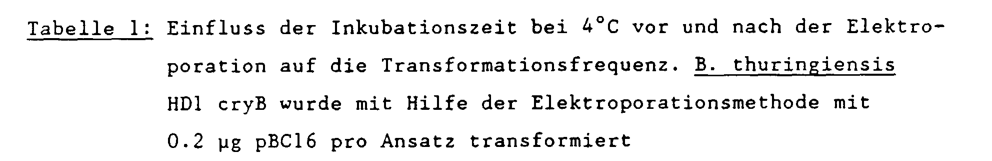

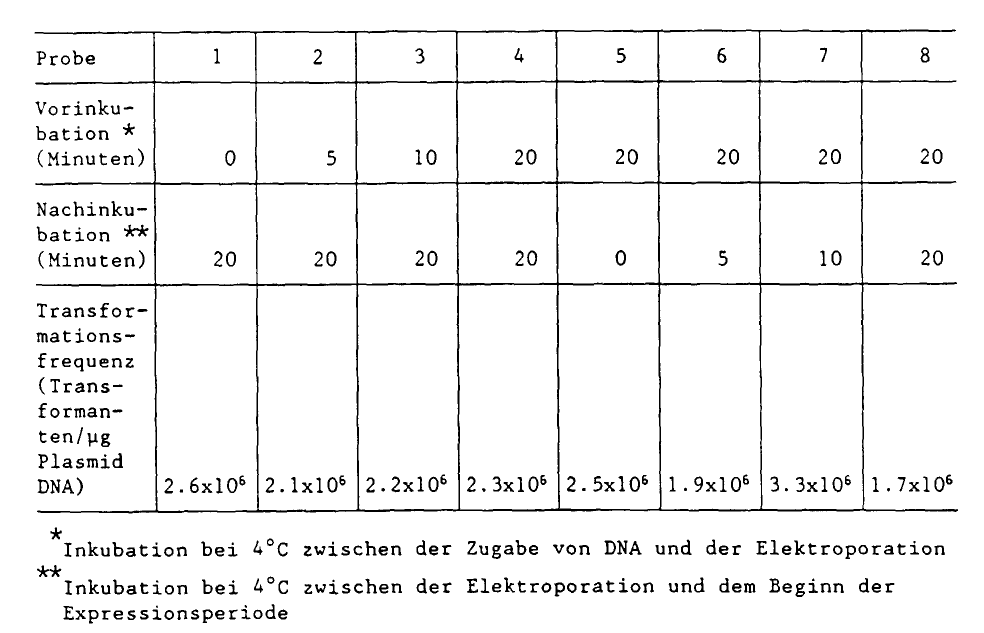

**Tabelle 2:**

| | | |
|---|---|---|
| Expression der Tetracyclin Resistenz von pBC 16 nach der Transformation in B. thuringiensis HD1cryB. B. thuringiensis HD1 cryB wurde mit pBC16 Plasmid DNA unter Verwendung des erfindungsgemässen Elektroporationsprotokolls transformiert. Nach unterschiedlichen Inkubationszeiten in LB Medium bei 30°C werden die transformierten Zellen durch Ausplattierung auf LB Agar, der 20 µg/ml Tetracyclin enthält, selektioniert. | | |

| Expressionszeit der Tetracyclinresistenz (Stunden) | Transformations-frequenz Trans-formanten/µgDNA) | Anzahl lebender Zellen |
|---|---|---|
| 0.5 | 0 | 4 x 10⁸ |
| 1 | 1.6 x 10⁶ | 10⁹ |
| 2 | 8.8 x 10⁶ | 1.4 x 10⁹ |
| 3 | 8 x 10⁶ | 1.6 x 10⁹ |

**Tabelle 3:**

| | | | | |
|---|---|---|---|---|
| Transformation des B. thuringiensis Stammes HD1 cryB mit verschiedenen Plasmiden | | | | |

| Plasmid | Herkunft Referenz | Resistenzmarker¹ | | Transformations-² |
|---|---|---|---|---|
| | | gram negativ | gram positiv | frequenz |
| natürlich vorkommende Plasmide | | | | |
| pBC16 | B. cereus | - | Tc | 1.9 x 10⁶ |
| pUB110 | Staphylococcus | | Km, Ble | 3.3 x 10^{6∗} |
| | aureus | - | | |
| pC194 | S. aureus | - | Cm | 6 x 10^{6∗} |
| pIM13 | B. subtilis | - | Em | 1.8 x 10⁵ |

| modifizierte Plasmide/Klonierungsvektoren | | | | |
|---|---|---|---|---|
| pBD64 | pUB110 replicon | - | Km, Cm | 5 x 10⁶ |
| pBD347 | pIM13 replicon, | - | Cm | 2.9 x 10⁵ |
| pBD348 | pIM13 replicon, | - | Em, Cm | 1.1 x 10⁵ |
| pUB1664 | pUB110 replicon, | - | Cm, Em | 3.5 x 10⁴ |

| "shuttle" Vektoren | | | | |
|---|---|---|---|---|
| pHV33 | pBR322/pC194, | Amp, Tc | Cm | < 50^{∗} |
| pK61 | pUC8/ pBC16, | Amp | Tc | 2.8 x 10⁴ |

| | | | | |
|---|---|---|---|---|
| 1: Tc: Tetrazyklin; Km: Kanamyzin; Ble: Bleomyzin; Cm: Chloramphenicol; Em: Erythromyzin | | | | |
| 2: Sämtliche Plasmid DNA stammt aus B. thuringiensis HD1 cryB mit Ausnahme von * isoliert von B. subtilis LBG4468 | | | | |

**Tabelle 4:**

| | | |
|---|---|---|
| Biotest von B. thuringiensis HDl cryB and HDl cryB (pXI 93) gegen Heliothis virescens. | | |
| Sprühgetrocknete sporulierte Kulturen (Sporen und (sofern vorhanden) Protoxinkristalle) wurden in den angegebenen Mengen der Diät von L-1 Larven von Heliothis virescens beigemischt. | | |

| Konzentration von Sporen und Protoxinkristallen (µg/g Diät) | Mortalität (%) von H. virescens verursacht durch: | |
|---|---|---|
| | HDl cryB | HDl cryB (pXI93) |
| 200 | 0 | 57 |
| 100 | 0 | 43 |
| 50 | 3 | 27 |
| 25 | 0 | 10 |
| 12.5 | 0 | 0 |

**Tabelle 5:**

| | |
|---|---|
| Transformierbarkeit von Stämmen von B. thuringiensis, B. cereus und B. subtilis. Alle Stämme wurden nach der in Beispiel 1 geschilderten Elektroporationsmethode mit dem Plasmid pBC16 transformiert. | |

| Stamm | Transformations-¹ Frequenz |
|---|---|
| B. thuringiensis var. kurstaki | |
| HDlcryB | 1 |
| HDl dipel | 0.25 |
| HDl-9 | 0.9 |
| HD 73 | 0.1 |
| HD 191 | 0.5 |
| B. thuringiensis var. thuringiensis | |
| HD 2-D6-4 | 13.8 |
| B. thuringiensis var. israelensis | |
| LBG B-4444 | 2.6 |
| B. cereus | |
| 569 K | 7.5 |
| B. subtilis | |
| LBG B-4468 | 0.0002 |

| | |
|---|---|
| ¹ relative Werte bezogen auf die als 1 definierte Transformationsfrequenz, die mit B. thuringiensis var. kurstaki HDl cryB erzielt wird. | |

### Hinterlegung von Mikroorganismen

Von jedem der im folgenden aufgelisteten Mikroorganismen, die im Rahmen der vorliegenden Erfindung verwendet werden, wurde eine Kultur bei der als internationale Hinterlegungsstelle anerkannten 'Deutschen Sammlung von Mikroorganismen' in Braunschweig, Bundesrepublik Deutschland, entsprechend den Anforderungen des Budapester Vertrages für die Internationale Anerkennung der Hinterlegung von Mikroorganismen zum Zwecke der Patentierung, hinterlegt. Eine Erklärung zur Lebensfähigkeit der hinterlegten Proben wurde durch die besagte Internationale Hinterlegungsstelle ausgefertigt.

### Hinterlegung von Mikroorganismen

| Mikroorganismen | Hinterlegungs-datum | Hinterlegungs-Nummer | Datum der Lebensfähigkeitsbescheinigung |
|---|---|---|---|
| HB 101 (pK36) (E. coli HB101 transformiert mit pK36 Plasmid DNA) | 4. März 1986 | DSM 3668 | 7. März 1986 |
| ∗HDI cryβ (Bacillus thuringiensis var. kurstaki HDl cryβ | 4. Mai 1988 | DSM 4574 | 4. Mai 1988 |
| ∗HDl cryβ (∗pK 61) (B. thuringiensis HDl cryβ transformiert mit ∗pK61 Plasmid DNA) | 4. Mai 1988 | DSM 4572 | 4. Mai 1988 |
| ∗HDl cryβ (∗pK 93) (B. thuringiensis HD1 cryβ transformiert mit ∗pK93 Plasmid DNA) | 4. Mai 1988 | DSM 4571 | 4. Mai 1988 |
| 569 K (Bacillus cereus 569 K) | 4. Mai 1988 | DSM 4575 | 4. Mai 1988 |
| 569 K (∗pK 93) (B. cereus 569 K transformiert mit ∗pK93 Plasmid DNA) | 4. Mai 1988 | DSM 4573 | 4. Mai 1988 |

Die im Prioritätsdokument für die Benennung der Plasmide gewählte interne Bezeichnung pK wurde für die Auslandsfassung durch die offiziell anerkannte Bezeichnung pXI ersetzt.

Auch die Bezeichung für die im Rahmen der Ausführungsbeispiele verwendete asporogene B. thuringiensis HD1 Mutante wurde von cryβ nach cryB geändert.

### Literaturverzeichnis

1. Goldberg L und Margalit J, Mosquito News, 37: 355-358, 1977
2. Krieg A et al, Z. Ang. Ent., 96: 500-508, 1983
3. Schnepf, HE and Whiteley HR, Proc. Natl. Acad. Sci, USA, 78: 2893-2897, 1981
4. Klier A et al, The EMBO J, 1: 791-799, 1982
5. Geiser M et al. Gene, 48: 109-118, 1986
6. Haider MZ et al., Gene, 52: 285-290, 1987
7. Gonzalez JM et al.. Proc. Natl. Acad. Sci. USA, 79: 6951-6955, 1982
8. Obukowicz MG et al., J. Bacteriol., 168: 982-989, 1986
9. Donovan LP et al., Mol. Gen. Genet., 214: 365-372, 1988
10. Schnepf HE and Whitely HR, J. Biol.Chem., 260: 6273, 1985
11. Klier A et al, Mol. Gen. Genet., 191: 257-262, 1983
12. Bibb JJ et al, Nature, 274: 398-400, 1978
13. Chang S and Cohen SN, Molec. Gen. Genet., 168: 111-115, 1979
14. Brown BJ and Carlton BC, J. Bacteriol., 142: 508-512, 1980
15. Kondo JK and McKay LL, Appl. Environ. Microbiol., 48: 252-259, 1984
16. Wirth R. et al, J. bacteriol., 165: 831-836, 1986
17. Yoshihama M et al,, J.Bacteriol., 162: 591-597, 1985
18. Alikhanian SJ et al, J. Bacteriol., 146, 7-9, 1981
19. Martin PA et al, J. Bacteriol., 145: 980-983, 1981
20. Fischer HM, Arch. Microbiol., 139: 213-217, 1984
21. Schall D, Genübertragung zwischen Isolaten von Bacillus thuringiensis durch Protoplastentransformation und -fusion. Dissertation, Universität Tübingen, 1986
22. Shivarova N, Zeitschr. Allgem. Mikrobiol., 23: 595-599, 1983
23. Youston AA und Rogoff MH, J. Bacteriol., 100: 1229-1236, 1969
24. Horinouchi S, and Weisblum B, J. Bacteriol., 150: 815-825, 1982
25. Polak J and Novick RP Plasmid, 7: 152-162, 1982
26. Mahler J and Halvorson HO, J. Gen. Microbiol., 120: 259-263, 1980
27. Gryczan T et al, J. Bacteriol., 141: 246-253, 1980
28. Vieira J and Messing J, Gene, 19: 259-268, 1982
29. Wong et al, J. Biol. Chem., 258: 1960 - 1967, 1983
30. Bolivar et al, Gene 2: 95 - 113, 1977
31. Norrander et al, Gene, 26: 101 - 104, 1983
32. Bevan et al, Nature, 304: 184 - 187, 1983
33. Maniatis et al, Molecular Cloning. A Laboratory Manual, Cold Spring Harbor Laboratory, Cold Spring Harbor, USA, 1982
34. Hinnen et al, Proc. Natl. Acad. Sci, USA, 75: 1929-1933, 1978
35. Young RA et al, Proc. Natl. Acad. Sci., USA, 80: 1194-1198, 1983
36. Huber-Luca 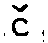 M, 'Zur Interaktion des delta-Endotoxins von Bacillus thuringiensis mit monoklonalen Antikörpern und Lipiden', Dissertation Nr. 7547, ETH Zürich, 1984
37. Huber-Luca 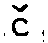 M et al, Infect. Immunol., 54: 228-232, 1986
38. McCutcheon's, 1986 International McCutcheon's Emulsifiers & Detergents, The Manufacturing Confections Publishing Co., Glen Rock, NJ, USA
39. Stahly DP et al, Biochem. Biophys. Res. Comm, 84: 581-588, 1978
40. Bernhard K et al, J. Bacteriol., 133: 897-903, 1978
41. Primrose SB, Ehrlich SD, Plasmid 6: 193-201, 1981
42. Huber-Lukac H, Dissertation, Eidgenössische Technische Hochschule, Zürich, Schweiz, No. 7050, 1982
43. Zoller JM and Smith M, Nucl. Acids Res., 10: 6487, 1982
44. Miller JH, Experiments in Molecular Genetics, Cold Spring Harbor Laboratory, 1972
45. Shulman et al, Nature, 276: 269, 1978
46. Towbin H et al, Proc. Natl. Acad. Sci., USA, 76: 4350-4354, 1979

### Patentliteratur

EP 162,725
EP 238,441
WO 86/01536
US-P 4,448,885
US-P 4,447,036
US-P 4,237,224
US-P 4,468,464

## Patentansprüche

1. Verfahren zur direkten *Einschleusung und Klonierung von DNA-Sequenzen in* gram positiven Bakterien ausgewählt aus der Gruppe bestehend aus B. thuringiensis und B. cereus, dadurch gekennzeichnet, dass man
(a) die einzuschleusende DNA isoliert;
(b) die so isolierte DNA in einem Vektor, der in der Lage ist in einer bakteriellen Wirtszelle, ausgewählt aus der Gruppe bestehend aus B. thuringiensis und B. cereus Zellen, zu replizieren, innerhalb eines heterologen Klonierungssystems kloniert;
(c) die so klonierte DNA isoliert und *via Elektroporation* direkt in *intakte* bakterielle Zellen *ausgewählt aus der Gruppe bestehend aus B. thuringiensis und B. cereus Zellen* einschleust, wobei die exponentielle Abklingzeit der Entladung(en) zwischen 2 ms und 50 ms beträgt; und
(d) die so transformierte bakterielle Zelle kultiviert und gegebenfalls die klonierte Vektor-DNA isoliert.

2. Verfahren gemäss Anspruch 1, dadurch gekennzeichnet, dass man die einzuschleusende DNA zunächst mit Expressionssequenzen ligiert, die in einer bakteriellen Zelle, ausgewählt aus der Gruppe bestehend aus B. thuringiensis und B. cereus Zellen, funktionsfähig sind und gegebenfalls das Expressionsprodukt isoliert.

3. Verfahren gemäss einem der Ansprüche 1 oder 2, dadurch gekennzeichnet, dass die direkte Einschleusung der DNA gemäss Schritt (c) via Elektroporation durchgeführt wird.

4. Verfahren gemäss Anspruch 3, dadurch gekennzeichnet, dass die Einschleusung *via* Elektroporation die folgenden Verfahrensmassnahmen umfasst:
a) Herstellen einer Zellsuspension mit geeigneter Zelldichte in einem für die Anzucht von bakteriellen Zellen ausgewählt aus der Gruppe bestehend aus B. thuringiensis und B. cereus Zellen geeigneten Kultivierungsmedium und bei einer für das Wachstum der Zellen ausreichenden Belüftung;
b) Abtrennen der Zellen aus der Zellsuspension und resuspendieren in einem für die nachfolgende Elektroporation geeigneten Inokulationspuffer;
c) Zugabe einer DNA-Probe in einer für die Elektroporation geeigneten Konzentration;
d) Einbringen des unter Punkt b) und c) beschriebenen Ansatzes in eine Elektroporationsapparatur,
e) Eine oder mehrere kurzzeitige Entladungen eines Kondensators über die Zellsuspension zur kurzfristigen Erzeugung hoher elektrischer Feldstärken wobei die exponentielle Abklingzeit der Entladung(en) zwischen 2ms und 50 ms beträgt;
f) gegebenenfalls Nachinkubation der elektroporierten Zellen;
g) Ausplattieren der elektroporierten Zellen auf einem geeigneten Selektionsmedium; und
h) Auslesen der transformierten Zellen.

5. Verfahren gemäss Anspruch 4, dadurch gekennzeichnet, dass man zur Herstellung der unter Punkt 4a) genannten Zellsuspension B. thuringiensis Sporen verwendet.

6. Verfahren gemäss Anspruch 4, dadurch gekennzeichnet, dass man bei der unter Punkt 4e) genannten Elektroporation zuvor tiefgefrorene und nunmehr aufgetaute Bacillus thuringiensis und/oder Bacillus cereus Zellen verwendet.

7. Verfahren gemäss Anspruch 4, dadurch gekennzeichnet, dass man für die Kultivierung von B. thuringiensis Zellen
a) komplexe Nährmedien mit leicht assimilierbarer Kohlenstoff- und Stickstoffquelle verwendet, wie sie üblicherweise für die Kultivierung von aeroben Bacillus-Arten eingesetzt werden;
a₁) gegebenenfalls zu den unter a) genannten Medien zusätzlich Vitamine und essentielle Metallionen zusetzt; oder
b) voll- oder halbsynthetische Nährmedien verwendet, die
b₁) eine komplexe oder aber eine definierte leicht assimilierbare Kohlenstoff- und Stickstoffquelle oder eine Kombination beider
sowie
b₂) essentielle Vitamine und Metallionen enthalten.

8. Verfahren gemäss Anspruch 4, dadurch gekennzeichnet, dass besagte Bacillus-Zellsuspension eine optische Dichte [OD₅₅₀] von 0.1 bis 1.0 aufweist

9. Verfahren gemäss Anspruch 4, dadurch gekennzeichnet, dass der unter Punkt b) genannte Inokulationspuffer ein Phosphatpuffer ist, der durch Zugabe eines osmotisch stabilisierenden Agens osmotisch stabilisiert ist.

10. Verfahren gemäss Anspruch 9, dadurch gekennzeichnet, dass besagter Phosphatpuffer als osmotisch stabilisierendes Agens Zucker oder Zuckeralkohole enthält

11. Verfahren gemäss Anspruch 10, dadurch gekennzeichnet, dass es sich bei besagtem stabilisierendem Agens um Saccharose handelt, die in einer Konzentration von 0.1 M bis 1.0 M vorliegt.

12. Verfahren gemäss Anspruch 9, dadurch gekennzeichnet, dass besagter Phosphatpuffer einen pH-Wert von pH 5.0 bis pH 8.0 aufweist.

13. Verfahren gemäss Anspruch 4, dadurch gekennzeichnet, dass die Inkubation der Bacillus-Zellen sowohl vor, während als auch nach der Elektroporation bei einer Temperatur zwischen 0°C und 35°C erfolgt.

14. Verfahren gemäss Anspruch 4, dadurch gekennzeichnet, dass die Inkubation der Bacillus-Zellen sowohl vor, während als auch nach der Elektroporation bei einer Temperatur zwischen 2°C und 15°C erfolgt.

15. Verfahren gemäss Anspruch 4, dadurch gekennzeichnet, dass die Konzentration der zugegebenen DNA-Probe 1 ng bis 20 µg beträgt.

16. Verfahren gemäss Anspruch 4, dadurch gekennzeichnet, dass die Feldstärkewerte 3000 V/cm bis 4500 V/cm betragen.

17. Verfahren gemäss Anspruch 4, dadurch gekennzeichnet, dass man die elektroporierten Zellen nach einer geeigneten Nachinkubationsphase auf Festmedien ausplattiert, die einen für die Selektionierung der transformierten Bacillus Zellen geeigneten Zusatz enthalten.

18. Verfahren gemäss Anspruch 20, dadurch gekennzeichnet, dass es sich bei besagtem Zusatz um ein für die Selektion von B. thuringiensis bzw. B. cereus geeignetes Antibiotikum ausgewählt aus der Gruppe bestehend aus Tetrazyklin, Kanamycin, Chloramphenicol, Erythromycin handelt.

19. Verfahren gemäss Anspruch 17, dadurch gekennzeichnet, dass es sich bei besagtem Zusatz um ein für die Selektion von B. thuringiensis bzw. B. cereus geeignetes chromogenes Substrat handelt.

20. Verfahren gemäss einem der Ansprüche 1 oder 2, dadurch gekennzeichnet, dass die für die Einschleusung in besagte bakterielle Zellen, ausgewählt aus der Gruppe bestehend aus B. thuringiensis und B. cereus, verwendete rekombinante DNA homologen oder heterologen Ursprungs ist oder es sich um eine Kombination aus homologer und heterologer DNA handelt.

21. Verfahren gemäss Anspruch 2, dadurch gekennzeichnet, dass besagte rekombinante DNA ein oder mehrere Strukturgene sowie 3'- und 5' flankierende, in Bacillus thuringiensis oder Bacillus cereus oder in beiden funktionsfähige, regulatorische Sequenzen enthält, die in operabler Weise mit dem (den) Strukturgen(en) verknüpft sind und somit die Expression besagten(r) Strukturgens(e) in Bacillus thuringiensis oder Bacillus cereus oder in beiden gewährleisten.

22. Verfahren gemäss Anspruch 21, dadurch gekennzeichnet, dass die 3'- und 5'-flankierenden DNA-Sequenzen einen sporulationsabhängigen Promotor aus Bacillus thuringiensis miteinschliessen.

23. Verfahren gemäss Anspruch 21, dadurch gekennzeichnet, dass besagtes Strukturgen ein δ-Endotoxin-Polypeptid kodiert, das natürlicherweise in B. thuringiensis vorkommt oder aber ein Polypeptid, das diesem im wesentlichen homolog ist, d.h. das zumindest im wesentlichen die Toxiszitätseigenschaften eines kristallinen δ-Endotoxin-Polypeptids aus B. thuringiensis aufweist.

24. Verfahren gemäss Anspruch 23, dadurch gekennzeichnet, dass besagte δ-Endotoxin-kodierende DNA-Sequenz im wesentlichen wenigstens dem Teil oder den Teilen der natürlichen δ-Endotoxin-kodierenden Sequenz homolog ist, der (die) für die insektizide Aktivität verantwortlich ist (sind).

25. Verfahren gemäss Anspruch 23, dadurch gekennzeichnet, dass besagtes Polypeptid einem δ-Endotoxin-Polypeptid aus geeigneten Unterarten von B. thuringiensis, ausgewählt aus der Gruppe bestehend aus kurstaki, berliner, alesti, sotto, tolworthi, dendrolimus, tenebrionis und israelensis im wesentlichen homolog ist.

26. Verfahren gemäss Anspruch 23, dadurch gekennzeichnet, dass es sich bei besagter δ-Endotoxin kodierender DNA Sequenz um ein DNA Fragment aus B. thuringiensis var. kurstaki HD1 handelt, das sich zwischen den Nukleotiden 156 und 3623 in Formel I befindet oder aber um jedes beliebige kürzere DNA-Fragment, das noch ein Polypeptid mit insektentoxischen Eigenschaften kodiert:

Formel I

10 20 30 40 50
GTTAACACCC TGGGTCAAAA ATTGATATTT AGTAAAATTA GTTGCACTTT

60 70 80 90 100
GTGCATTTTT TCATAAGATG AGTCATATGT TTTAAATTGT AGTAATGAAA

110 120 130 140 150
AACAGTATTA TATCATAATG AATTGGTATC TTAATAAAAG AGATGGAGGT

160 170 180 190 200
AACTTATGGA TAACAATCCG AACATCAATG AATGCATTCC TTATAATTGT

210 220 230 240 250
TTAAGTAACC CTGAAGTAGA AGTATTAGGT GGAGAAAGAA TAGAAACTGG

260 270 280 290 300

TTACACCCCA ATCGATATTT CCTTGTCGCT AACGCAATTT CTTTTGAGTG

310 320 330 340 350
AATTTGTTCC CGGTGCTGGA TTTGTGTTAG GACTAGTTGA TATAATATGG

360 370 380 390 400
GGAATTTTTG GTCCCTCTCA ATGGGACGCA TTTCTTGTAC AAATTGAACA

410 420 430 440 450
GTTAATTAAC CAAAGAATAG AAGAATTCGC TAGGAACCAA GCCATTTCTA

460 470 480 490 500
GATTAGAAGG ACTAAGCAAT CTTTATCAAA TTTACGCAGA ATCTTTTAGA

510 520 530 540 550
GAGTGGGAAG CAGATCCTAC TAATCCAGCA TTAAGAGAAG AGATGCGTAT

560 570 580 590 600
TCAATTCAAT GACATGAACA GTGCCCTTAC AACCGCTATT CCTCTTTTTG

610 620 630 640 650
CAGTTCAAAA TTATCAAGTT CCTCTTTTAT CAGTATATGT TCAAGCTGCA

660 670 680 690 700
AATTTACATT TATCAGTTTT GAGAGATGTT TCAGTGTTTG GACAAAGGTG

710 720 730 740 750
GGGATTTGAT GCCGCGACTA TCAATAGTCG TTATAATGAT TTAACTAGGC

760 770 780 790 800
TTATTGGCAA CTATACAGAT CATGCTGTAC GCTGGTACAA TACGGGATTA

810 820 830 840 850
GAGCGTGTAT GGGGACCGGA TTCTAGAGAT TGGATAAGAT ATAATCAATT

860 870 880 890 900

TAGAAGAGAA TTAACACTAA CTGTATTAGA TATCGTTTCT CTATTTCCGA

910 920 930 940 950
ACTATGATAG TAGAACGTAT CCAATTCGAA CAGTTTCCCA ATTAACAAGA

960 970 980 990 1000
GAAATTTATA CAAACCCAGT ATTAGAAAAT TTTGATGGTA GTTTTCGAGG

1010 1020 1030 1040 1050
CTCGGCTCAG GGCATAGAAG GAAGTATTAG GAGTCCACAT TTGATGGATA

1060 1070 1080 1090 1100
TACTTAACAG TATAACCATC TATACGGATG CTCATAGAGG AGAATATTAT

1110 1120 1130 1140 1150
TGGTCAGGGC ATCAAATAAT GGCTTCTCCT GTAGGGTTTT CGGGGCCAGA

1160 1170 1180 1190 1200
ATTCACTTTT CCGCTATATG GAACTATGGG AAATGCAGCT CCACAACAAC

1210 1220 1230 1240 1250
GTATTGTTGC TCAACTAGGT CAGGGCGTGT ATAGAACATT ATCGTCCACT

1260 1270 1280 1290 1300
TTATATAGAA GACCTTTTAA TATAGGGATA AATAATCAAC AACTATCTGT

1310 1320 1330 1340 1350
TCTTGACGGG ACAGAATTTG CTTATGGAAC CTCCTCAAAT TTGCCATCCG

1360 1370 1380 1390 1400
CTGTATACAG AAAAAGCGGA ACGGTAGATT CGCTGGATGA AATACCGCCA

1410 1420 1430 1440 1450
CAGAATAACA ACGTGCCACC TAGGCAAGGA TTTAGTCATC GATTAAGCCA

1460 1470 1480 1490 1500

TGTTTCAATG TTTCGTTCAG GCTTTAGTAA TAGTAGTGTA AGTATAATAA

1510 1520 1530 1540 1550
GAGCTCCTAT GTTCTCTTGG ATACATCGTA GTGCTGAATT TAATAATATA

1560 1570 1580 1590 1600
ATTCCTTCAT CACAAATTAC ACAAATACCT TTAACAAAAT CTACTAATCT

1610 1620 1630 1640 1650
TGGCTCTGGA ACTTCTGTCG TTAAAGGACC AGGATTTACA GGAGGAGATA

1660 1670 1680 1690 1700
TTCTTCGAAG AACTTCACCT GGCCAGATTT CAACCTTAAG AGTAAATATT

1710 1720 1730 1740 1750
ACTGCACCAT TATCACAAAG ATATCGGGTA AGAATTCGCT ACGCTTCTAC

1760 1770 1780 1790 1800
CACAAATTTA CAATTCCATA CATCAATTGA CGGAAGACCT ATTAATCAGG

1810 1820 1830 1840 1850
GGAATTTTTC AGCAACTATG AGTAGTGGGA GTAATTTACA GTCCGGAAGC

1860 1870 1880 1890 1900
TTTAGGACTG TAGGTTTTAC TACTCCGTTT AACTTTTCAA ATGGATCAAG

1910 1920 1930 1940 1950
TGTATTTACG TTAAGTGCTC ATGTCTTCAA TTCAGGCAAT GAAGTTTATA

1960 1970 1980 1990 2000
TAGATCGAAT TGAATTTGTT CCGGCAGAAG TAACCTTTGA GGCAGAATAT

2010 2020 2030 2040 2050
GATTTAGAAA GAGCACAAAA GGCGGTGAAT GAGCTGTTTA CTTCTTCCAA

2060 2070 2080 2090 2100

TCAAATCGGG TTAAAAACAG ATGTGACGGA TTATCATATT GATCAAGTAT

2110 2120 2130 2140 2150
CCAATTTAGT TGAGTGTTTA TCTGATGAAT TTTGTCTGGA TGAAAAAAAA

2160 2170 2180 2190 2200
GAATTGTCCG AGAAAGTCAA ACATGCGAAG CGACTTAGTG ATGAGCGGAA

2210 2220 2230 2240 2250
TTTACTTCAA GATCCAAACT TTAGAGGGAT CAATAGACAA CTAGACCGTG

2260 2270 2280 2290 2300
GCTGGAGAGG AAGTACGGAT ATTACCATCC AAGGAGGCGA TGACGTATTC

2310 2320 2330 2340 2350
AAAGAGAATT ACGTTACGCT ATTGGGTACC TTTGATGAGT GCTATCCAAC

2360 2370 2380 2390 2400
GTATTTATAT CAAAAAATAG ATGAGTCGAA ATTAAAAGCC TATACCCGTT

2410 2420 2430 2440 2450
ACCAATTAAG AGGGTATATC GAAGATAGTC AAGACTTAGA AATCTATTTA

2460 2470 2480 2490 2500
ATTCGCTACA ATGCCAAACA CGAAACAGTA AATGTGCCAG GTACGGGTTC

2510 2520 2530 2540 2550
CTTATGGCCG CTTTCAGCCC CAAGTCCAAT CGGAAAATGT GCCCATCATT

2560 2570 2580 2590 2600
CCCATCATTT CTCCTTGGAC ATTGATGTTG GATGTACAGA CTTAAATGAG

2610 2620 2630 2640 2650
GACTTAGGTG TATGGGTGAT ATTCAAGATT AAGACGCAAG ATGGCCATGC

2660 2670 2680 2690 2700

AAGACTAGGA AATCTAGAAT TTCTCGAAGA GAAACCATTA GTAGGAGAAG

2710 2720 2730 2740 2750
CACTAGCTCG TGTGAAAAGA GCGGAGAAAA AATGGAGAGA CAAACGTGAA

2760 2770 2780 2790 2800
AAATTGGAAT GGGAAACAAA TATTGTTTAT AAAGAGGCAA AAGAATCTGT

2810 2820 2830 2840 2850
AGATGCTTTA TTTGTAAACT CTCAATATGA TAGATTACAA GCGGATACCA

2860 2870 2880 2890 2900
ACATCGCGAT GATTCATGCG GCAGATAAAC GCGTTCATAG CATTCGAGAA

2910 2920 2930 2940 2950
GCTTATCTGC CTGAGCTGTC TGTGATTCCG GGTGTCAATG CGGCTATTTT

2960 2970 2980 2990 3000
TGAAGAATTA GAAGGGCGTA TTTTCACTGC ATTCTCCCTA TATGATGCGA

3010 3020 3030 3040 3050
GAAATGTCAT TAAAAATGGT GATTTTAATA ATGGCTTATC CTGCTGGAAC

3060 3070 3080 3090 3100
GTGAAAGGGC ATGTAGATGT AGAAGAACAA AACAACCACC GTTCGGTCCT

3110 3120 3130 3140 3150
TGTTGTTCCG GAATGGGAAG CAGAAGTGTC ACAAGAAGTT CGTGTCTGTC

3160 3170 3180 3190 3200
CGGGTCGTGG CTATATCCTT CGTGTCACAG CGTACAAGGA GGGATATGGA

3210 3220 3230 3240 3250
GAAGGTTGCG TAACCATTCA TGAGATCGAG AACAATACAG ACGAACTGAA

3260 3270 3280 3290 3300

GTTTAGCAAC TGTGTAGAAG AGGAAGTATA TCCAAACAAC ACGGTAACGT

3310 3320 3330 3340 3350
GTAATGATTA TACTGCGACT CAAGAAGAAT ATGAGGGTAC GTACACTTCT

3360 3370 3380 3390 3400
CGTAATCGAG GATATGACGG AGCCTATGAA AGCAATTCTT CTGTACCAGC

3410 3420 3430 3440 3450
TGATTATGCA TCAGCCTATG AAGAAAAAGC ATATACAGAT GGACGAAGAG

3460 3470 3480 3490 3500
ACAATCCTTG TGAATCTAAC AGAGGATATG GGGATTACAC ACCACTACCA

3510 3520 3530 3540 3550
GCTGGCTATG TGACAAAAGA ATTAGAGTAC TTCCCAGAAA CCGATAAGGT

3560 3570 3580 3590 3600
ATGGATTGAG ATCGGAGAAA CGGAAGGAAC ATTCATCGTG GACAGCGTGG

3610 3620 3630 3640 3650
AATTACTTCT TATGGAGGAA TAATATATGC TTTATAATGT AAGGTGTGCA

3660 3670 3680 3690 3700
AATAAAGAAT GATTACTGAC TTGTATTGAC AGATAAATAA GGAAATTTTT

3710 3720 3730 3740 3750
ATATGAATAA AAAACGGGCA TCACTCTTAA AAGAATGATG TCCGTTTTTT

3760 3770 3780 3790 3800
GTATGATTTA ACGAGTGATA TTTAAATGTT TTTTTTGCGA AGGCTTTACT

3810 3820 3830 3840 3850
TAACGGGGTA CCGCCACATG CCCATCAACT TAAGAATTTG CACTACCCCC

3860 3870 3880 3890 3900

AAGTGTCAAA AAACGTTATT CTTTCTAAAA AGCTAGCTAG AAAGGATGAC

3910 3920 3930 3940 3950
ATTTTTTATG AATCTTTCAA TTCAAGATGA ATTACAACTA TTTTCTGAAG

3960 3970 3980 3990 4000
AGCTGTATCG TCATTTAACC CCTTCTCTTT TGGAAGAACT CGCTAAAGAA

4010 4020 4030 4040 4050
TTAGGTTTTG TAAAAAGAAA ACGAAAGTTT TCAGGAAATG AATTAGCTAC

4060 4070 4080 4090 4100
CATATGTATC TGGGGCAGTC AACGTACAGC GAGTGATTCT CTCGTTCGAC

4110 4120 4130 4140 4150
TATGCAGTCA ATTACACGCC GCCACAGCAC TCTTATGAGT CCAGAAGGAC

4160 4170 4180 4190 4200
TCAATAAACG CTTTGATAAA AAAGCGGTTG AATTTTTGAA ATATATTTTT

4210 4220 4230 4240 4250
TCTGCATTAT GGAAAAGTAA ACTTTGTAAA ACATCAGCCA TTTCAAGTGC

4260 4270 4280 4290 4300
AGCACTCACG TATTTTCAAC GAATCCGTAT TTTAGATGCG ACGATTTTCC

4310 4320 4330 4340 4350
AAGTACCGAA ACATTTAGCA CATGTATATC CTGGGTCAGG TGGTTGTGCA

4360
CAAACTGCAG

27. Verfahren gemäss einem der Ansprüche 1 oder 2, dadurch gekennzeichnet, dass es sich bei dem in Schritt (b) verwendeten Vektor um einen bifunktionellen Vektor handelt, der nicht nur in der Lage ist in bakteriellen Zellen ausgewählt aus der Gruppe bestehend aus B. thuringiensis und B. cereus zu replizieren sondern der zumindest noch in einem oder aber in mehreren anderen heterologen Wirtsorganismen replizieren kann und der sowohl in den homologen als auch in den heterologen Wirtssystemen identifizierbar ist.

28. Verfahren gemäss Anspruch 27, dadurch gekennzeichnet, dass es sich bei besagten heterologen Wirtsorganismen um
a) prokaryontische Organismen, ausgewählt aus der Gruppe bestehend aus den Gattungen Bacillus, Staphylococcus, Streptococcus, Streptomyces, Pseudomonas, Escherichia, Agrobacterium, Salmonella, Erwinia, etc.,
oder
b) eukaryontische Organismen ausgewählt aus der Gruppe bestehend aus Hefe, tierischen und pflanzlichen Zellen, etc. handelt

29. Verfahren gemäss Anspruch 28, dadurch gekennzeichnet, dass es sich bei besagtem heterologen Wirtsorganismus um E. coli handelt.

30. Verfahren gemäss Anspruch 27, dadurch gekennzeichnet, dass besagter bifunktioneller Vektor neben den für die Replikation und Selektion in homologen und heterologen Wirtssystemen essentiellen Funktionen zusätzlich noch ein Strukturgen unter der Kontrolle von in bakteriellen Zellen, ausgewählt aus der Gruppe bestehend aus B. thuringiensis und B. cereus Zellen, funktionsfähigen Expressionssequenzen enthält, das für ein δ-Endotoxin-Polypeptid kodiert, das natürlicherweise in B. thuringiensis vorkommt oder aber ein Polypeptid, das diesem im wesentlichen homolog ist, d.h. das zumindest im wesentlichen die Toxizitätseigenschaften eines kristallinen δ-Endotoxin Polypeptids aus B. thuringiensis aufweist.

31. Verfahren gemäss Anspruch 30, dadurch gekennzeichnet, dass besagte Expressionssignale einen Sporulations-abhängigen Promotor aus B. thuringiensis miteinschliessen.

32. Verfahren gemäss Anspruch 1, dadurch gekennzeichnet, dass die einzuschleusende DNA gemäss Schritt (a) durch Verdauung der Gesamt-DNA eines bakteiellen Donors ausgewählt aus der Gruppe bestehend aus B. thuringiensis und B. cereus erhältlich ist.

## Claims

1. A process for the direct introduction of DNA sequences into, and cloning thereof in, gram positive bacteria selected from the group consisting of B. thuringiensis and B. cereus, which comprises
(a) isolating the DNA to be introduced;
(b) cloning the thus isolated DNA within a heterologous cloning system in a vector that is capable of replicating in a bacterial host cell selected from the group consisting of B. thuringiensis and B. cereus cells;
(c) isolating the thus cloned DNA and introducing it via electroporation directly into intact bacterial cells selected from the group consisting of B. thuringiensis and B. cereus cells, the exponential decay time of the discharge(s) being from 2 ms to 50 ms; and
(d) culturing the thus transformed bacterial cell and, where appropriate, isolating the cloned vector-DNA.

2. A process according to claim 1, wherein the DNA to be introduced is first of all ligated with expression sequences that are capable of functioning in a bacterial cell selected from the group consisting of B. thuringiensis and B. cereus cells, and the expression product is optionally isolated.

3. A process according to either claim 1 or claim 2, which comprises carrying out the direct introduction of the DNA according to Step (c) via electroporation.

4. A process according to claim 3, wherein the introduction via electroporation comprises the following process steps:
a) preparation of a cell suspension of suitable cell density in a culture medium suitable for growing bacterial cells selected from the group consisting of B. thuringiensis and B. cereus cells and with aeration adequate for the growth of the cells;
b) separation of the cells from the cell suspension and resuspension in an inoculation buffer suitable for the subsequent electroporation;
c) addition of a DNA sample in a concentration suitable for the electroporation;
d) introduction of the mixture described under points b) and c) into an electroporation apparatus;
e) one or more brief discharges of a capacitor across the cell suspension for the short-term production of high electric field strengths, the exponential decay time of the discharge(s) being from 2 ms to 50 ms;
f) optional reincubation of the electroporated cells;
g) plating out of the electroporated cells onto a suitable selection medium; and
h) selection of the transformed cells.

5. A process according to claim 4, which comprises using B. thuringiensis spores for the preparation of the cell suspension mentioned under point 4a).

6. A process according to claim 4, which comprises using previously deep-frozen and then thawed Bacillus thuringiensis and/or Bacillus cereus cells in the electroporation mentioned under point 4e).

7. A process according to claim 4, wherein for culturing B. thuringiensis cells
a) complex nutrient media with readily assimilable carbon and nitrogen sources as conventionally employed for culturing aerobic Bacillus species are used;
a₁) if desired, in addition vitamins and essential metal ions are added to the media mentioned under a) ; or
b) fully synthetic or semi-synthetic nutrient media are used that comprise
b₁) a complex or alternatively a defined readily assimilable carbon and nitrogen source or a combination of the two
and also
b₂) essential vitamins and metal ions.

8. A process according to claim 4, wherein the said Bacillus cell suspension has an optical density [OD₅₅₀] of from 0.1 to 1.0.

9. A process according to claim 4, wherein the inoculation buffer mentioned under point b) is a phosphate buffer that has been osmotically stabilised by the addition of an osmotically stabilising agent.

10. A process according to claim 9, wherein the said phosphate buffer comprises sugars or sugar alcohols as osmotically stabilising agent.

11. A process according to claim 10, wherein the said stabilising agent is saccharose, which is present in a concentration of from 0.1M to 1.0M.

12. A process according to claim 9, wherein the said phosphate buffer has a pH value of from pH 5.0 to pH 8.0.

13. A process according to claim 4, wherein the incubature of from 0°C to 35°C before, during and after electroporation.

14. A process according to claim 4, wherein the incubation of the Bacillus cells is carried out at a temperature of from 2°C to 15°C before, during and after electroporation.

15. A process according to claim 4, wherein the concentration of the added DNA sample is from 1 ng to 20 µg.

16. A process according to claim 4, wherein the field strengths are from 3000 V/cm to 4500 V/cm.

17. A process according to claim 4, which comprises plating out the electroporated cells, after a suitable reincubation phase, onto solid media that comprise an additive suitable for the selection of the transformed Bacillus cells.

18. A process according to claim 20, wherein the said additive is an antibiotic suitable for the selection of B. thuringiensis or B. cereus selected from the group consisting of tetracycline, kanamycin, chloramphenicol and erythromycin.

19. A process according to claim 17, wherein the said additive is a chromogenic substrate suitable for the selection of B. thuringiensis or B. cereus.

20. A process according to either claim 1 or claim 2, wherein the recombinant DNA used for the introduction into the said bacterial cells selected from the group consisting of B. thuringiensis and B. cereus is of homologous or heterologous origin or is a combination of homologous and heterologous DNA.

21. A process according to claim 2, wherein the said recombinant DNA comprises one or more structural genes and 3' and 5' flanking regulatory sequences that are capable of functioning in Bacillus thuringiensis or Bacillus cereus or in both, which sequences are operably joined to the structural gene(s) and thus ensure the expression of the said structural gene(s) in Bacillus thuringiensis or Bacillus cereus or in both.

22. A process according to claim 21, wherein the 3' and 5' flanking DNA sequences include a sporulation-dependent promoter from Bacillus thuringiensis.

23. A process according to claim 21, wherein the said structural gene codes for a δ-endotoxin polypeptide that occurs naturally in B. thuringiensis, or alternatively for a polypeptide that is substantially homologous therewith, that is to say that at least has substantially the toxicity properties of a crystalline 6-endotoxin polypeptide from B. thuringiensis.

24. A process according to claim 23, wherein the said δ-endotoxin-encoding DNA sequence is substantially homologous with at least the part or parts of the natural δ-endotoxin-encoding sequence that is (are) responsible for the insecticidal activity.

25. A process according to claim 23, wherein the said polypeptide is substantially homologous with a δ-endotoxin polypeptide of a suitable sub-species of B. thuringiensis selected from the group consisting of kurstaki, berliner, alesti, sotto, tolworthi, dendrolimus, tenebrionis and israelensis.

26. A process according to claim 23, wherein the said δ-endotoxin-encoding DNA sequence is a DNA fragment of B. thuringiensis var. kurstaki HDl located between nucleotides 156 and 3623 in formula I, or is any shorter DNA fragment that still codes for a polypeptide having insect-toxic properties:

Formula I

10 20 30 40 50
GTTAACACCC TGGGTCAAAA ATTGATATTT AGTAAAATTA GTTGCACTTT

60 70 80 90 100
GTGCATTTTT TCATAAGATG AGTCATATGT TTTAAATTGT AGTAATGAAA

110 120 130 140 150
AACAGTATTA TATCATAATG AATTGGTATC TTAATAAAAG AGATGGAGGT

160 170 180 190 200
AACTTATGGA TAACAATCCG AACATCAATG AATGCATTCC TTATAATTGT

210 220 230 240 250
TTAAGTAACC CTGAAGTAGA AGTATTAGGT GGAGAAAGAA TAGAAACTGG

260 270 280 290 300

TTACACCCCA ATCGATATTT CCTTGTCGCT AACGCAATTT CTTTTGAGTG

310 320 330 340 350
AATTTGTTCC CGGTGCTGGA TTTGTGTTAG GACTAGTTGA TATAATATGG

360 370 380 390 400
GGAATTTTTG GTCCCTCTCA ATGGGACGCA TTTCTTGTAC AAATTGAACA

410 420 430 440 450
GTTAATTAAC CAAAGAATAG AAGAATTCGC TAGGAACCAA GCCATTTCTA

460 470 480 490 500
GATTAGAAGG ACTAAGCAAT CTTTATCAAA TTTACGCAGA ATCTTTTAGA

510 520 530 540 550
GAGTGGGAAG CAGATCCTAC TAATCCAGCA TTAAGAGAAG AGATGCGTAT

560 570 580 590 600
TCAATTCAAT GACATGAACA GTGCCCTTAC AACCGCTATT CCTCTTTTTG

610 620 630 640 650
CAGTTCAAAA TTATCAAGTT CCTCTTTTAT CAGTATATGT TCAAGCTGCA

660 670 680 690 700
AATTTACATT TATCAGTTTT GAGAGATGTT TCAGTGTTTG GACAAAGGTG

710 720 730 740 750
GGGATTTGAT GCCGCGACTA TCAATAGTCG TTATAATGAT TTAACTAGGC

760 770 780 790 800
TTATTGGCAA CTATACAGAT CATGCTGTAC GCTGGTACAA TACGGGATTA

810 820 830 840 850
GAGCGTGTAT GGGGACCGGA TTCTAGAGAT TGGATAAGAT ATAATCAATT

860 870 880 890 900

TAGAAGAGAA TTAACACTAA CTGTATTAGA TATCGTTTCT CTATTTCCGA

910 920 930 940 950
ACTATGATAG TAGAACGTAT CCAATTCGAA CAGTTTCCCA ATTAACAAGA

960 970 980 990 1000
GAAATTTATA CAAACCCAGT ATTAGAAAAT TTTGATGGTA GTTTTCGAGG

1010 1020 1030 1040 1050
CTCGGCTCAG GGCATAGAAG GAAGTATTAG GAGTCCACAT TTGATGGATA

1060 1070 1080 1090 1100
TACTTAACAG TATAACCATC TATACGGATG CTCATAGAGG AGAATATTAT

1110 1120 1130 1140 1150
TGGTCAGGGC ATCAAATAAT GGCTTCTCCT GTAGGGTTTT CGGGGCCAGA

1160 1170 1180 1190 1200
ATTCACTTTT CCGCTATATG GAACTATGGG AAATGCAGCT CCACAACAAC

1210 1220 1230 1240 1250
GTATTGTTGC TCAACTAGGT CAGGGCGTGT ATAGAACATT ATCGTCCACT

1260 1270 1280 1290 1300
TTATATAGAA GACCTTTTAA TATAGGGATA AATAATCAAC AACTATCTGT

1310 1320 1330 1340 1350
TCTTGACGGG ACAGAATTTG CTTATGGAAC CTCCTCAAAT TTGCCATCCG

1360 1370 1380 1390 1400
CTGTATACAG AAAAAGCGGA ACGGTAGATT CGCTGGATGA AATACCGCCA

1410 1420 1430 1440 1450
CAGAATAACA ACGTGCCACC TAGGCAAGGA TTTAGTCATC GATTAAGCCA

1460 1470 1480 1490 1500

TGTTTCAATG TTTCGTTCAG GCTTTAGTAA TAGTAGTGTA AGTATAATAA

1510 1520 1530 1540 1550
GAGCTCCTAT GTTCTCTTGG ATACATCGTA GTGCTGAATT TAATAATATA

1560 1570 1580 1590 1600
ATTCCTTCAT CACAAATTAC ACAAATACCT TTAACAAAAT CTACTAATCT

1610 1620 1630 1640 1650
TGGCTCTGGA ACTTCTGTCG TTAAAGGACC AGGATTTACA GGAGGAGATA

1660 1670 1680 1690 1700
TTCTTCGAAG AACTTCACCT GGCCAGATTT CAACCTTAAG AGTAAATATT

1710 1720 1730 1740 1750
ACTGCACCAT TATCACAAAG ATATCGGGTA AGAATTCGCT ACGCTTCTAC

1760 1770 1780 1790 1800
CACAAATTTA CAATTCCATA CATCAATTGA CGGAAGACCT ATTAATCAGG

1810 1820 1830 1840 1850
GGAATTTTTC AGCAACTATG AGTAGTGGGA GTAATTTACA GTCCGGAAGC

1860 1870 1880 1890 1900
TTTAGGACTG TAGGTTTTAC TACTCCGTTT AACTTTTCAA ATGGATCAAG

1910 1920 1930 1940 1950
TGTATTTACG TTAAGTGCTC ATGTCTTCAA TTCAGGCAAT GAAGTTTATA

1960 1970 1980 1990 2000
TAGATCGAAT TGAATTTGTT CCGGCAGAAG TAACCTTTGA GGCAGAATAT

2010 2020 2030 2040 2050
GATTTAGAAA GAGCACAAAA GGCGGTGAAT GAGCTGTTTA CTTCTTCCAA

2060 2070 2080 2090 2100

TCAAATCGGG TTAAAAACAG ATGTGACGGA TTATCATATT GATCAAGTAT

2110 2120 2130 2140 2150
CCAATTTAGT TGAGTGTTTA TCTGATGAAT TTTGTCTGGA TGAAAAAAAA

2160 2170 2180 2190 2200
GAATTGTCCG AGAAAGTCAA ACATGCGAAG CGACTTAGTG ATGAGCGGAA

2210 2220 2230 2240 2250
TTTACTTCAA GATCCAAACT TTAGAGGGAT CAATAGACAA CTAGACCGTG

2260 2270 2280 2290 2300
GCTGGAGAGG AAGTACGGAT ATTACCATCC AAGGAGGCGA TGACGTATTC

2310 2320 2330 2340 2350
AAAGAGAATT ACGTTACGCT ATTGGGTACC TTTGATGAGT GCTATCCAAC

2360 2370 2380 2390 2400
GTATTTATAT CAAAAAATAG ATGAGTCGAA ATTAAAAGCC TATACCCGTT

2410 2420 2430 2440 2450
ACCAATTAAG AGGGTATATC GAAGATAGTC AAGACTTAGA AATCTATTTA

2460 2470 2480 2490 2500
ATTCGCTACA ATGCCAAACA CGAAACAGTA AATGTGCCAG GTACGGGTTC

2510 2520 2530 2540 2550
CTTATGGCCG CTTTCAGCCC CAAGTCCAAT CGGAAAATGT GCCCATCATT

2560 2570 2580 2590 2600
CCCATCATTT CTCCTTGGAC ATTGATGTTG GATGTACAGA CTTAAATGAG

2610 2620 2630 2640 2650
GACTTAGGTG TATGGGTGAT ATTCAAGATT AAGACGCAAG ATGGCCATGC

2660 2670 2680 2690 2700

AAGACTAGGA AATCTAGAAT TTCTCGAAGA GAAACCATTA GTAGGAGAAG

2710 2720 2730 2740 2750
CACTAGCTCG TGTGAAAAGA GCGGAGAAAA AATGGAGAGA CAAACGTGAA

2760 2770 2780 2790 2800
AAATTGGAAT GGGAAACAAA TATTGTTTAT AAAGAGGCAA AAGAATCTGT

2810 2820 2830 2840 2850
AGATGCTTTA TTTGTAAACT CTCAATATGA TAGATTACAA GCGGATACCA

2860 2870 2880 2890 2900
ACATCGCGAT GATTCATGCG GCAGATAAAC GCGTTCATAG CATTCGAGAA

2910 2920 2930 2940 2950
GCTTATCTGC CTGAGCTGTC TGTGATTCCG GGTGTCAATG CGGCTATTTT

2960 2970 2980 2990 3000
TGAAGAATTA GAAGGGCGTA TTTTCACTGC ATTCTCCCTA TATGATGCGA

3010 3020 3030 3040 3050
GAAATGTCAT TAAAAATGGT GATTTTAATA ATGGCTTATC CTGCTGGAAC

3060 3070 3080 3090 3100
GTGAAAGGGC ATGTAGATGT AGAAGAACAA AACAACCACC GTTCGGTCCT

3110 3120 3130 3140 3150
TGTTGTTCCG GAATGGGAAG CAGAAGTGTC ACAAGAAGTT CGTGTCTGTC

3160 3170 3180 3190 3200
CGGGTCGTGG CTATATCCTT CGTGTCACAG CGTACAAGGA GGGATATGGA

3210 3220 3230 3240 3250
GAAGGTTGCG TAACCATTCA TGAGATCGAG AACAATACAG ACGAACTGAA

3260 3270 3280 3290 3300

GTTTAGCAAC TGTGTAGAAG AGGAAGTATA TCCAAACAAC ACGGTAACGT

3310 3320 3330 3340 3350
GTAATGATTA TACTGCGACT CAAGAAGAAT ATGAGGGTAC GTACACTTCT

3360 3370 3380 3390 3400
CGTAATCGAG GATATGACGG AGCCTATGAA AGCAATTCTT CTGTACCAGC

3410 3420 3430 3440 3450
TGATTATGCA TCAGCCTATG AAGAAAAAGC ATATACAGAT GGACGAAGAG

3460 3470 3480 3490 3500
ACAATCCTTG TGAATCTAAC AGAGGATATG GGGATTACAC ACCACTACCA

3510 3520 3530 3540 3550
GCTGGCTATG TGACAAAAGA ATTAGAGTAC TTCCCAGAAA CCGATAAGGT

3560 3570 3580 3590 3600
ATGGATTGAG ATCGGAGAAA CGGAAGGAAC ATTCATCGTG GACAGCGTGG

3610 3620 3630 3640 3650
AATTACTTCT TATGGAGGAA TAATATATGC TTTATAATGT AAGGTGTGCA

3660 3670 3680 3690 3700
AATAAAGAAT GATTACTGAC TTGTATTGAC AGATAAATAA GGAAATTTTT

3710 3720 3730 3740 3750
ATATGAATAA AAAACGGGCA TCACTCTTAA AAGAATGATG TCCGTTTTTT

3760 3770 3780 3790 3800
GTATGATTTA ACGAGTGATA TTTAAATGTT TTTTTTGCGA AGGCTTTACT

3810 3820 3830 3840 3850
TAACGGGGTA CCGCCACATG CCCATCAACT TAAGAATTTG CACTACCCCC

3860 3870 3880 3890 3900

AAGTGTCAAA AAACGTTATT CTTTCTAAAA AGCTAGCTAG AAAGGATGAC

3910 3920 3930 3940 3950
ATTTTTTATG AATCTTTCAA TTCAAGATGA ATTACAACTA TTTTCTGAAG

3960 3970 3980 3990 4000
AGCTGTATCG TCATTTAACC CCTTCTCTTT TGGAAGAACT CGCTAAAGAA

4010 4020 4030 4040 4050
TTAGGTTTTG TAAAAAGAAA ACGAAAGTTT TCAGGAAATG AATTAGCTAC

4060 4070 4080 4090 4100
CATATGTATC TGGGGCAGTC AACGTACAGC GAGTGATTCT CTCGTTCGAC

4110 4120 4130 4140 4150
TATGCAGTCA ATTACACGCC GCCACAGCAC TCTTATGAGT CCAGAAGGAC

4160 4170 4180 4190 4200
TCAATAAACG CTTTGATAAA AAAGCGGTTG AATTTTTGAA ATATATTTTT

4210 4220 4230 4240 4250
TCTGCATTAT GGAAAAGTAA ACTTTGTAAA ACATCAGCCA TTTCAAGTGC

4260 4270 4280 4290 4300
AGCACTCACG TATTTTCAAC GAATCCGTAT TTTAGATGCG ACGATTTTCC

4310 4320 4330 4340 4350
AAGTACCGAA ACATTTAGCA CATGTATATC CTGGGTCAGG TGGTTGTGCA

4360
CAAACTGCAG

27. A process according to either claim 1 or claim 2, wherein the vector used in Step (b) is a bifunctional vector that, in addition to being capable of replicating in bacterial cells selected from the group consisting of B. thuringiensis and B. cereus, is capable of replicating in at least one other, or alternatively in several other, heterologous host organisms, and that is identifiable in both homologous and heterologous host systems.

28. A process according to claim 27, wherein the said heterologous host organisms are
a) prokaryotic organisms selected from the group consisting of the genera Bacillus, Staphylococcus, Streptococcus, Streptomyces, Pseudomonas, Escherichia, Agrobacterium, Salmonella, Erwinia, etc.,
or
b) eukaryotic organisms selected from the group consisting of yeast, animal and plant cells, etc..

29. A process according to claim 28, wherein the said heterologous host organism is E. coli.

30. A process according to claim 27, wherein the said bifunctional vector, in addition to the functions essential for replication and selection in homologous and heterologous host systems, also comprises a structural gene under the control of expression sequences that are capable of functioning in bacterial cells selected from the group consisting of B thuringiensis and B. cereus cells, which gene codes for a δ-endotoxin polypeptide that occurs naturally in B. thuringiensis, or alternatively for a polypeptide that is substantially homologous therewith, that is to say that at least has substantially the toxicity properties of a crystalline δ-endotoxin polypeptide of B. thuringiensis.

31. A process according to claim 30, wherein the said expression signals include a sporulation-dependent promoter of B. thuringiensis.

32. A process according to claim 1, wherein the DNA to be introduced is obtainable in accordance with Step (a) by digestion of the total DNA of a bacterial donor selected from the group consisting of B. thuringiensis and B. cereus.

## Revendications

1. Procédé d'incorporation directe et de clonage de séquences ADN dans des bactéries gram-positives choisies dans le groupe constitué de B. thuringiensis et B. cereus, caractérisé en ce que
(a) on isole l'ADN à incorporer ;
(b) on clone l'ADN ainsi isolé dans un vecteur qui peut se répliquer dans une cellule hôte bactérienne choisie dans le groupe constitué de cellules de B. thuringiensis et B. cereus ;
(c) on incorpore directement l'ADN ainsi cloné isolé par électroporation dans des cellules bactériennes intactes choisies dans le groupe constitué des cellules de B. thuringiensis et B. cereus, où la durée de décroissance exponentielle de la ou des décharges est comprise entre 2 ms et 50 ms ; et
(d) on cultive les cellules bactériennes ainsi transformées et, le cas échéant, on isole l'ADN vecteur cloné.

2. Procédé selon la revendication 1, caractérisé en ce qu'on soude l'ADN à incorporer d'abord avec des séquences d'expression, qui fonctionnent dans une cellule bactérienne choisie dans le groupe constitué des cellules de B. thuringiensis et B. cereus, et le cas échéant on isole le produit d'expression.

3. Procédé selon l'une des revendications 1 ou 2, caractérisé en ce qu'on réalise l'incorporation directe d'ADN selon l'étape (c) par électroporation.

4. Procédé selon la revendication 3, caractérisé en ce que l'incorporation par électroporation comprend les dispositions de procédé suivantes :
a) préparer une suspension de cellules avec une densité de cellules appropriée dans un milieu de culture approprié au développement de cellules bactériennes constituées de B. thuringiensis et B. cereus et avec une aération suffisante pour la croissance des cellules ;
b) séparer les cellules de la suspension de cellules et remettre en suspension dans un tampon d'inoculation approprié à l'électroporation consécutive ;
c) additionner d'une sonde d'ADN à une concentration appropriée à l'électroporation ;
d) introduire le milieu décrit au point b) et au point c) dans un appareil d'électroporation ;
e) provoquer une ou plusieurs décharges de courte durée d'un condensateur sur la suspension de cellules pour produire pendant un court intervalle de temps des champs électriques importants où la durée de décroissance exponentielle de la ou des décharges est comprise entre 2 ms et 50 ms ;
f) le cas échéant, réaliser la post-incubation des cellules qui ont subi l'électroporation ;
g) traiter sur plaque les cellules qui ont subi l'électroporation sur un milieu de sélection approprié ; et
h) récolter les cellules transformées.

5. Procédé selon la revendication 4, caractérisé en ce que, pour préparer la suspension de cellules citée au point 4 a), on utilise des spores de B. thuringiensis.

6. Procédé selon la revendication 4, caractérisé en ce qu'on utilise dans l'électroporation citée au point 4 e) des cellules de Bacillus thuringiensis et/ou Bacillus cereus congelées au préalable et dégelées pour l'essai.

7. Procédé selon la revendication 4, caractérisé en ce que pour la culture des cellules de B. thuringiensis
a) on utilise des milieux nutritifs complexes à source de carbone et d'azote facilement assimilables tels qu'on les utilise habituellement à la culture des espèces de bacilles aérobies ;
a₁) le cas échéant, on ajoute aux milieux cités en a) en plus des vitamines et des ions métalliques essentiels ; ou
b) on utilise les milieux nutritifs totalement ou semi-synthétiques, qui contiennent
b₁) une source de carbone et d'azote facilement assimilable complexe ou bien définie, ou une combinaison des deux ainsi que
b₂) des vitamines essentielles et des ions métalliques.

8. Procédé selon la revendication 4, caractérisé en ce que ladite suspension de cellules Bacillus présente une densité optique (OD₅₅₀) de 0,1 à 1,0.

9. Procédé selon la revendication 4, caractérisé en ce que le tampon d'inoculation cité au point b) est un tampon au phosphate, qui est stabilisé osmotiquement par addition d'un agent stabilisant osmotique.

10. Procédé selon la revendication 9, caractérisé en ce que ledit tampon au phosphate contient comme agent stabilisant osmotique du sucre ou des alcools de sucre.

11. Procédé selon la revendication 10, caractérisé en ce qu'il s'agit pour ledit agent stabilisant, de saccharose, qui est à une concentration de 0,1 M à 1,0 M.

12. Procédé selon la revendication 9, caractérisé en ce que ledit tampon au phosphate présente un pH compris entre pH 5,0 et pH 8,0.

13. Procédé selon la revendication 4, caractérisé en ce que l'incubation des cellules de Bacillus a lieu avant, pendant et aussi après l'électroporation à une température comprise entre 0°C et 35°C.

14. Procédé selon la revendication 4, caractérisé en ce que l'incubation des cellules de Bacillus a lieu aussi bien avant, pendant qu'après l'électroporation à une température comprise entre 2°C et 15°C.

15. Procédé selon la revendication 4, caractérisé en ce que la concentration de la sonde d'ADN ajoutée est de 1 ng à 20 µg.

16. Procédé selon la revendication 4, caractérisé en ce que les valeurs d'intensité de champ électrique sont comprises entre 3 000 V/cm et 4 500 V/cm.

17. Procédé selon la revendication 4, caractérisé en ce qu'on dépose sur plaque les cellules qui ont subi l'électroporation après une phase de post-incubation appropriée sur des milieux solides, qui contiennent des substances appropriées à la sélection des cellules de Bacillus transformées.

18. Procédé selon la revendication 20, caractérisé en ce que, pour ladite addition pour la sélection de B. thuringiensis ou B. cereus, il s'agit d'un antibiotique choisi dans le groupe constitué de tétracycline, kanamycine, chloramphénicol, érythromycine.

19. Procédé selon la revendication 17, caractérisé en ce que pour ledit ajout, il s'agit d'un substrat chromogène approprié à la sélection de B. thuringiensis ou B. cereus.

20. Procédé selon l'une des revendications 1 ou 2, caractérisé en ce que l'ADN recombinant utilisé à l'incorporation dans lesdites cellules bactériennes, choisies dans le groupe constitué de B. thuringiensis et B. cereus, est d'origine homologue ou hétérologue, ou il s'agit d'une combinaison d'ADN homologue et hétérologue.

21. Procédé selon la revendication 2, caractérisé en ce que ledit ADN recombinant contient un ou plusieurs gènes de structure ainsi que des séquences contiguës en 3' et 5' régulatrices pouvant fonctionner dans Bacillus thuringiensis ou Bacillus cereus, ou dans les deux, qui sont reliées de manière fonctionnelle avec le ou les gènes de structure et qui permettent ainsi l'expression du ou des gènes de structures dans Bacillus thuringiensis ou Bacillus cereus, ou dans les deux.

22. Procédé selon la revendication 21, caractérisé en ce que les séquences d'ADN contiguës de 3' et 5' incluent un promoteur de sporulation de Bacillus thuringiensis.

23. Procédé selon la revendication 21, caractérisé en ce que ledit gène de structure code pour un polypeptide de δ-endotoxine, qui se présente naturellement dans B. thuringiensis, mais aussi pour un polypeptide, qui est essentiellement homologue du premier, c'est-à-dire qu'il présente essentiellement les propriétés de toxicité d'un polypeptide de δ-endotoxine cristalline de B. thuringiensis.

24. Procédé selon la revendication 23, caractérisé en ce que ladite séquence d'ADN codant pour la δ-endotoxine est homologue au moins essentiellement à une partie ou à des parties de la séquence naturelle codant pour la δ-endotoxine, qui est ou qui sont responsables de l'activité insecticide.

25. Procédé selon la revendication 23, caractérisé en ce que ledit polypeptide est essentiellement homologue d'un polypeptide de δ-endotoxine provenant de sous-espèces appropriées de B. thuringiensis, choisies dans le groupe constitué de kurstaki, berliner, alesti, sotto, tolworthi, dendrolimus, tenebrionis et israelensis.

26. Procédé selon la revendication 23, caractérisé en ce qu'il s'agit, pour la séquence d'ADN codant pour ladite δ-endotoxine d'un fragment d'ADN de B. thuringiensis var. kurstaki HDl, qui se trouve entre les nucléotides 156 et 3 623 dans la formule I, mais aussi de tout autre fragment d'ADN plus court, qui code encore pour un polypeptide ayant des propriétés toxiques pour les insectes :

Formule I

10 20 30 40 50
GTTAACACCC TGGGTCAAAA ATTGATATTT AGTAAAATTA GTTGCACTTT

60 70 80 90 100
GTGCATTTTT TCATAAGATG AGTCATATGT TTTAAATTGT AGTAATGAAA

110 120 130 140 150
AACAGTATTA TATCATAATG AATTGGTATC TTAATAAAAG AGATGGAGGT

160 170 180 190 200
AACTTATGGA TAACAATCCG AACATCAATG AATGCATTCC TTATAATTGT

210 220 230 240 250
TTAAGTAACC CTGAAGTAGA AGTATTAGGT GGAGAAAGAA TAGAAACTGG

260 270 280 290 300

TTACACCCCA ATCGATATTT CCTTGTCGCT AACGCAATTT CTTTTGAGTG

310 320 330 340 350
AATTTGTTCC CGGTGCTGGA TTTGTGTTAG GACTAGTTGA TATAATATGG

360 370 380 390 400
GGAATTTTTG GTCCCTCTCA ATGGGACGCA TTTCTTGTAC AAATTGAACA

410 420 430 440 450
GTTAATTAAC CAAAGAATAG AAGAATTCGC TAGGAACCAA GCCATTTCTA

460 470 480 490 500
GATTAGAAGG ACTAAGCAAT CTTTATCAAA TTTACGCAGA ATCTTTTAGA

510 520 530 540 550
GAGTGGGAAG CAGATCCTAC TAATCCAGCA TTAAGAGAAG AGATGCGTAT

560 570 580 590 600
TCAATTCAAT GACATGAACA GTGCCCTTAC AACCGCTATT CCTCTTTTTG

610 620 630 640 650
CAGTTCAAAA TTATCAAGTT CCTCTTTTAT CAGTATATGT TCAAGCTGCA

660 670 680 690 700
AATTTACATT TATCAGTTTT GAGAGATGTT TCAGTGTTTG GACAAAGGTG

710 720 730 740 750
GGGATTTGAT GCCGCGACTA TCAATAGTCG TTATAATGAT TTAACTAGGC

760 770 780 790 800
TTATTGGCAA CTATACAGAT CATGCTGTAC GCTGGTACAA TACGGGATTA

810 820 830 840 850
GAGCGTGTAT GGGGACCGGA TTCTAGAGAT TGGATAAGAT ATAATCAATT

860 870 880 890 900

TAGAAGAGAA TTAACACTAA CTGTATTAGA TATCGTTTCT CTATTTCCGA

910 920 930 940 950
ACTATGATAG TAGAACGTAT CCAATTCGAA CAGTTTCCCA ATTAACAAGA

960 970 980 990 1000
GAAATTTATA CAAACCCAGT ATTAGAAAAT TTTGATGGTA GTTTTCGAGG

1010 1020 1030 1040 1050
CTCGGCTCAG GGCATAGAAG GAAGTATTAG GAGTCCACAT TTGATGGATA

1060 1070 1080 1090 1100
TACTTAACAG TATAACCATC TATACGGATG CTCATAGAGG AGAATATTAT

1110 1120 1130 1140 1150
TGGTCAGGGC ATCAAATAAT GGCTTCTCCT GTAGGGTTTT CGGGGCCAGA

1160 1170 1180 1190 1200
ATTCACTTTT CCGCTATATG GAACTATGGG AAATGCAGCT CCACAACAAC

1210 1220 1230 1240 1250
GTATTGTTGC TCAACTAGGT CAGGGCGTGT ATAGAACATT ATCGTCCACT

1260 1270 1280 1290 1300
TTATATAGAA GACCTTTTAA TATAGGGATA AATAATCAAC AACTATCTGT

1310 1320 1330 1340 1350
TCTTGACGGG ACAGAATTTG CTTATGGAAC CTCCTCAAAT TTGCCATCCG

1360 1370 1380 1390 1400
CTGTATACAG AAAAAGCGGA ACGGTAGATT CGCTGGATGA AATACCGCCA

1410 1420 1430 1440 1450
CAGAATAACA ACGTGCCACC TAGGCAAGGA TTTAGTCATC GATTAAGCCA

1460 1470 1480 1490 1500

TGTTTCAATG TTTCGTTCAG GCTTTAGTAA TAGTAGTGTA AGTATAATAA

1510 1520 1530 1540 1550
GAGCTCCTAT GTTCTCTTGG ATACATCGTA GTGCTGAATT TAATAATATA

1560 1570 1580 1590 1600
ATTCCTTCAT CACAAATTAC ACAAATACCT TTAACAAAAT CTACTAATCT

1610 1620 1630 1640 1650
TGGCTCTGGA ACTTCTGTCG TTAAAGGACC AGGATTTACA GGAGGAGATA

1660 1670 1680 1690 1700
TTCTTCGAAG AACTTCACCT GGCCAGATTT CAACCTTAAG AGTAAATATT

1710 1720 1730 1740 1750
ACTGCACCAT TATCACAAAG ATATCGGGTA AGAATTCGCT ACGCTTCTAC

1760 1770 1780 1790 1800
CACAAATTTA CAATTCCATA CATCAATTGA CGGAAGACCT ATTAATCAGG

1810 1820 1830 1840 1850
GGAATTTTTC AGCAACTATG AGTAGTGGGA GTAATTTACA GTCCGGAAGC

1860 1870 1880 1890 1900
TTTAGGACTG TAGGTTTTAC TACTCCGTTT AACTTTTCAA ATGGATCAAG

1910 1920 1930 1940 1950
TGTATTTACG TTAAGTGCTC ATGTCTTCAA TTCAGGCAAT GAAGTTTATA

1960 1970 1980 1990 2000
TAGATCGAAT TGAATTTGTT CCGGCAGAAG TAACCTTTGA GGCAGAATAT

2010 2020 2030 2040 2050
GATTTAGAAA GAGCACAAAA GGCGGTGAAT GAGCTGTTTA CTTCTTCCAA

2060 2070 2080 2090 2100

TCAAATCGGG TTAAAAACAG ATGTGACGGA TTATCATATT GATCAAGTAT

2110 2120 2130 2140 2150
CCAATTTAGT TGAGTGTTTA TCTGATGAAT TTTGTCTGGA TGAAAAAAAA

2160 2170 2180 2190 2200
GAATTGTCCG AGAAAGTCAA ACATGCGAAG CGACTTAGTG ATGAGCGGAA

2210 2220 2230 2240 2250
TTTACTTCAA GATCCAAACT TTAGAGGGAT CAATAGACAA CTAGACCGTG

2260 2270 2280 2290 2300
GCTGGAGAGG AAGTACGGAT ATTACCATCC AAGGAGGCGA TGACGTATTC

2310 2320 2330 2340 2350
AAAGAGAATT ACGTTACGCT ATTGGGTACC TTTGATGAGT GCTATCCAAC

2360 2370 2380 2390 2400
GTATTTATAT CAAAAAATAG ATGAGTCGAA ATTAAAAGCC TATACCCGTT

2410 2420 2430 2440 2450
ACCAATTAAG AGGGTATATC GAAGATAGTC AAGACTTAGA AATCTATTTA

2460 2470 2480 2490 2500
ATTCGCTACA ATGCCAAACA CGAAACAGTA AATGTGCCAG GTACGGGTTC

2510 2520 2530 2540 2550
CTTATGGCCG CTTTCAGCCC CAAGTCCAAT CGGAAAATGT GCCCATCATT

2560 2570 2580 2590 2600
CCCATCATTT CTCCTTGGAC ATTGATGTTG GATGTACAGA CTTAAATGAG

2610 2620 2630 2640 2650
GACTTAGGTG TATGGGTGAT ATTCAAGATT AAGACGCAAG ATGGCCATGC

2660 2670 2680 2690 2700

AAGACTAGGA AATCTAGAAT TTCTCGAAGA GAAACCATTA GTAGGAGAAG

2710 2720 2730 2740 2750
CACTAGCTCG TGTGAAAAGA GCGGAGAAAA AATGGAGAGA CAAACGTGAA

2760 2770 2780 2790 2800
AAATTGGAAT GGGAAACAAA TATTGTTTAT AAAGAGGCAA AAGAATCTGT

2810 2820 2830 2840 2850
AGATGCTTTA TTTGTAAACT CTCAATATGA TAGATTACAA GCGGATACCA

2860 2870 2880 2890 2900
ACATCGCGAT GATTCATGCG GCAGATAAAC GCGTTCATAG CATTCGAGAA

2910 2920 2930 2940 2950
GCTTATCTGC CTGAGCTGTC TGTGATTCCG GGTGTCAATG CGGCTATTTT

2960 2970 2980 2990 3000
TGAAGAATTA GAAGGGCGTA TTTTCACTGC ATTCTCCCTA TATGATGCGA

3010 3020 3030 3040 3050
GAAATGTCAT TAAAAATGGT GATTTTAATA ATGGCTTATC CTGCTGGAAC

3060 3070 3080 3090 3100
GTGAAAGGGC ATGTAGATGT AGAAGAACAA AACAACCACC GTTCGGTCCT

3110 3120 3130 3140 3150
TGTTGTTCCG GAATGGGAAG CAGAAGTGTC ACAAGAAGTT CGTGTCTGTC

3160 3170 3180 3190 3200
CGGGTCGTGG CTATATCCTT CGTGTCACAG CGTACAAGGA GGGATATGGA

3210 3220 3230 3240 3250
GAAGGTTGCG TAACCATTCA TGAGATCGAG AACAATACAG ACGAACTGAA

3260 3270 3280 3290 3300

GTTTAGCAAC TGTGTAGAAG AGGAAGTATA TCCAAACAAC ACGGTAACGT

3310 3320 3330 3340 3350
GTAATGATTA TACTGCGACT CAAGAAGAAT ATGAGGGTAC GTACACTTCT

3360 3370 3380 3390 3400
CGTAATCGAG GATATGACGG AGCCTATGAA AGCAATTCTT CTGTACCAGC

3410 3420 3430 3440 3450
TGATTATGCA TCAGCCTATG AAGAAAAAGC ATATACAGAT GGACGAAGAG

3460 3470 3480 3490 3500
ACAATCCTTG TGAATCTAAC AGAGGATATG GGGATTACAC ACCACTACCA

3510 3520 3530 3540 3550
GCTGGCTATG TGACAAAAGA ATTAGAGTAC TTCCCAGAAA CCGATAAGGT

3560 3570 3580 3590 3600
ATGGATTGAG ATCGGAGAAA CGGAAGGAAC ATTCATCGTG GACAGCGTGG

3610 3620 3630 3640 3650
AATTACTTCT TATGGAGGAA TAATATATGC TTTATAATGT AAGGTGTGCA

3660 3670 3680 3690 3700
AATAAAGAAT GATTACTGAC TTGTATTGAC AGATAAATAA GGAAATTTTT

3710 3720 3730 3740 3750
ATATGAATAA AAAACGGGCA TCACTCTTAA AAGAATGATG TCCGTTTTTT

3760 3770 3780 3790 3800
GTATGATTTA ACGAGTGATA TTTAAATGTT TTTTTTGCGA AGGCTTTACT

3810 3820 3830 3840 3850
TAACGGGGTA CCGCCACATG CCCATCAACT TAAGAATTTG CACTACCCCC

3860 3870 3880 3890 3900

AAGTGTCAAA AAACGTTATT CTTTCTAAAA AGCTAGCTAG AAAGGATGAC

3910 3920 3930 3940 3950
ATTTTTTATG AATCTTTCAA TTCAAGATGA ATTACAACTA TTTTCTGAAG

3960 3970 3980 3990 4000
AGCTGTATCG TCATTTAACC CCTTCTCTTT TGGAAGAACT CGCTAAAGAA

4010 4020 4030 4040 4050
TTAGGTTTTG TAAAAAGAAA ACGAAAGTTT TCAGGAAATG AATTAGCTAC

4060 4070 4080 4090 4100
CATATGTATC TGGGGCAGTC AACGTACAGC GAGTGATTCT CTCGTTCGAC

4110 4120 4130 4140 4150
TATGCAGTCA ATTACACGCC GCCACAGCAC TCTTATGAGT CCAGAAGGAC

4160 4170 4180 4190 4200
TCAATAAACG CTTTGATAAA AAAGCGGTTG AATTTTTGAA ATATATTTTT

4210 4220 4230 4240 4250
TCTGCATTAT GGAAAAGTAA ACTTTGTAAA ACATCAGCCA TTTCAAGTGC

4260 4270 4280 4290 4300
AGCACTCACG TATTTTCAAC GAATCCGTAT TTTAGATGCG ACGATTTTCC

4310 4320 4330 4340 4350
AAGTACCGAA ACATTTAGCA CATGTATATC CTGGGTCAGG TGGTTGTGCA

4360
CAAACTGCAG

27. Procédé selon l'une des revendications 1 ou 2, caractérisé en ce qu'à l'étape (b), le vecteur utilisé est un vecteur bifonctionnel qui est non seulement capable de se répliquer dans des cellules bactériennes choisies dans le groupe constitué de B. thuringiensis et B. cereus, mais qui peut aussi se répliquer dans au moins un ou dans plusieurs autres organismes hôtes hétérologues, et qu'on peut identifier aussi bien dans les systèmes hôtes homologues que dans les systèmes hétérologues.

28. Procédé selon la revendication 27, caractérisé en ce que pour lesdits organismes hôtes hétérologues, il s'agit de
a) d'organismes procaryotes, choisis dans le groupe constitué des genres Bacillus, Staphylococcus, Streptococcus, Streptomyces, Pseudomonas, Escherichia, Agrobacterium, Salmonella, Erwinia, etc.
ou
b) d'organismes eucaryotes choisis dans le groupe constitué des levures, des cellules animales et végétales, etc.

29. Procédé selon la revendication 28, caractérisé en ce que pour ledit organisme hôte hétérologue, il s'agit de E. coli.

30. Procédé selon la revendication 27, caractérisé en ce que ledit vecteur bifonctionnel contient outre les fonctions essentielles à la réplication et la sélection dans les systèmes hôtes homologues et hétérologues, en plus encore un gène de structure sous le contrôle de séquences d'expression pouvant fonctionner dans les cellules bactériennes, choisies dans le groupe constitué de B. thuringiensis et B. cereus, qui codent pour un polypeptide de δ-endotoxine, qui se trouve naturellement dans B. thuringiensis ou pour un polypeptide qui est essentiellement homologue du premier, c'est-à-dire qu'il présente essentiellement les propriétés de toxicité d'un polypeptide de δ-endotoxine cristalline de B. thuringiensis.

31. Procédé selon la revendication 30, caractérisé en ce que lesdits signaux d'expression incluent un promoteur de sporulation de B. thuringiensis.

32. Procédé selon la revendication 1, caractérisé en ce que l'on peut obtenir l'ADN à incorporer selon l'étape (a) par digestion de l'ADN total d'un donneur bactérien choisi dans le groupe constitué de B. thuringiensis et B. cereus.
